# EUROPEAN PATENT APPLICATION

(11) **EP 3 511 341 A1**
(43) Date of publication of application: **17.07.2019**
(21) Application number: 17849118.9
(22) Date of filing: 08.09.2017
(51) Int. Cl.: C07K 16/18, A61K 39/395, A61K 39/00

(54) **DEGLYCOSYLATED ANTIBODY SPECIFICALLY BINDING TO CLEC14A AND USES THEREOF**

(30) Priority: 08.09.2016 KR 20160115577
(71) Applicant: WOORI TECHNOLOGIES CORPORATION, 9, World Cup buk-ro 56-gil, Mapo-gu Seoul 03923 (KR)
(72) Inventor: LEE, Sukmook, Seoul 05393 (KR); KIM, Taek-Keun, Chuncheon-si Gangwon-do 24271 (KR); KIM, Mi Ra, Hongcheon-gun Gangwon-do 25125 (KR); JANG, Ji Hye, Chuncheon-si Gangwon-do 24438 (KR)
(74) Representative: Mewburn Ellis LLP
(86) International application number: PCT/KR2017/009851
(87) International publication number: WO 2018/048234

(57) **Abstract**

The present disclosure relates to a deglycosylated antibody binding specifically to C-type lectin domain family 14, member A (clecl4a). More particularly, the present disclosure relates to a deglycosylated antibody that comprises a light chain variable region comprising CDR1 having a certain sequence and binds specifically to clec14, and use thereof, for example, a pharmaceutical composition containing the antibody for preventing or treating an angiogenesis-related disease.

## Description

### [Technical Field]

The present disclosure relates to a deglycosylated antibody binding specifically to C-type lectin domain family 14, member A (clec14a). More particularly, the present disclosure relates to a deglycosylated antibody that comprises a light chain variable region comprising CDR1 having a certain sequence and binds specifically to clec14, and use thereof, for example, a pharmaceutical composition containing the antibody for preventing or treating an angiogenesis-related disease.

### [Background Art]

Tumor angiogenesis plays a key role in the process of tumors. Vascular endothelial growth factors (VEGFs) and epidermal growth factor receptors (EGFRs) are key factors in angiogenesis which is a very promising target in the treatment of cancers. Bevacizumab (Avastin®), which is an anti-VEGF antibody, is used to treat patients who suffer from a disease such as metastatic colorectal cancer, renal cell carcinoma, non-small cell lung cancer, or malignant brain glioma. Cetuximab, which is an anti-EGFR antibody, can inhibit cell-contacts between endothelial cells and suppress expression of angiogenesis factors such as VFGFs, interleukin-8 and basic fibroblast growth factors.

However, Avastin®, which is a single agent, has no efficacy in clinic trials and is used for combination therapy with a variety of chemical drugs. Combination therapy involves use of various chemical drugs in conjunction with the treatment, thus having a risk of various side effects on patients. In addition, Avastin® is known to inhibit VEGF signaling actions of both tumor vessels and normal blood vessels, and thus result in side effects such as proteinuria, hypertension, bleeding and gastrointestinal perforation through induction of defects to the normal blood vessels.

Furthermore, Avastin® may cause tolerance when used for a long time. Since high levels of VEGF-A, -B and -C, PIGFs (placental growth factors) and VEGF receptor-1 are expressed in tolerant colorectal cancer cells, expression of various pro-angiogenic soluble factors and receptors may be increased in spite of treatment with VEGF-neutralizing antibodies.

There are unmet-needs for developing novel antibody medications to solve these side-effects and tolerance of Avastin. In this regard, the present inventors found that CTLD of Clec14a, so called C-type lectin-like domain (CTLD), a series of epidermal growth factor-like domain and with sushi-like domain, all of which are named as an extracellular domain included in a type I transmembrane protein, plays a key role in actin cytoskeletal rearrangement that is important for cell migration. In addition, based on this fact, the present inventors screened human antibodies specific to clec14a-CTLD and found that the screened antibodies can inhibit tumor angiogenesis by exhibiting high cross-reactivity to human and mouse clec14a-CTLDs, and suppressing migration of vascular endothelial cells, tube formation and endothelial cell-cell contacts. As a result, the present inventors filed a PCT patent application (WO 2013-187556).

In order to evaluate efficacy and toxicity of developed antibodies in an animal model, large scale production of antibodies is required and stability of antibodies is important. However, in the process of purifying human antibodies specific to clec14a-CTLD, protein aggregation occurs. This indicates that the process of improving stability is needed to increase an antibody yield.

The present disclosure was completed based on the finding that not only desired efficacies, but also antibody stability can be accomplished by glycosylation changes in antibodies based on prediction of glycosylation, since the glycosylation of antibodies may affect stability as well as functions of the antibodies during large scale production.

### [Disclosure]

### [Technical Problem]

Therefore, the present disclosure has been made in view of the above problems, and it is one object of the present disclosure to provide a deglycosylated antibody binding specifically to clec14a, wherein CDR of clec14a-CTLD IgG of clone 1, which is a clec14a-CTLD-specific antibody disclosed in PCT Patent Application Laid-open No. WO2013/187556, is grafted to commercially available therapeutic antibodies, and is replaced with the framework of the therapeutic antibody, and a part of glycosylation sites in light chain CDR1 amino acid sequences is substituted with other amino acid sequences.

It is another object of the present disclosure to provide a pharmaceutical composition containing the antibody for preventing or treating an angiogenesis-related disease.

### [Technical Solution]

In accordance with the present disclosure, the above and other objects can be accomplished by the provision of an antibody binding specifically to clec14a, wherein the antibody comprises a light chain variable region comprising CDR1 of TGSSSNIGXXXVT (SEQ ID NO: 1), wherein each of amino acids X at positions 9, 10 and 11 in the SEQ ID NO: 1 is any one selected from the group consisting of R, C, G, A, T, W, S, N, and V.

In accordance with another aspect of the present disclosure, provided is a pharmaceutical composition containing the antibody for preventing or treating an angiogenesis-related disease.

Other technical features and examples of the present disclosure will be more clearly described in the following Detailed Description of the Invention and Claims mentioned later.

### [BRIEF DESCRIPTION OF THE DRAWINGS]

The above and other objects, features and other advantages of the present disclosure will be more clearly understood from the following detailed description taken in conjunction with the accompanying drawings, in which:
FIG. 1A is a schematic diagram illustrating CDR grafting of parent antibody IgG from four types of therapeutic antibodies, i.e., adalimumab (Humira®), omalizumab (Xolair®), trastuzumab (Herceptin®) and bevacizumab (Avastin®) ;
FIG. 1B shows results of visual observation regarding aggregation of antibodies in parent antibody IgG and clone 1 IgG preparations;
FIG. 1C shows results of measurement using spectrophotometry regarding aggregation indices of parent antibody IgG (green) and clone 1 IgG (orange), wherein the aggregation index is calculated in accordance with 100 X [Abs340/(Abs280 - Abs340)];
FIG. 1D shows results of analysis regarding parent antibody IgG (green) and clone 1 IgG (orange) before and after precipitation of antibodies;
FIG. 2A is a schematic diagram illustrating a deglycosylation process to screen four types of glycosylated IgG clones (deglyco C1-C4) by phage display technology;
FIG. 2B shows results of measurement using ELISA regarding the binding specificity of four types of deglycosylated IgG to hclec14a-CTLD-Fc, mclec14a-CTLD-Fc and Fc alone;
FIG. 2C shows results of screening using HUVEC tube formation assay regarding optimized candidate antibodies for suppressing clec14a-mediated angiogenesis, wherein clone 1 IgG is used as a positive control group;
FIG. 2D shows results of screening using HUVEC tube formation assay regarding optimized candidate antibodies for suppressing clec14a-mediated angiogenesis, wherein the total number of branches is represented as percentage (%) of tube formation of control group (MOCK);
FIG. 2E shows results of investigation using wound healing assay regarding effects of deglyco C1 IgG on migration of endothelial cells;
FIG. 2F is a graph showing results of investigation using wound healing assay regarding effects of deglyco C1 IgG on migration of endothelial cells, wherein wound difference is represented as a percentage (%) of cell migration of control group (MOCK);
FIG. 2G shows results of investigation regarding mobility of deglyco C1 IgG and clone 1 IgG under reduction conditions using one-dimensional electrophoresis;
FIG. 2H shows results of investigation regarding homogeneity of deglyco C1 IgG and clone 1 IgG using two-dimensional electrophoresis;
FIG. 3A shows results of competitive ELISA through addition of parent antibody IgG binding to deglyco C1 IgG-HRP and hclec14a-CTLD-Fc;
FIG. 3B shows results of flow cytometry in the presence of parent antibody IgG (green) or deglyco C1 IgG (red), or the absence thereof (MOCK, black);
FIG. 3C shows results of measurement regarding affinity of parent antibody IgG (green) or deglyco C1 IgG (red) to hclec14a-ECD-myc using biolayer interferometry assay through Octet RED96 system (*K*_{D}*=equilibrium dissociation constant; Kₒₙ=association rate constant; K_{off}=dissociation rate constant);
FIG. 3D shows results of HUVEC tube formation assay in the presence of parent antibody IgG (green), deglyco C1 IgG (red) or bevacizumab (blue) or in the absence thereof (MOCK, black);
FIG. 3E shows results of HUVEC tube formation assay, wherein the total number of branches is represented as a percentage of tube formation of control group (MOCK);
FIG. 3F shows results of wound healing assay in the presence of parent antibody IgG (green), deglyco C1 IgG (red) or bevacizumab (blue) or in the absence thereof (MOCK, black). An image was captured at 0h (upper part) and 8h (lower part) using an optical microscope;
FIG. 3G shows results of wound healing assay, wherein wound difference is represented as a percentage (%) of cell migration of control group (MOCK);
FIG. 4A shows results of counting using an optical microscope regarding HEK293F cells transfected with wild-type clec14a and cultured for 6h in the presence of parent antibody IgG (green) or deglyco C1 IgG (red) or the absence thereof (MOCK, black);
FIG. 4B shows the number of aggregates per field calculated as a percentage (%) of clec14a-mediated cell-cell contacts of the control group (MOCK);
FIG. 4C shows results of measurement using cell ELISA regarding hclec14a-CTLD-Fc-HRP bound to HUVECs coated on a microtiter plate in the presence or absence of parent antibody IgG (green) or deglyco C1 IgG (red) with an increasing concentration;
FIG. 4D shows results of measurement using cell ELISA regarding clec14a-ECD-myc bound to clec14a-CTLD-Fc coated on a microtiter plate in the absence or presence of parent antibody IgG (green) or deglyco C1 IgG (red) with an increasing concentration;
FIG. 5A shows results of investigation on cell viability based on an absorbance at 450 nm regarding HUVECs incubated in the absence of deglyco C1 IgG (black) or presence (red) or in the presence of 5-FU (yellow) for 2 days;
FIG. 5B shows results of culture of HUVECs in the presence of hTNFα (pink), the absence of deglyco C1 IgG (black), or the presence of deglyco C1 IgG (red), wherein analysis is conducted by flow cytometry after staining with anti-ICAM-1 (upper part) or VCAM-1 (lower part) polyclonal antibody. hTNFα is a positive control group for endothelial cell activity;
FIG. 5C shows results of Rhodamine-Phalloidin and DAPI staining regarding HUVECs cultured in the absence or presence of deglyco C1 IgG, wherein the morphology of HUVECs is observed with a confocal microscope;
FIG. 5D shows results of immunoblot analysis on phosphorylation of VEGF-dependent VEGFR (VEGF receptor), Akt and ERK (extracellular signal-regulated kinase) in the presence of VEGF or VEGF and deglyco C1 IgG, or the absence thereof (MOCK);
FIG. 5E shows evaluation results of *in vitro* and *in vivo* toxicity of optimized candidate antibodies, specifically, measurement results of serum concentrations of GOT, GPT, BUN, CRE, and TBIL 30 days after antibody injection;
FIG. 5F shows evaluation results of *in vitro* and *in vivo* toxicity of optimized candidate antibodies, specifically, measurement results of mouse body weight one day and 28 days after antibody injection;
FIG. 5G shows evaluation results of *in vitro* and *in vivo* toxicity of optimized candidate antibodies, specifically, results of TUNEL assay analysis regarding apoptosis conditions of renal and hepatic tissues 30 days after antibody injection;
FIG. 6A shows results of VEGF-dependent tube formation assay in the presence of deglyco C1 IgG (red) or the absence thereof (MOCK);
FIG. 6B shows results of VEGF-dependent tube formation assay, wherein the total number of branches is represented as a percentage (%) of tube formation of control group (MOCK);
FIG. 6C is an image showing VEGF-dependent blood vessel growth in a cut aortic ring cultured in the presence of parent antibody IgG (green), deglyco C1 IgG (red) and bevacizumab (blue), or the absence thereof (MOCK);
FIG. 6D shows results of counting regarding the number of grown blood vessels;
FIG. 6E is an image showing VEGF-dependent microvessel formation in a Matrigel plug of a nude mouse in the presence of parent antibody IgG, deglyco C1 IgG and bevacizumab, or the absence thereof (MOCK);
FIG. 6F shows results of investigation on microvessel formation, based on measured hemoglobin content, wherein the hemoglobin content is represented as a percentage (%) of hemoglobin content of control group (MOCK);
FIG. 7A shows results of immunohistochemistry using antibodies to clec14a and CD31 to detect specific expression of clec14a in blood vessels of SNU182 cancer cell xenograft mouse tissues;
FIG. 7B shows results of immunohistochemistry using antibodies to clec14a and CD31 to detect specific expression of clec14a in blood vessels of CFPAC-1 cancer cell xenograft mouse tissues;
FIG. 7C shows results of immunohistochemistry staining using antibodies to clec14a and CD31 to detect specific expression of clec14a in tumor vessels of liver cancer tissues;
FIG. 7D shows results of immunohistochemistry staining using antibodies to clec14a and CD31 to detect specific expression of clec14a in tumor vessels of pancreatic cancer tissues;
FIG. 7E shows measurement results of SNU182-, CFPAC-1 or U87 cell-derived microvessel formation in the absence (MOCK), the presence of deglyco C1 IgG (red) or the presence of bevacizutab (blue);
FIG. 7F shows measurement results of SNU182-, CFPAC-1 or U87 cell-derived microvessel formation, wherein the hemoglobin content is represented as a percentage of hemoglobin content of control group (MOCK);
FIG. 7G shows measurement results of immunohistochemistry using CD31 regarding microvessel formation by HCT116 and HCT116/Beva cell-derived tumors in the presence of deglyco C1 IgG and bevacizumab or the absence thereof (MOCK);
FIG. 7H shows a percentage of CD31 positive per field represented with respect to the microvessel density of control group (MOCK);
FIG. 7I shows results of evaluation regarding effects of deglyco C1 IgG on tumor growth, wherein deglyco C1 IgG can reduce tumor size while not affecting body weight, and tumor volume and body weight are measured on a weekly basis for one month;
FIG. 8A shows final antibody production of produced optimized candidate antibodies;
FIG. 8B shows results of investigation using SDS-PAGE regarding 90% purification of produced optimized candidate antibodies and molecular weights of light chains and heavy chains;
FIG. 9A shows results of investigation regarding changes in tube length after treatment of HUVECs with VEGF and the optimized antibodies;
FIG. 9B shows results of investigation regarding the number of branches after treatment of HUVEC with VEGF and the optimized antibodies;
FIG. 10 is a representative image showing changes in tube length (progression of angiogenesis) after treatment with VEGF and optimized antibodies;
FIGS. 11A and 11B show results of tube formation analysis after treatment with clone 1, 13 and 16 antibodies exhibiting anti-angiogenesis activity against HUVEC cells in the presence of EGM;
FIGS. 12A and 12B show results of analysis regarding whether or not the optimized antibodies including clone 1, 13 and 16 antibodies suppress clec14a-mediated cell-cell contacts in clec14a-expressed HEK293 cells;
FIGS. 13A and 13B show results of analysis regarding whether or not clone 1, 13 and 16 antibodies exhibit inhibitory activity against endothelial migration;
FIG. 14A is a schematic diagram illustrating competitive ELISA to identify antigen-binding sites of screened antibodies;
FIG. 14B shows results of analysis regarding whether or not the optimized candidate antibodies bind to antigen, comparatively with deglyco C1;
FIG. 15 shows results of investigation using flow cytometry regarding whether or not clone 1, 13 and 16 antibodies have the ability to bind to human umbilical vein endothelial cells (HUVECs) and mouse aortic endothelial cells (MAECs);
FIG. 16A is a schematic diagram illustrating the role of CLEC14a-CTLD on cell-cell contacts in vascular endothelial cells and the mechanism by which the CLEC14-CTLD-conjugated antibody acts as an inhibitor of cell-cell contacts;
FIG. 16B shows investigation results of the role of CTLD on CLEC14a-CLEC14a bonding;
FIG. 16C shows results of analysis using competitive ELISA regarding whether or not clone 1, 13 and 16 antibodies suppress CTLD domain-mediated CLEC14a molecular bonding;
FIG. 17A illustrates CLEC14a down-regulation on the surface of vascular endothelial cells by a CLEC14a-CTLD-conjugated antibody; and
FIG. 17B illustrates results of investigation using cell ELISA regarding CLEC14a down-regulation of the optimized antibody.

### [Best Mode]???

In one aspect, the present disclosure describes an antibody or antigen binding fragment thereof binding specifically to clec14a (C-type lectin domain family 14, member A), wherein the antibody comprises a light chain variable region comprising CDR1 of TGSSSNIGXXXVT (SEQ ID NO: 1), wherein each of amino acids X at positions 9, 10 and 11 of SEQ ID NO: 1 is any one selected from the group consisting of R, C, G, A, T, W, S, N, and V.

The term "antibody" as used herein refers to a protein molecule including an immunoglobulin molecule that specifically recognizes an antigen and thus immunologically reacts with the specific antigen, and includes a polyclonal antibody, a monoclonal antibody, whole antibody and an antibody fragment. In addition, chimeric antibodies (*e.g.,* humanized mouse antibodies), bivalent or bispecific molecules (*e.g.,* bispecific antibodies), diabodies, triabodies and tetrabodies fall within the scope of the antibody used in the present disclosure.

The whole antibody is composed of two overall length light chains and two overall length heavy chains, wherein each light chain is linked to a heavy chain by a disulfide bond. There are five antibody isotypes known as IgA, IgD, IgE, IgM, and IgG existing in mammals, and IgG is further classified into four antibody subtypes of IgG1, IgG2, IgG3 and IgG4.

The term "antibody fragment" as used herein refers to a fragment that at least maintains an antigen-binding ability and includes Fab, F(ab'), F(ab')₂, and Fv. Fab includes a variable region of each of the heavy chain and the light chain, the constant domain of the light chain, and the first constant domain (CH1) of the heavy chain, each having an antigen-binding site. Fab' is different from Fab in that it further includes at least one cysteine residue at a C-terminus of the CH1 domain of the heavy chain. F(ab')₂ includes two Fab' molecules having a disulfide bond between cysteine residues in a hinge region. An Fv (variable fragment) including a variable region of each of the heavy chain and the light chain is the minimal antibody fragment having original specificity of parent immunoglobulin. Disulfide-stabilized Fv (dsFv) is formed by binding the variable region of the light chain to the variable region of the heavy chain via a disulfide bond. Single chain Fv (scFV) is an Fv where the respective variable regions of the heavy chain and the light chain are covalently linked via a peptide linker. These antibody fragments can be obtained by treating the whole antibody with a protease (for example, papain or pepsin providing Fab or F(ab')₂), and are preferably constructed by genetic recombination technology.

The term "monoclonal antibody" as used herein refers to an antibody molecule having a uniform molecule composition which is obtained from a substantially identical population of antibodies and exhibits a binding specificity and affinity to a single epitope.

In general, immunoglobulin has a basic structural unit including one heavy chain and two light chains. Each heavy chain includes one variable region and three constant domains, whereas each light chain includes one variable region and one constant domain. The variable region of each of the heavy chain and the light chain includes three complementarity-determining regions (referred to as "CDRs") and four framework regions. CDRs function to bind to epitopes of antibodies. CDRs on each chain start from the N-terminus and are arranged in an order of CDR1, CDR2, and CDR3. These CDRs are distinguished from one another by the chain on which they are positioned.

Regarding the antibodies according to the present disclosure, firstly, CDR grafting was conducted to conjugate six sequences of CDR1 to CDR3 (SEQ ID NOS: 11 to 16) of heavy chain and light chain variable regions of clec14a-CTLD IgG clone 1, the clec14a-CTLD human antibody (hereinafter, parent antibody) disclosed in WO 2013/187556, filed by the present inventors, with framework regions of four types of therapeutic antibodies approved by the FDA, i.e., adalimumab (Humira®), omalizumab (Xolair®), trastuzumab (Herceptin®), and bevacizumab (Avastin®).

According to one embodiment of the present disclosure, aggregation degree and developability index (DI) of sequences of the four types of CDR-grafted antibodies and the parent antibody were observed. An aggregation score is a parameter for predicting an aggregation degree on sequences. As aggregation score decreases, aggregation decreases. The DI index is a parameter for predicting protein stability in a solution. As DI index decreases, solubility and stability of antibody increase. As a result, the parent antibody is predicted to have a relatively high aggregation degree and DI index, whereas, among four types of antibodies, the antibody substituted with the framework of omalizumab (Xolair®) exhibits superior aggregation degree and DI index.

In addition, the antibody substituted with the framework of omalizumab (Xolair®) secures excellent productivity, as compared to three other types of CDR-grafted antibodies, and shows no aggregation. In addition, only the antibody substituted with the framework of omalizumab (Xolair®) exhibits cross-reactivity to human and mouse CTLDs, similar antigen reactivity to the parent antibody and inhibitory activity against tube formation, comparable to the parent antibody.

Accordingly, the antibody according to the present disclosure may include at least one heavy chain variable region framework selected from the group consisting of SEQ ID NOS: 17 to 20. In addition, the antibody according to the present disclosure may include at least one light chain variable region framework selected from the group consisting of SEQ ID NOS: 21 to 24.

The antibody according to the present disclosure may be a deglycosylated antibody. Regarding the term "glycosylation" as used herein, in the case of a glycoprotein, for example, an antibody, whether or not glycosylation occurs, and structures or morphologies of glycoforms may be changed depending on the type of host cells, methods for manipulating recombinants and culture conditions. That is, in the process of producing glycoproteins, various types of glycoforms are produced depending on differences in glycoform structures or amounts of constituent saccharides of glycoforms, or the like, so heterogeneity may be present due to differences in production conditions. Glycoproteins having different glycoform structures are different from natural type in terms of *in vivo* kinetics and tissue distribution, or are antagonistic to the natural type, which may result in adverse reactions, and act as an antigen when administered continuously for a long time, which may cause immunological problems. As such, glycoforms may be key factors that can affect pharmacological effects and *in vivo* kinetics.

In an attempt to regulate glycoforms, the antibody according to the present disclosure may be an antibody obtained by deglycosylating an antibody substituted with an omalizumab (Xolair®) framework. The deglycosylated antibody is intended to include a non-deglycosylated immunoglobulin Fc fragment and, for example, an N-linked glycosylation site or an O-linked glycosylation site may be modified or removed. N-linked glycosylation may mean the attachment of the sugar (glycan) chain to a side chain of an asparagine (Asn) residue, and O-linked glycosylation may mean the attachment of one of N-acetyl-galactosamine, galactose or xylose to hydroxyamino acid, more commonly, serine or threonine.

The modification or removal of glycosylation sites may be carried out by an ordinary method such as a chemical, enzymatic or genetically engineered method using microorganisms, but is not limited thereto. In a specific embodiment of the present disclosure, the presence of one N-glycosylation site in L-CDR1 of an antibody is predicted, a random scFv library at a probable N-glycosylation site is produced using (NNK)₃, and a deglycosylated antibody is then screened by phage display technology.

Based on this trial, the present disclosure provides an antibody comprising a light chain variable region comprising CDR1 of TGSSSNIGXXXVT (SEQ ID NO: 1), wherein each of the amino acids at positions 9, 10 and 11 of the SEQ ID NO: 1 is any one selected from the group consisting of R, C, G, A, T, W, S, N, and V.

In one embodiment, each of the amino acids at positions 9, 10 and 11 of the SEQ ID NO: 1 may be RCG, ATA, WSN or AVV. When the amino acid is RCG, the antibody may include a light chain variable region including CDR1 of TGSSSNIGRCGVT (SEQ ID NO: 2), when the amino acid is ATA, the antibody may include a light chain variable region including CDR1 of TGSSSNIGATAVT (SEQ ID NO: 3), when the amino acid is WSN, the antibody may include a light chain variable region including CDR1 of TGSSSNIGWSNVT (SEQ ID NO: 4), and when the amino acid is AVV, the antibody may include a light chain variable region including CDR1 of TGSSSNIGAVVVT (SEQ ID NO: 5).

According to one embodiment of the present disclosure, it can be seen that the four types of screened deglycosylated antibodies, i.e., deglyco-Cl to deglyco-C4, do not show aggregation and have high final purification yields. In addition, results of investigation as to whether or not characteristics of the four types of screened deglycosylated antibodies are maintained well, as compared with an antibody substituted with a Xolair framework, show that all of the four types of deglycosylated antibodies, i.e., deglyco-Cl to deglyco-C4 maintain cross-reactivity to human and mouse CTLDs.

The amino acids at positions 9, 10 and 11 of the SEQ ID NO: 1 are preferably RCG. When amino acids at positions 9, 10 and 11 of the SEQ ID NO: 1 are RCG, the antibody according to the present disclosure may include a light chain variable region including CDR1 of TGSSSNIGRCGVT (SEQ ID NO: 2).

According to one embodiment of the present disclosure, the antibody comprising a light chain variable region comprising CDR1 of SEQ ID NO: 2 is represented by "deglyco-C1". In order to identify whether or not efficacies of the deglycosylated antibodies are maintained, tube formation ability is observed. As a result, it can be seen that deglyco-Cl exhibits similar inhibitory activity against tube formation to the antibody (clone 1 IgG) CDR-grafted to the framework region of the omalizumab antibody. Furthermore, deglycosylation degree of deglyco-Cl is identified. As a result, it can be seen that glycosylation patterns are removed and disappeared.

The term "human antibody" as used herein refers to a molecule that consists entirely of amino acid sequences of all components of human immunoglobulin including CDRs, framework regions and the like. Human antibodies have at least three potential benefits in the treatment of human diseases. First, human antibodies further preferably interact with the human immune system to destroy target cells more effectively by, for example, complement-dependent cytotoxicity (CDC) or antibody dependent cell-mediated cytotoxicity (ADCC). Another benefit is that the human immune system does not recognize the human antibody as being a foreign molecule. Furthermore, the half-lives of human antibodies are similar to those of antibodies endogenously derived from the human circulatory system, even when administered in smaller amounts or with less frequency. The antibody according to the present disclosure is preferably a human monoclonal antibody and is useful for the treatment of angiogenesis-related diseases or cancer, because it has strong affinity to clec14a, preferably, clec14a-CTLD expressed on human endothelial cells which effectively inhibits clec14a-mediated angiogenesis, and because it has low immunogenicity since both heavy chains and light chains are derived from humans.

As used herein, the term "clec14a (C-type lectin domain family 14, member A)" means a member of C-type lectin/C-type lectin-like domain (CTL/CTLD) superfamily. Clec14a is a type I transmembrane protein, the extracellular domain of which consists of a C-type lectin-like domain (CTLD), a series of epidermal growth factor-like domains, and a sushi-like domain. Information associated with clec14a can be obtained from certified database such as NCBI GenBank. For example, human clec14a may have Gene ID No 161198, but is not limited thereto. "C-type lectin-like domain (CTLD) of clec14a (C-type lectin domain family 14, member A)" may be also referred to as "clec14a-CTLD" or "clec14a-CTLD", wherein all thereof may be interchangeably used with one another.

The term "epitope" as used herein refers to a site that determines antigen-specificity, which may be interchangeably used with an antigenic determinant or an antigen determining site.

In addition, according to the present disclosure, in order to improve affinity of the deglycosylated antibody, major amino acid residues of HCDR3 relating to antigen-antibody reactivity can be modified. For example, determination is conducted by alanine scanning, randomized antibody (ab) libraries with induced random mutation are constructed, and antibodies having higher reactivity than the parent antibody are screened. Respective antibodies capable of maintaining characteristics and functions better than the parent antibody can be screened by yeast surface display and phage display technologies.

In one embodiment, the antibody according to the present disclosure may include a light chain variable region including CDR1 selected from the group consisting of SEQ ID NOS: 2 to 5. The antibody according to the present disclosure may include a light chain variable region further including CDR2 of SEQ ID NO: 15 and CDR3 of SEQ ID NO: 16, in addition to CDR1 described above. The antibody according to the present disclosure may include: light chain CDR1 of SEQ ID NO: 2, light chain CDR2 of SEQ ID NO: 15 and light chain CDR3 of SEQ ID NO: 16; light chain CDR1 of SEQ ID NO: 3, light chain CDR2 of SEQ ID NO: 15 and light chain CDR3 of SEQ ID NO: 16; light chain CDR1 of SEQ ID NO: 4, light chain CDR2 of SEQ ID NO: 15 and light chain CDR3 of SEQ ID NO: 16; or light chain CDR1 of SEQ ID NO: 5, light chain CDR2 of SEQ ID NO: 15 and light chain CDR3 of SEQ ID NO: 16.

The antibody according to the present disclosure may include a light chain variable region selected from the group consisting of SEQ ID NOS: 7 to 10. The antibody according to the present disclosure may include a light chain variable region including CDR1 OF SEQ ID NO: 2. The antibody may include a light chain variable region including SEQ ID NO: 7.

In one embodiment, the antibody according to the present disclosure may include a heavy chain variable region including CDR3 selected from the group consisting of SEQ ID NO: 13, and SEQ ID NOS: 25 to 60. The antibody may include a heavy chain variable region further including CDR1 OF SEQ ID NO: 11 and CDR2 OF SEQ ID NO: 13, in addition to the CDR3. The antibody according to the present disclosure may include: heavy chain CDR1 of SEQ ID NO: 11, heavy chain CDR2 of SEQ ID NO: 12 and heavy chain CDR3 of SEQ ID NO: 13; heavy chain CDR1 of SEQ ID NO: 11, heavy chain CDR2 of SEQ ID NO: 12 and heavy chain CDR3 of SEQ ID NO: 25; heavy chain CDR1 of SEQ ID NO: 11, heavy chain CDR2 of SEQ ID NO: 12 and heavy chain CDR3 of SEQ ID NO: 37; or heavy chain CDR1 of SEQ ID NO: 11, heavy chain CDR2 of SEQ ID NO: 12 and heavy chain CDR3 of SEQ ID NO: 40.

The antibody may include a heavy chain variable region selected from the group consisting of SEQ ID NOS: 6, and 61 to 96. The antibody according to the present disclosure may include a heavy chain variable region including CDR3 of SEQ ID NO: 13 and/or a heavy chain variable region of SEQ ID NO: 6.

The antibody according to the present disclosure may comprise:
a light chain CDR1 of SEQ ID NO: 2, a light chain CDR2 of SEQ ID NO: 15 and a light chain CDR3 of SEQ ID NO: 16, a heavy chain CDR1 of SEQ ID NO: 11, a heavy chain CDR2 of SEQ ID NO: 12 and a heavy chain CDR3 of SEQ ID NO: 13;
a light chain CDR1 of SEQ ID NO: 3, a light chain CDR2 of SEQ ID NO: 15 and a light chain CDR3 of SEQ ID NO: 16, a heavy chain CDR1 of SEQ ID NO: 11, a heavy chain CDR2 of SEQ ID NO: 12 and a heavy chain CDR3 of SEQ ID NO: 13;
a light chain CDR1 of SEQ ID NO: 4, a light chain CDR2 of SEQ ID NO: 15 and a light chain CDR3 of SEQ ID NO: 16, a heavy chain CDR1 of SEQ ID NO: 11, a heavy chain CDR2 of SEQ ID NO: 12 and a heavy chain CDR3 of SEQ ID NO: 13;
a light chain CDR1 of SEQ ID NO: 5, a light chain CDR2 of SEQ ID NO: 15 and a light chain CDR3 of SEQ ID NO: 16, a heavy chain CDR1 of SEQ ID NO: 11, a heavy chain CDR2 of SEQ ID NO: 12 and a heavy chain CDR3 of SEQ ID NO: 13;
a light chain CDR1 of SEQ ID NO: 2, a light chain CDR2 of SEQ ID NO: 15 and a light chain CDR3 of SEQ ID NO: 16, a heavy chain CDR1 of SEQ ID NO: 11, a heavy chain CDR2 of SEQ ID NO: 12 and a heavy chain CDR3 of SEQ ID NO: 25;
a light chain CDR1 of SEQ ID NO: 2, a light chain CDR2 of SEQ ID NO: 15 and a light chain CDR3 of SEQ ID NO: 16, a heavy chain CDR1 of SEQ ID NO: 11, a heavy chain CDR2 of SEQ ID NO: 12 and a heavy chain CDR3 of SEQ ID NO: 37; or
a light chain CDR1 of SEQ ID NO: 2, a light chain CDR2 of SEQ ID NO: 15 and a light chain CDR3 of SEQ ID NO: 16, a heavy chain CDR1 of SEQ ID NO: 11, a heavy chain CDR2 of SEQ ID NO: 12 and a heavy chain CDR3 of SEQ ID NO: 40.

The antibody according to the present disclosure may include:
a light chain variable region of SEQ ID NO: 7 and a heavy chain variable region of SEQ ID NO: 6;
a light chain variable region of SEQ ID NO: 8 and a heavy chain variable region of SEQ ID NO: 6;
a light chain variable region of SEQ ID NO: 9 and a heavy chain variable region of SEQ ID NO: 6;
a light chain variable region of SEQ ID NO: 10 and a heavy chain variable region of SEQ ID NO: 6;
a light chain variable region of SEQ ID NO: 7 and a heavy chain variable region of SEQ ID NO: 61;
a light chain variable region of SEQ ID NO: 7 and a heavy chain variable region of SEQ ID NO: 73; or
a light chain variable region of SEQ ID NO: 7 and a heavy chain variable region of SEQ ID NO: 76.

When the antibody of the present disclosure includes a constant domain, it can be derived from IgG, IgA, IgD, IgE or IgM, or a combination or hybrid thereof.

The term "combination" as used herein means that a polypeptide encoding a single chain immunoglobulin Fc fragment having the identical origin is linked to a single chain polypeptide having a different origin to produce a dimer or multimer. This dimer or multimer may be produced from two or more constant domains selected from the group consisting of constant domains of IgG, IgA, IgD, IgE and IgM.

The term "hybrid" as used herein means that sequences encoding two or more heavy chain constant domains having different origins are present in a single chain immunoglobulin heavy chain constant domain. For example, a domain hybrid may be composed of one to four domains selected from the group consisting of CH1, CH2, CH3 and CH4 of IgG, IgA, IgD, IgE and IgM. In addition, a combination of hybrids may be formed from heavy chain constant domains of IgG subtypes, i.e., IgG1, IgG2, IgG3 and IgG4. The combination of hybrids is as defined above.

In addition, the antibody of the present disclosure may further include a light chain constant region, which may be derived from a lambda (λ) or kappa (κ) light chain.

Preferably, the antibody may be a human monoclonal antibody that binds specifically to not only human clec14a-CTLD, but also mouse clec14a-CTLD to inhibit angiogenesis. The ability of human antibodies to function in both humans and mice, i.e., cross-reactivity, provides an advantage of enabling the human antibodies to be applicable to a preclinical study in mice.

In another aspect, the present disclosure is directed to a composition containing the antibody for suppressing angiogenesis, or a pharmaceutical composition containing the antibody for preventing or treating an angiogenesis-related disease.

Since the antibody regarding the present disclosure is capable of effectively suppressing angiogenesis, a composition containing the antibody as an effective ingredient is also useful for suppressing angiogenesis, and is also useful for preventing or treating an angiogenesis-related disease.

The term "suppression of angiogenesis" as used herein means suppression of formation or growth of new blood vessels from previously existing vessels. For the purposes of the present disclosure, suppression of angiogenesis can be accomplished by suppressing cell migration or intercellular contacts, more preferably, by suppressing clec14a-mediated cell migration, clec14a-mediated intercellular contacts, HUVEC migration, tube formation, or clec14a CLTD-CLTD complex formation.

The term "angiogenesis-related disease" as used herein means a disease that is related to incidence or development of angiogenesis. Any disease may fall within the scope of the angiogenesis-related disease without limitation so long as it can be treated with the antibody. Examples of the angiogenesis-related disease include, but are not limited to, cancer, metastasis, diabetic retinopathy, retinopathy of prematurity, corneal graft rejection, macular degeneration, neovascular glaucoma, erythrosis, proliferative retinopathy, psoriasis, hemophilic arthritis, microvessel formation of atherosclerotic plaques, keloid, wound granulation, vascular adhesion, rheumatoid arthritis, osteoarthritis, autoimmune diseases, Crohn's disease, restenosis, atherosclerosis, intestinal adhesions, cat scratch disease, ulcer, liver cirrhosis, nephritis, diabetic nephropathy, diabetes mellitus, inflammatory diseases and neurodegenerative diseases. In addition, the cancer is selected from the group consisting of esophageal cancer, stomach cancer, large intestine cancer, rectal cancer, oral cancer, pharynx cancer, larynx cancer, lung cancer, colon cancer, breast cancer, uterine cervical cancer, endometrial cancer, ovarian cancer, prostate cancer, testis cancer, bladder cancer, renal cancer, liver cancer, pancreatic cancer, bone cancer, connective tissue cancer, skin cancer, brain cancer, thyroid cancer, leukemia, Hodgkin's lymphoma, lymphoma and multiple myeloid blood cancer, but is not limited thereto.

The term "prevention" or "prophylaxis" as used herein refers to any action causing the suppression or delay of the onset of a disease of interest by administering the antibody or composition according to the present disclosure. The term "treatment" or "therapy" as used herein refers to any action causing an improvement in symptoms of a disease of interest or the beneficial alteration of the symptoms by administering the antibody or composition according to the present disclosure.

The composition including the antibody of the present disclosure is preferably a pharmaceutical composition and may further contain an appropriate vehicle, excipient or diluent typically used in the art.

The pharmaceutical composition containing a pharmaceutically acceptable vehicle may be in a variety of oral or parenteral dosage forms such as tablets, pills, powders, granules, capsules, suspensions, internal use solutions, emulsions, syrups, sterile aqueous solutions, non-aqueous solutions, lyophilizates and suppositories. In this regard, the pharmaceutical composition of the present disclosure may be formulated in combination with a diluent or excipient such as a filler, a thickener, a binder, a wetting agent, a disintegrant, a surfactant or the like. Solid formulations for oral administration may take the form of tablets, pills, powders, granules, capsules and the like. Regarding these solid suspensions, the compound of the present disclosure may be formulated in combination with one or more excipients such as starch, calcium carbonate, sucrose, lactose or gelatin. In addition to a simple excipient, a lubricant such as magnesium stearate or talc may be further used. Liquid formulations for oral administration may include suspensions, solutions, emulsions, syrups and the like. A simple diluent such as water or liquid paraffin, various excipients such as wetting agents, sweeteners, aromatics, preservatives and the like may be incorporated in the liquid formulations. In addition, the pharmaceutical composition of the present disclosure may be in a parenteral dosage form such as a sterile aqueous solution, a non-aqueous solvent, a suspension, an emulsion, a lyophilizate, a suppository or the like. Injectable propylene glycol, polyethylene glycol, vegetable oils such as olive oil and esters such as ethyl oleate may be suitable for non-aqueous solvents and suspensions. Basic ingredients of the suppositories include Witepsol, macrogol, Tween 61, cacao butter, laurin butter and glycerogelatin.

The composition of the present disclosure is administered in a pharmaceutically effective amount. The term "pharmaceutically effective amount" as used herein refers to an amount of a pharmaceutical composition for treating a disease which is sufficient at a reasonable benefit/risk ratio applicable to all medical treatments. The effective amount may be changed depending on a variety of factors including severity of the disease to be treated, age and gender of the patient, type of disease, drug activity, sensitivity to the drug, administration time, administration route, excretion rate, treatment period, coadministration of drugs and other parameters known in the art. The composition of the present disclosure may be administered alone or in combination with other therapeutics. In this case, the composition of the present disclosure may be administered sequentially or simultaneously with conventional therapeutics. In addition, the composition may be administered in a single dose or may be divided into multiple doses. When thoroughly taking into consideration these factors, it is important to administer a minimal amount sufficient to achieve maximum efficacy without side effects, and this dosage can be readily determined by those skilled in the art. The dosage of the pharmaceutical composition of the present disclosure is not particularly limited, but depends on a variety of factors including health conditions and body weight of patient, severity of disease, type of drug, administration route and administration period. The composition may be administered in a single dose or multiple doses daily to mammals including rats, mice, domestic animals, humans and the like, via a typically acceptable route, for example, orally, rectally, intravenously, subcutaneously, intrauterinely, or intracerebrovascularly.

In another aspect, the present disclosure is directed to a method for suppressing angiogenesis or a method for preventing or treating an angiogenesis-related disease, each method including administering the antibody or the composition to a subject in need thereof.

The antibody, the composition and suppression of angiogenesis are as described above.

More specifically, the suppression method according to the present disclosure includes administering a pharmaceutical composition at a pharmaceutically effective amount to a subject in need of suppression of angiogenesis. The subject may be a mammal such as a dog, cow, horse, rabbit, mouse, rat, chicken or human, but is not limited thereto. The pharmaceutical composition may be administered parenterally, subcutaneously, intraperitoneally, intrapulmonarily or intranasally, or by an appropriate method including intralesional administration for local treatment, if needed. A preferred dosage of the pharmaceutical composition of the present disclosure may depend on a variety of factors including health conditions and body weight of the subject, severity of the disease, type of drug, administration route and administration period and can be readily determined by those skilled in the art.

In another aspect, the present disclosure is directed to a method for preventing or treating cancer including administering a pharmaceutical composition including the antibody for preventing or treating cancer, or the antibody, to a subject in need thereof. The terms "antibody", "prevention" and "treatment" are as mentioned above.

Any cancer can be applied without limitation so long as it can be treated with the antibody of the present disclosure. Cancer in which progression of clec14a-mediated tumor occurs is preferred. Specifically, the antibody of the present disclosure can prevent the onset or progression of cancer by suppressing angiogenesis. Examples of the cancer include, but are not limited to, esophageal cancer, stomach cancer, large intestine cancer, rectal cancer, oral cancer, pharynx cancer, larynx cancer, lung cancer, colon cancer, breast cancer, uterine cervical cancer, endometrial cancer, ovarian cancer, prostate cancer, testis cancer, bladder cancer, renal cancer, liver cancer, pancreatic cancer, bone cancer, connective tissue cancer, skin cancer, brain cancer, thyroid cancer, leukemia, Hodgkin's lymphoma, lymphoma and multiple myeloid blood cancer.

In addition, the antibody of the present disclosure may be used in combination with other antibodies or biologically active agents or substances for various purposes.

Hereinafter, the present disclosure will be described more in detail with reference to examples. However, it is obvious to those skilled in the art that these examples are provided only for illustration of the present disclosure and should not be construed as limiting the scope of the present disclosure.

### Example 1: Formation of antibodies with improved stability by in silico-based CDR grafting

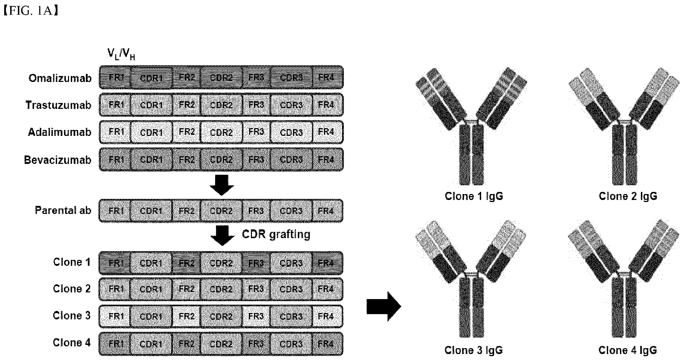

It was reported in WO 2013/187556 that the clec14a-CTLD parent antibody can specifically regulate angiogenesis characteristics *in vitro.* In this regard, it was found that the parent antibody (clone 1 of WO 2013/187556) shows significant aggregation during purification and an additional optimal process was needed to improve an antibody yield. Accordingly, *in silico-based* CDR grafting was conducted. Six CDRs in heavy and light chain variable regions of the parent antibody (Table 1) were grafted to respective framework regions of four types of therapeutic antibodies approved by FDA, i.e., adalimumab (Humira®), omalizumab (Xolair®), trastuzumab (Herceptin®), bevacizumab (Avastin®) (FIG. 1A).

**[Table 1] Six CDRs in heavy and light chain variable regions of parent antibody**

| Types | CDR | Sequence | SEQ ID NO: |
|---|---|---|---|
| VH | CDR1 | GFTFSGYDMS | SEQ ID NO: 11 |
| | CDR2 | GIYPDGGNTYYADSVKG | SEQ ID NO: 12 |
| | CDR3 | GATWWVLGPFDY | SEQ ID NO: 13 |
| VL | CDR1 | TGSSSNIGNNSVT | SEQ ID NO: 14 |
| | CDR2 | ADSHRPS | SEQ ID NO: 15 |
| | CDR3 | GAWDDSLSGYV | SEQ ID NO: 16 |

Developability indices (DIs) of the four CDR-grafted antibodies (clone 1 to 4 IgGs) and the parent antibody were compared using *in silico-based* analysis and Discovery Studio 3.1 Software available from Accelrys Inc. DI is a rapid *in silico* prediction parameter used to evaluate a monoclonal antibody according to aggregation characteristics. As DI increases, aggregation degree increases.

As can be seen from Table 2, among the parent antibody and the four CDR-grafted antibodies, clone 1 IgG having the framework region (specific sequence of the framework region is shown in Table 3) of omalizumab (Xolair®) exhibits lower DI than the parent antibody and other CDR-grafted antibodies (clone 2-4 IgGs), which means that clone 1 IgG has excellent stability.

**[Table 2] Summary of theoretical solubility of parent antibody IgG and CDR-grafted IgG antibodies**

| Antibodies | Rank | Developability Index (DI) |
|---|---|---|
| Clone 1 IgG | 1 | 156.13 |
| Clone 2 IgG | 2 | 170.17 |
| Clone 3 IgG | 3 | 181.75 |
| Clone 4 IgG | 4 | 182.46 |
| Parental IgG | 5 | 244.20 |

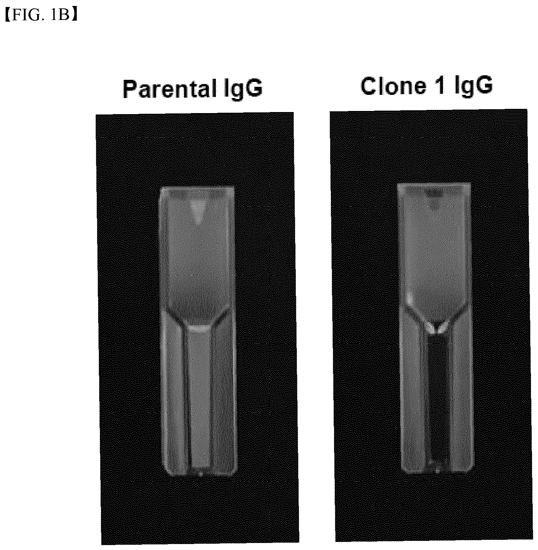

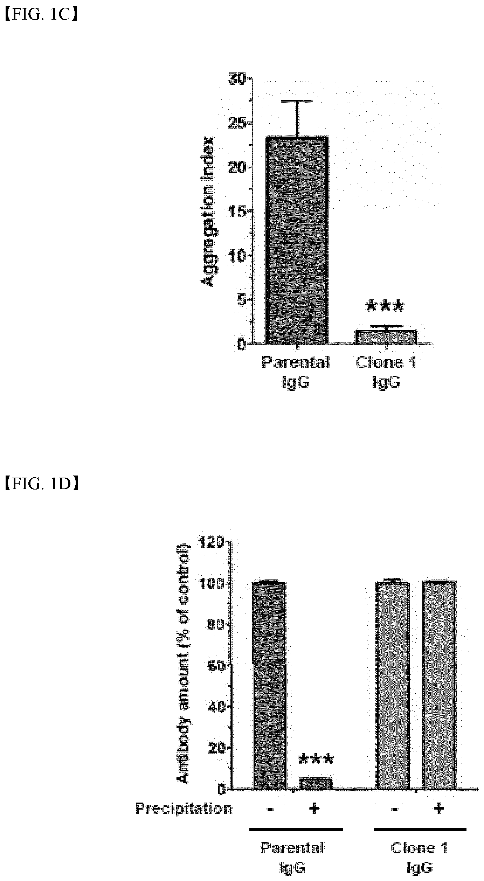

In order to identify improved aggregation degree of clone 1 IgG, aggregation of purified parent antibody IgG and clone 1 IgG was compared by visual observation and then was confirmed by spectrophotometry. Aggregation was observed only in the parent antibody IgG, and was not visually observed in clone 1 IgG (FIG. 1B). Aggregation indices of the parent antibody IgG and clone 1 IgG measured by spectrophotometry were about 23.4 and 1.5, respectively, which means that clone 1 IgG has higher solubility than parent antibody IgG (FIG. 1C).

For further comparison in stability between parent antibody IgG and clone 1 IgG, a water-soluble antibody was weighed after aggregates were precipitated and removed by centrifugation. The antibody of about 95% of the parent antibody IgG preparation was aggregated, whereas no antibody aggregation was observed from clone 1 IgG (FIG. 1D). This result suggests that *in silico-based* CDR grafting is a method which is effective for producing an antibody platform with improved stability.

### Example 2: Selection (screening) of optimal antibodies by continuous deglycosylation process and functional separation

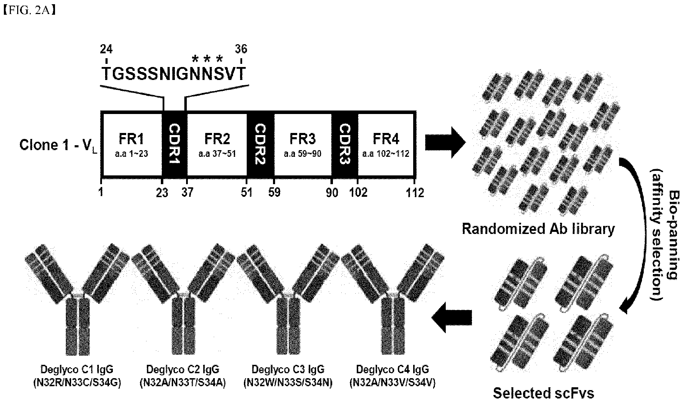

Heterogeneity in glycosylation of therapeutic proteins can have significant impacts on characteristics and qualities in the development and production of biopharmaceuticals. Predicted N-glycosylation sites in light chain CDR1 of clone 1 IgG were investigated through glycosylation evaluation of *in silico-based* clone 1 IgG. In order to remove such predicted N-glycosylation site, a semi-synthetic antibody library having random mutation at the N-glycosylation site was established and deglycosylation process was then conducted. Specifically, semi-synthetic scFvs (single chain variable fragments) randomized with NNK trinucleotide oligonucleotides were established and were subjected to continuous bio-panning by phage display technology including human and mouse clec14a-CTLDs coated with magnetic beads. Finally, DNAs of the selected scFv clones were sequenced and different amino acid sequences were randomly selected from four types of clones and predicted N-glycosylation sites strongly reactive to both human and mouse clec14a-CTLDs (hclec14a-CTLD and mclec14a-CTLD) (FIG. 2A).

**[Table 3]**

| Ab | Types | FR1 | CDR1 | FR2 | CDR2 | FR3 | CDR3 | FR4 |
|---|---|---|---|---|---|---|---|---|
| Deg C1 | VH | | GFTFS GYDMS | WIRQ APGK GLEW VA | GIYPD GGNTY YADSV KG | RFTIS RDDS KNTF YLQM NSLR AEDT AVYY CAR | GATW WVLGP FDY | WGQG TLVT VSS |
| | VL | DIQLT QSPSS LSASV GDRVT ITC | TGSSS NIGRC GVT | WYQQ KPGK APKL LIY | ADSHR PS | GVPS RFSGS GSGT DFTLT ISSLQ PEDFA TYYC | GAWD DSLSG YV | FGQG TKVEI KR |
| Deg C2 | VH | | GFTFS GYDMS | WIRQ APGK GLEW VA | GIYPD GGNTY YADSV KG | RFTIS RDDS KNTF YLQM NSLR AEDT AVYY CAR | GATW WVLGP FDY | WGQG TLVT VSS |
| | VL | DIQLT QSPSS LSASV | TGSSS NIGAT AVT | WYQQ KPGK APKL | ADSHR PS | GVPS RFSGS GSGT | GAWD DSLSG YV | FGQG TKVEI KR |
| | | GDRVT ITC | | LIY | | DFTLT ISSLQ PEDFA TYYC | | |
| Deg C3 | VH | | GFTFS GYDMS | WIRQ APGK GLEW VA | GIYPD GGNTY YADSV KG | RFTIS RDDS KNTF YLQM NSLR AEDT AVYY CAR | GATW WVLGP FDY | WGQG TLVT VSS |
| | VL | DIQLT QSPSS LSASV GDRVT ITC | TGSSS NIGWS NVT | WYQQ KPGK APKL LIY | ADSHR PS | GVPS RFSGS GSGT DFTLT ISSLQ PEDFA TYYC | GAWD DSLSG YV | FGQG TKVEI KR |
| Deg C4 | VH | | GFTFS GYDMS | WIRQ APGK GLEW VA | GIYPD GGNTY YADSV KG | RFTIS RDDS KNTF YLQM NSLR AEDT AVYY CAR | GATW WVLGP FDY | WGQG TLVT VSS |
| | VL | DIQLT QSPSS LSASV GDRVT | TGSSS NIGAV VVT | WYQQ KPGK APKL LIY | ADSHR PS | GVPS RFSGS GSGT DFTLT | GAWD DSLSG YV | FGQG TKVEI KR |
| | | ITC | | | | ISSLQ PEDFA TYYC | | |

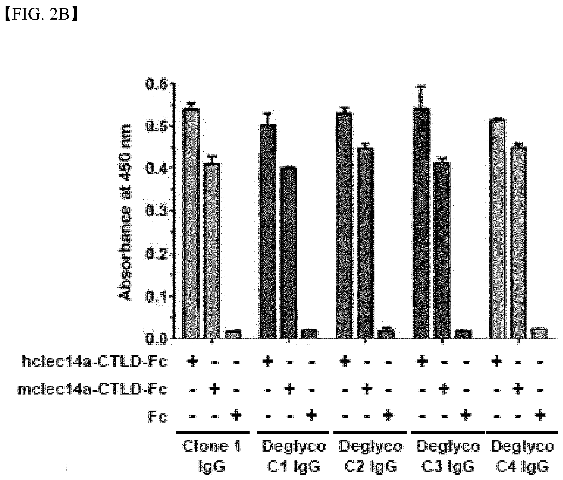

Then, the scFv clones were converted into IgG, expressed in human embryonic kidney (HEK) 293 cells and then purified. Results of ELISA analysis showed that all purified deglycosylated IgGs (deglyco C1-C4 IgGs) specifically bound to both hclec14a-CTLD-Fc and mclec14a-CTLD-Fc, but did not bind to Fc alone, which means that the deglycosylated IgGs are antibodies that bind specifically to clec14a-CTLD (FIG. 2B).

In addition, as can be seen from Table 4, four types of deglycosylated IgGs (deglyco C1-C4 IgGs) were not aggregated at all, and in particular, deglyco C1 and deglyco C2 had high final purification yields.

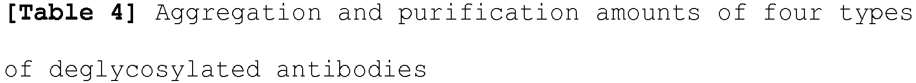

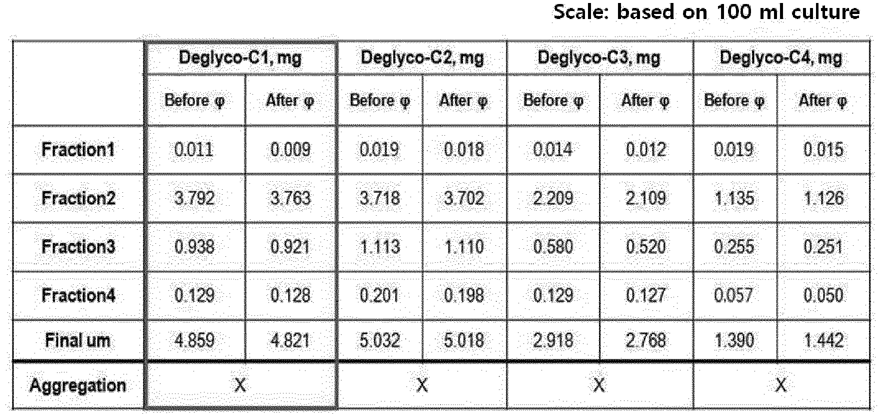

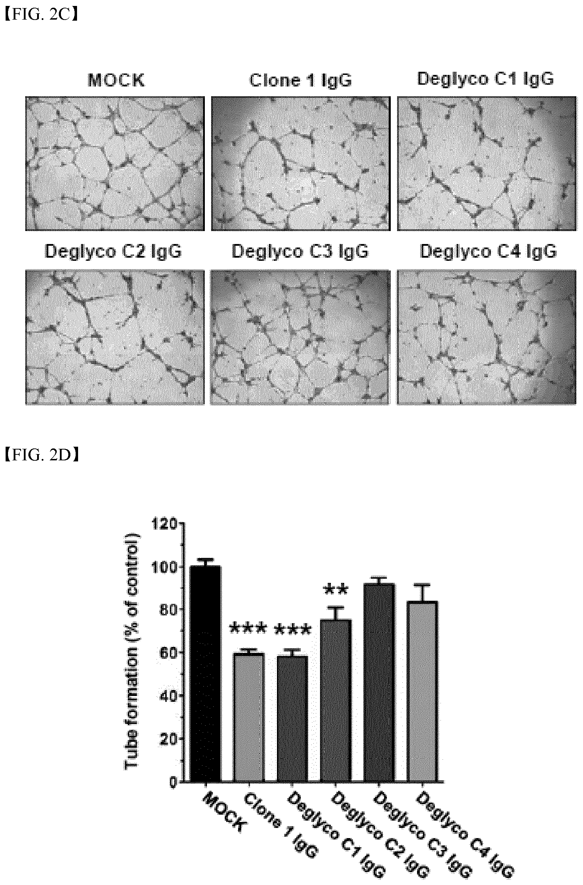

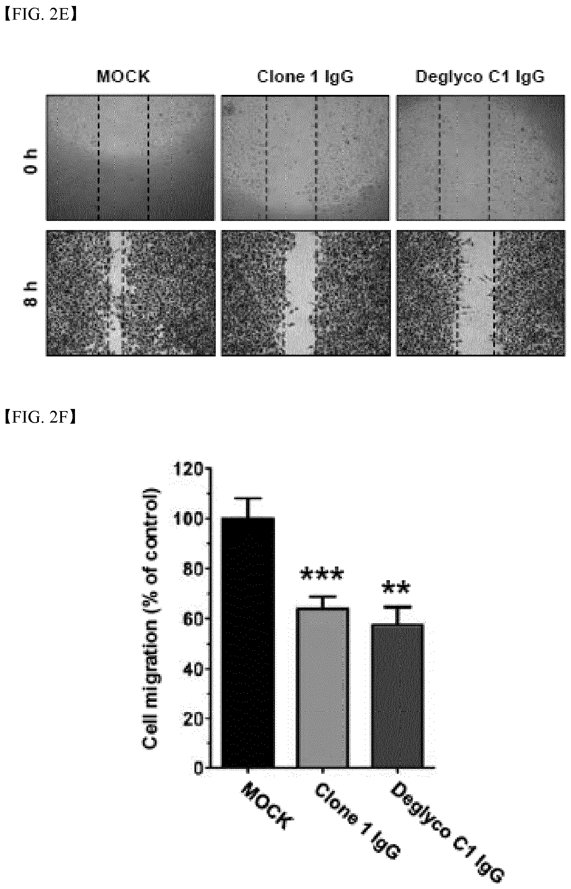

Genetic mutation introduced during antibody engineering induces unpredicted defects, resulting in deterioration in antibody functions. In order to isolate antibodies for suppressing clec14a-mediated angiogenesis, tube formation and endothelial cell migration assay using human umbilical vein endothelial cells (HUVECs) were conducted in the presence or absence of clone 1 IgG and deglyco C1-C4 IgGs. Among the four types of deglyco IgGs, deglyco C1 IgG was selected as a candidate antibody. The reason for this is that deglyco C1 IgG exhibited the most excellent inhibitory activity against HUVEC tube formation (FIGS. 2C and 2D) and migration (FIGS. 2E and 2F). In addition, it can be seen that deglyco C1 IgG includes desired alterations (N32R/N33C/S34G) in the amino acid sequence in light chain CDR1.

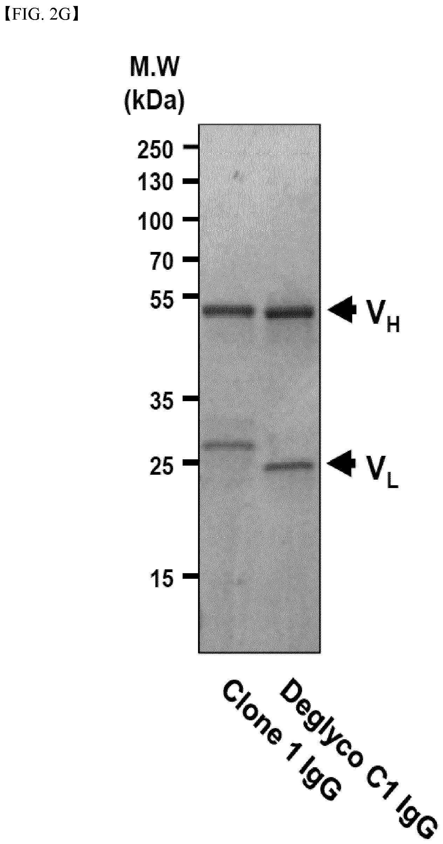

In order to analyze biochemical properties of deglyco C1 IgG, mobility of deglyco C1 IgG and clone 1 IgG was evaluated under reduction conditions using one-dimensional electrophoresis. The molecular weight of deglyco C1 VL was 25 kDa, which is similar to an estimated molecular weight, whereas the molecular weight of clone 1 VL taking the glycosylation form before deglycosylation was higher (FIG. 2G) .

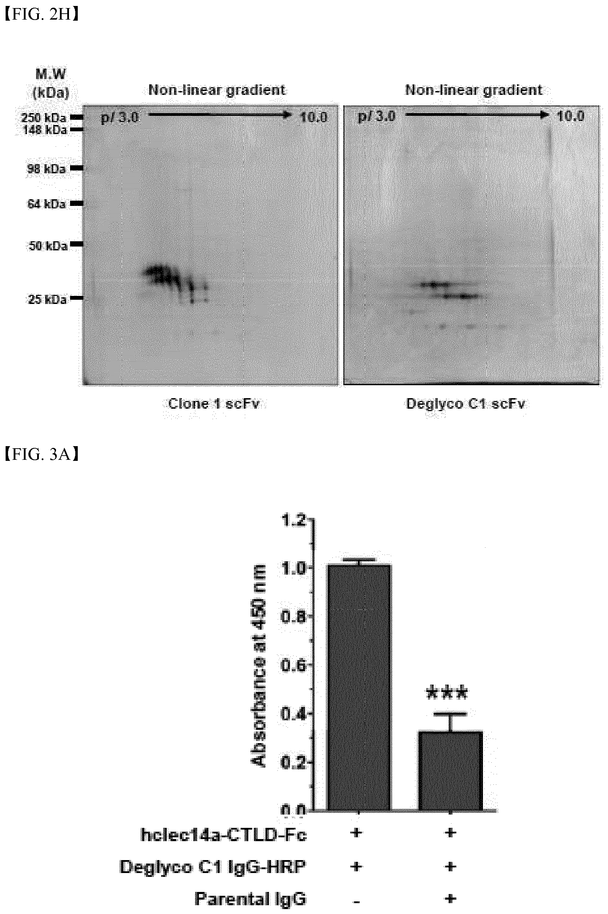

Results of two-dimensional electrophoresis showed that the heterogeneous pattern of clone 1 scFv present within a range lower than an isoelectric point (pI) was not present in deglyco C1 scFv, which means improved homogeneity of deglyco C1 IgG (FIG. 2H).

In conclusion, these results suggest that the selected candidate antibodies are anti-angiogenesis antibodies that effectively suppress pathological angiogenesis and have improved homogeneity.

### Example 3: Biochemical and functional properties of optimized candidate antibodies

In order to identify the position of clec14a-CTLD to which deglyco C1 IgG binds, HRP (horseradish peroxidase)-conjugated deglyco C1 IgG (deglyco C1 IgG-HRP) was produced and competitive ELISA was then conducted.

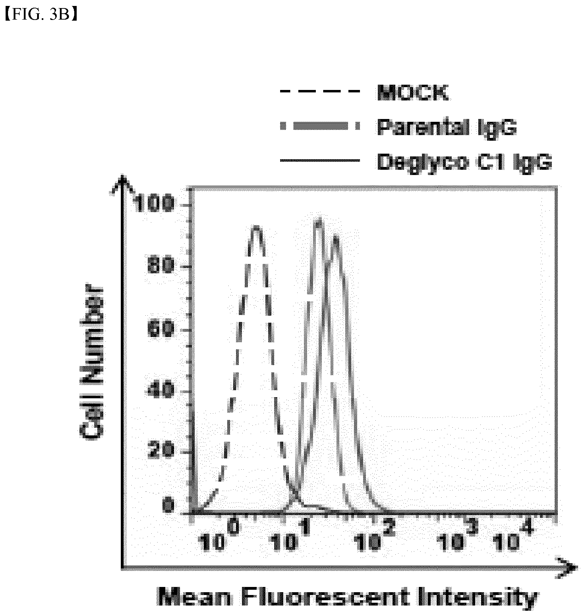

The deglyco C1 IgG-HRP binding to hclec14a-CTLD-Fc was potently competitive by addition of parent antibody IgG, which may mean that the parent antibody and deglyco C1 have a similar clec14a-CTLD binding position (FIG. 3A).

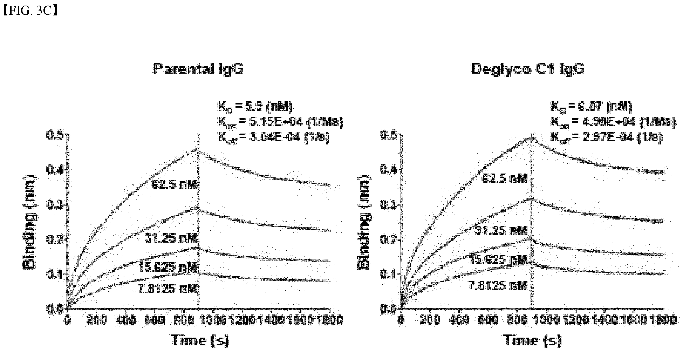

In order to identify whether or not deglyco C1 IgG binds to human endothelial cells, flow cytometry was conducted with HUVECs. Deglyco C1 IgG specifically bound to the surfaces of HUVECs in a similar manner to the parent antibody IgG. In addition, in order to measure binding affinity of C1 IgG to hclec14a-ECD (extracellular domain of human clec14a), binding affinity of antibody-antigen binding was measured in real time. It can be seen that deglyco C1 IgG has a KD constant (equilibrium dissociation constant) of 6 nM, which is similar to that of parent antibody IgG (FIG. 3C) .

Overall, these results indicate that the binding properties of the optimized candidate antibody were maintained to a similar extent to those of the parent antibody IgG, in spite of the two-step optimization process.

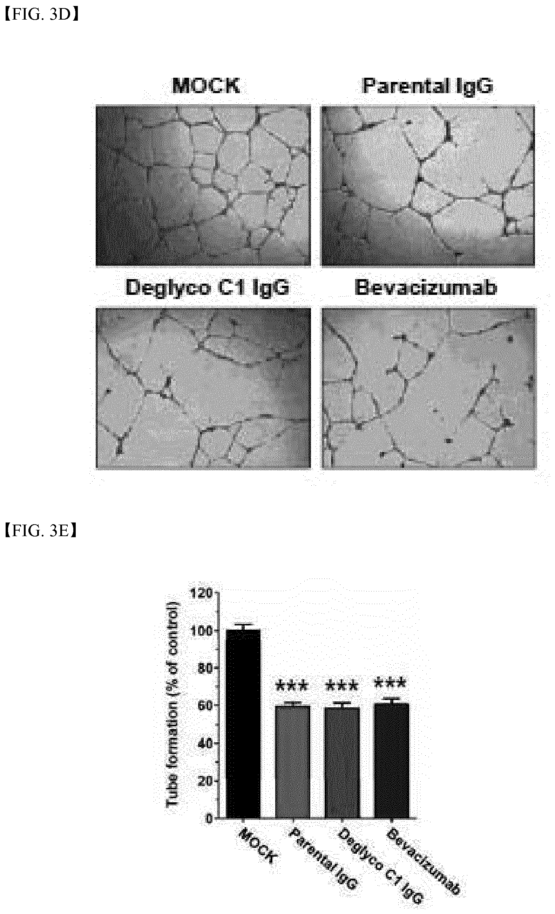

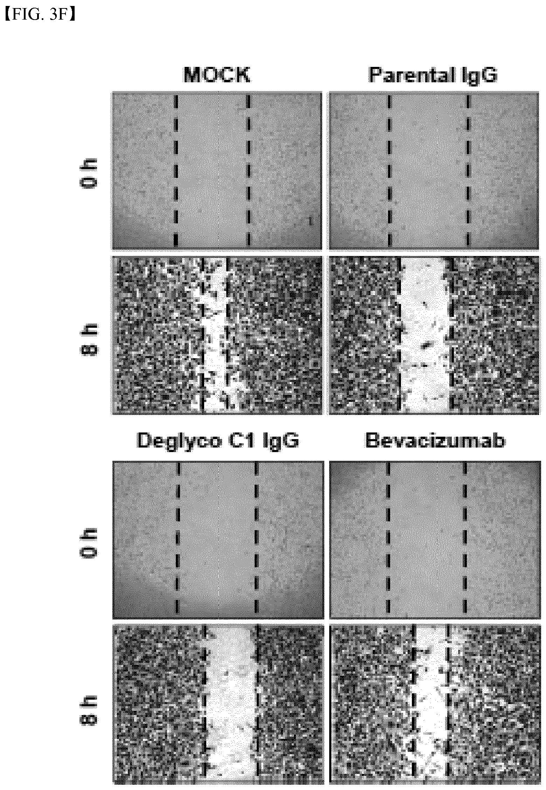

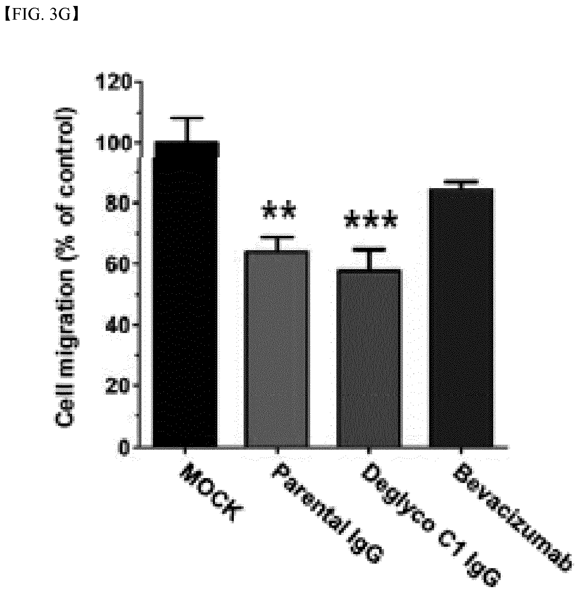

In order to compare *in vitro* angiogenesis suppression effect between deglyco C1 IgG and bevacizumab, tube formation and migration assay were conducted in the presence or absence of parent antibody IgG, deglyco C1 IgG or bevacizumab as a positive control group. Deglyco C1 IgG significantly suppressed HUVEC tube formation (FIGS. 3D and 3E) and inhibited migration to a similar extent to bevacizumab *in vitro* (FIGS. 3F and 3G). This means that the candidate antibody has anti-angiogenesis activity and efficacy, comparable to bevacizumab.

### Example 4: New action mechanisms of optimized candidate antibodies

In order to identify the mechanism by which deglyco C1 IgG acts on angiogenesis, the number of cell aggregates formed from HEK293F cells transfected with wild-type clec14a and cultured in the presence or absence of parent antibody IgG or deglyco C1 IgG was monitored to conduct functional assay on clec14a-mediated cell-cell contacts.

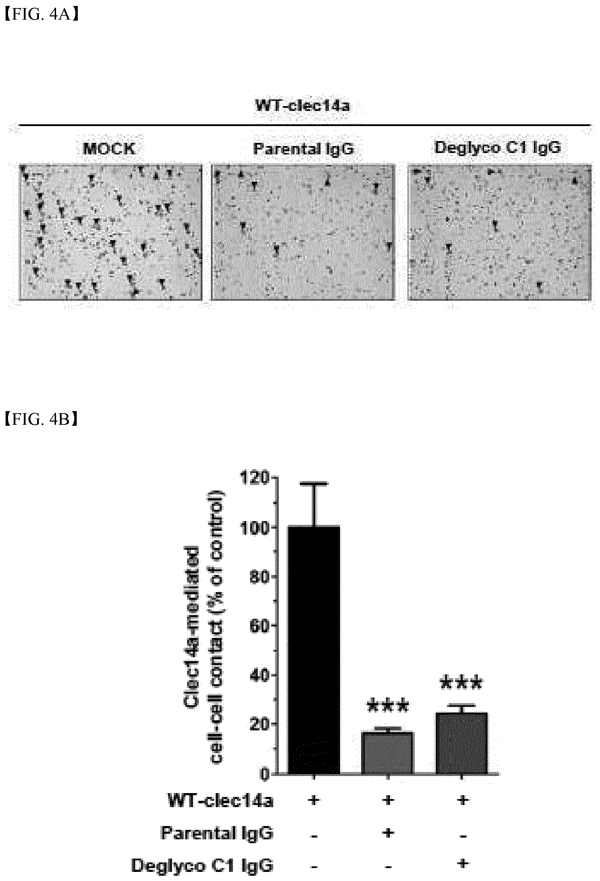

The parent antibody IgG was used as a positive control group to specifically block clec14a-mediated cell-cell contacts. Deglyco C1 IgG had inhibitory efficacy against aggregation of wild-type clec14a-transfected cells in the similar manner to the parent antibody IgG, which suggests that deglyco C1 IgG has inhibitory efficacy on clec14a-mediated cell-cell contacts in angiogenesis (FIGS. 4A and 4B) .

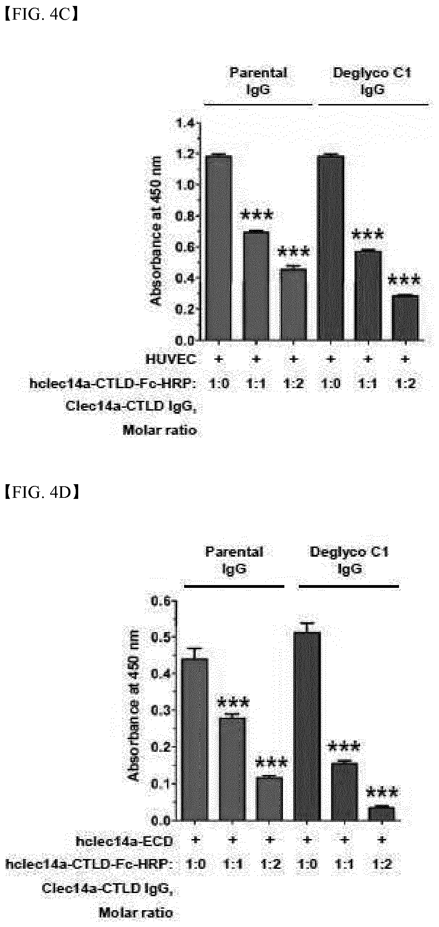

In order to analyze action mechanisms of deglyco C1 IgG at a molecule-scale, specifically, in angiogenesis, HUVECs were treated with hclec14a-CTLD-Fc-HRP (HRP-labeled hclec14a-CTLD-Fc) in the absence or presence of an increasing concentration of parent antibody IgG or deglyco C1 IgG. Deglyco C1 IgG significantly suppressed binding of clec14a-CTLD to HUVECs, in a concentration-dependent manner, like parent antibody IgG (FIG. 4C).

In addition, hclec14a-CTLD-Fc-HRP was incubated together with purified hclec14a-ECD in the presence of an increasing concentration of parent antibody IgG or deglyco C1 IgG, or in the absence thereof. Deglyco C1 IgG directly suppressed molecular interaction between hclec14-CTLD and hclec14a-ECD, in a concentration-dependent manner, like parent antibody IgG (FIG. 4D).

Overall, these results suggest that the produced antibody serves as an interaction blocker specifically suppressing endothelial cell-cell contacts during angiogenesis by directly blocking molecular interaction between clec14a-CTLD-mediated clec14a molecules.

### Example 5: Effects of optimized candidate antibodies on toxicity of endothelial cells and VEGF signaling in endothelial cells

In order to evaluate effects of deglyco C1 IgG on toxicity of endothelial cells, after treatment with deglyco C1 IgG, viability of HUVECs was measured. Viability was measured in accordance with the manufacturer's manual through cell counting Kit-8 (#CK04-13, Dojindo Laboratories, Kumamoto, Japan).

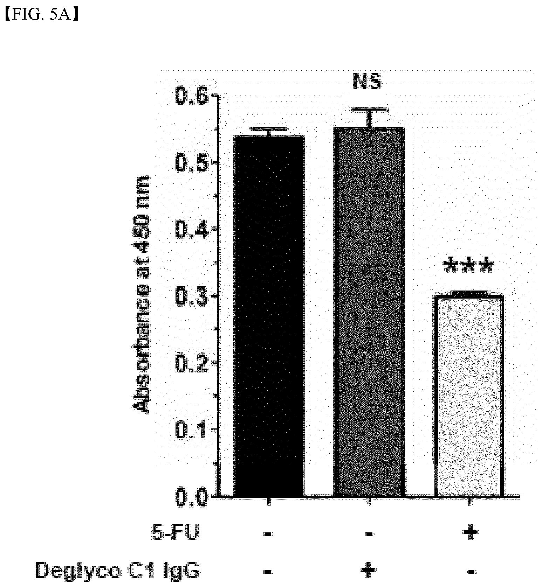

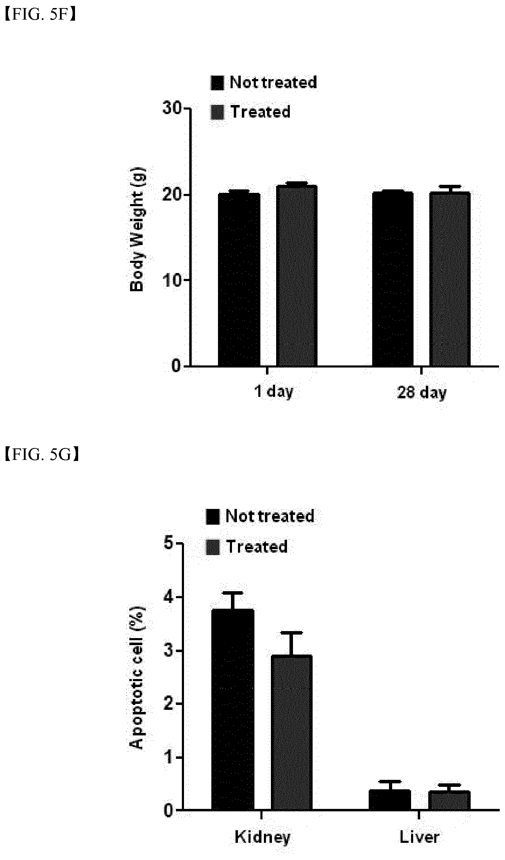

HUVECs did not exhibit cytotoxic effects on these cells, whereas 5-FU (5-fluorouracil) significantly reduced viability of HUVECs (FIG. 5A).

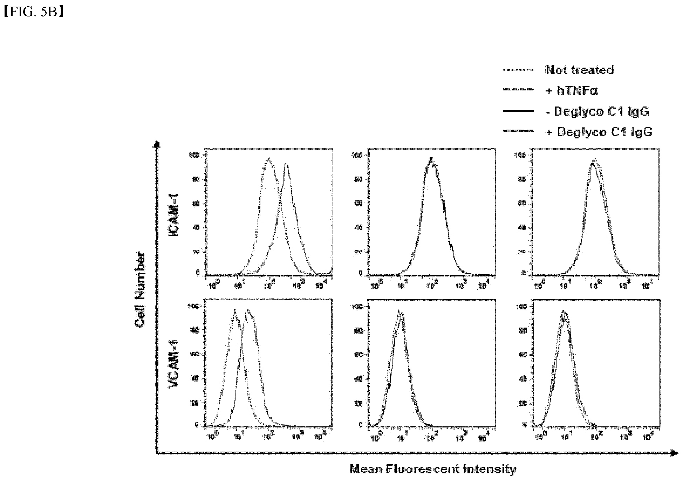

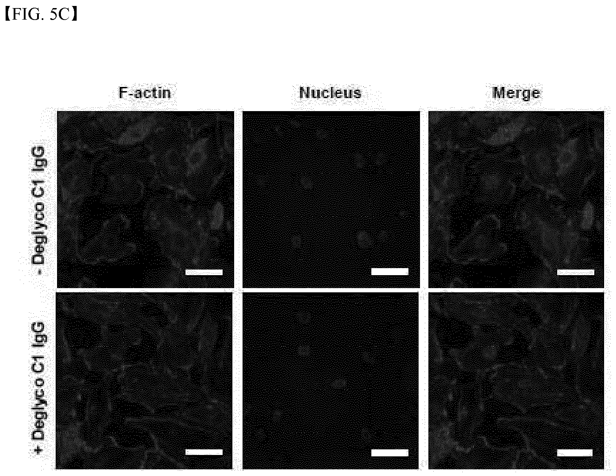

In addition, the morphology of HUVEC was evaluated in the presence or absence of deglyco C1 IgG using immunocytochemistry. Deglyco C1 IgG did not change the morphology of HUVECs (FIG. 5B). In order to evaluate effects of deglyco C1 IgG on the activity of endothelial cells, which is an initial inflammatory response to a harmful stimulus, HUVECs were treated with deglyco C1 IgG, and expression of an endothelial cell activity marker including VCAM-1 (vascular cell adhesion molecule-1) and ICAM-1 (intercellular cell adhesion molecule-1) was measured to monitor HUVEC activity. Human tumor necrosis factor-alpha (hTNFα) was used as a positive control group for endothelial cell activity. Deglyco C1 IgG had almost no impact on HUVEC activity, whereas hTNFα induced HUVEC activity, as expected (FIG. 5C).

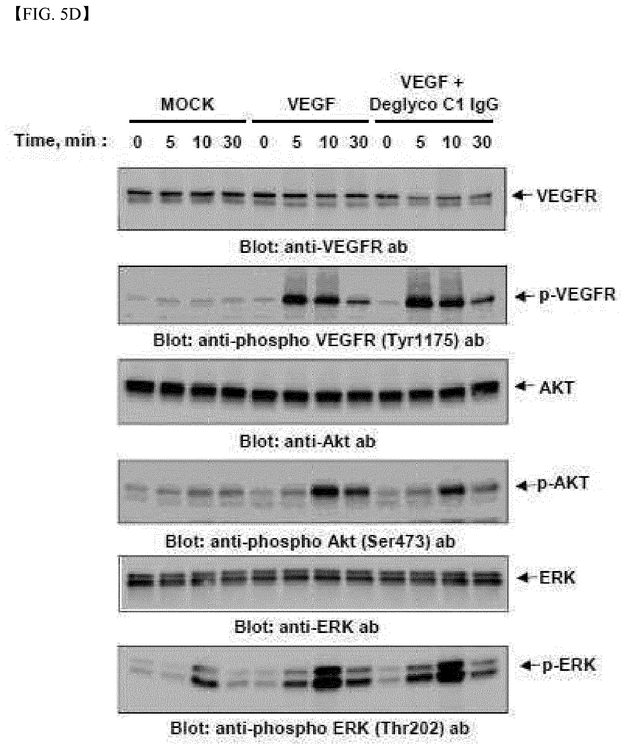

In order to investigate effects of deglyco C1 IgG on VEGF-dependent signaling actions in endothelial cells, VEGF-treated HUVECs were subjected to immunoblot analysis in the presence or absence of deglyco C1 IgG to monitor changes in phosphorylation of VEGFR (VEGF receptor), Akt and ERK (extracellular signal-regulated kinase). Deglyco C1 IgG had almost no effect on VEGF-dependent phosphorylation of VEGFR, Akt and ERK in HUVECs (FIG. 5D).

In order to investigate *in vivo* toxicity of deglyco C1 IgG, deglyco C1 IgG was intravenously injected into a normal mouse twice a week, and hepatic and renal functions, body weight, and apoptosis conditions of hepatic and renal tissues between control groups and experimental groups were then compared. The hepatic function was investigated by measuring serum concentrations of glutamic-oxaloacetic transaminase (GOT), glutamic pyruvic transaminase (GPT), and total bilirubin (TBIL), and the renal function was investigated by measuring the concentrations of blood urea nitrogen (BUN) and creatinine (CRE). Apoptosis was measured by TUNEL staining.

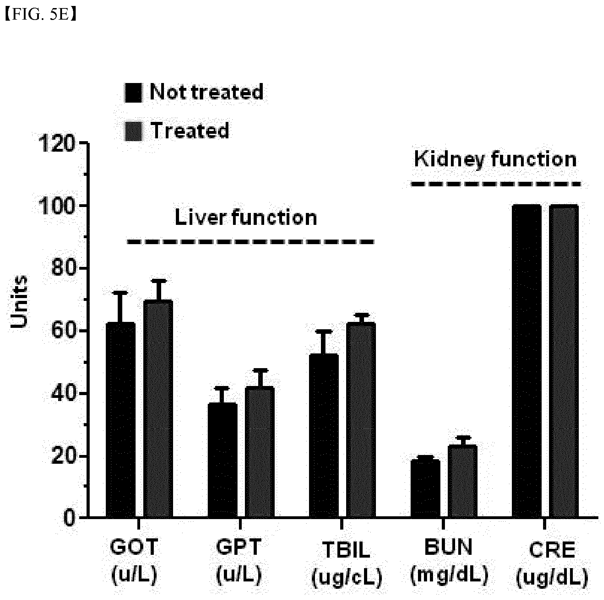

As a result, there was no significant change in hepatic and renal functions (FIG. 5D). There was no significant change in body weight as well (FIG. 5E). In addition, apoptosis conditions were observed (FIG. 5F). These results suggest that deglyco C1 IgG does not induce serious *in vivo* toxicity.

Overall, these results suggest that deglyco C1 IgG neither induces serious endothelial cytotoxicity nor has a negative impact on VEGF-mediated signaling in normal endothelial cells *in vivo.*

### Example 6: Effects of optimized candidate antibodies on VEGF-dependent angiogenesis

In order to investigate *in vitro* effects *of* deglyco C1 IgG on VEGF-dependent angiogenesis, HUVECs were treated with VEGF in the presence or absence of deglyco C1 IgG, and then HUVEC tube formation assay was conducted. 150 µl of Matrigel was added to a 48-well plate and incubated at 37°C for 30 minutes. HUVEC (10⁵) cultured in EGM-2 were collected, seeded on the Matrigel-coated plate and incubated at 37°C for 18 hours in the presence or absence of clone 1 IgG, parent antibody IgG, deglycosylated clec14a-CTLD IgG or bevacizumab. HUVECs cultured in VEGF-containing EBM (endothelial cell basal medium) were seeded to the Matrigel-coated plate and incubated at 37°C for 18 hours in the presence or absence of deglyco C1 IgG (20 µg ml⁻¹).

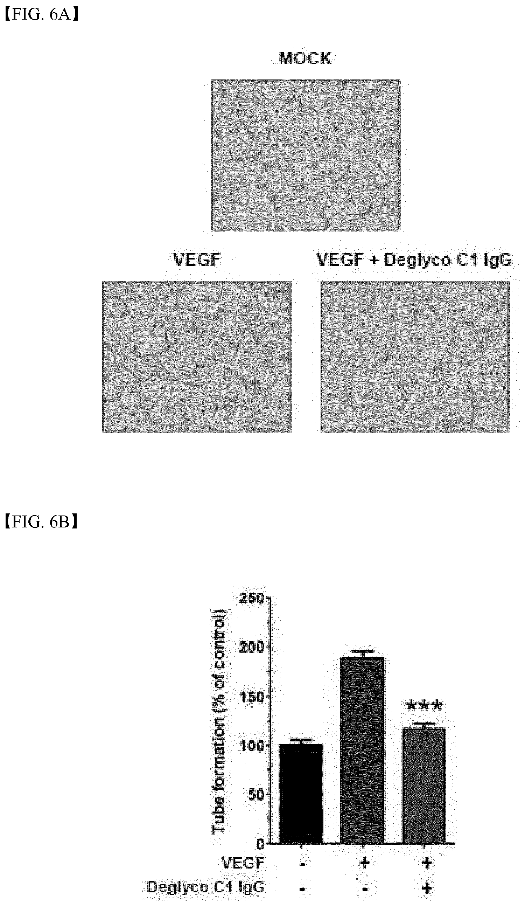

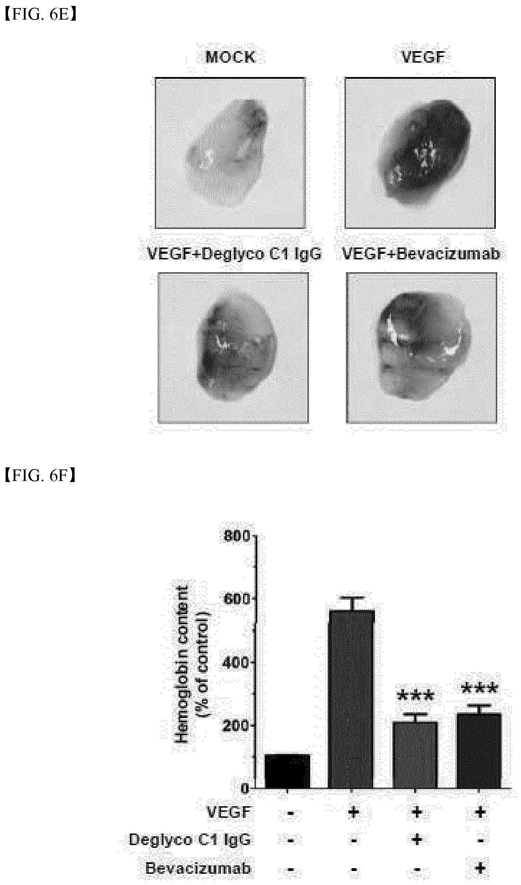

Deglyco C1 IgG removed VEGF-dependent tube formation significantly and almost perfectly (FIG. 6A).

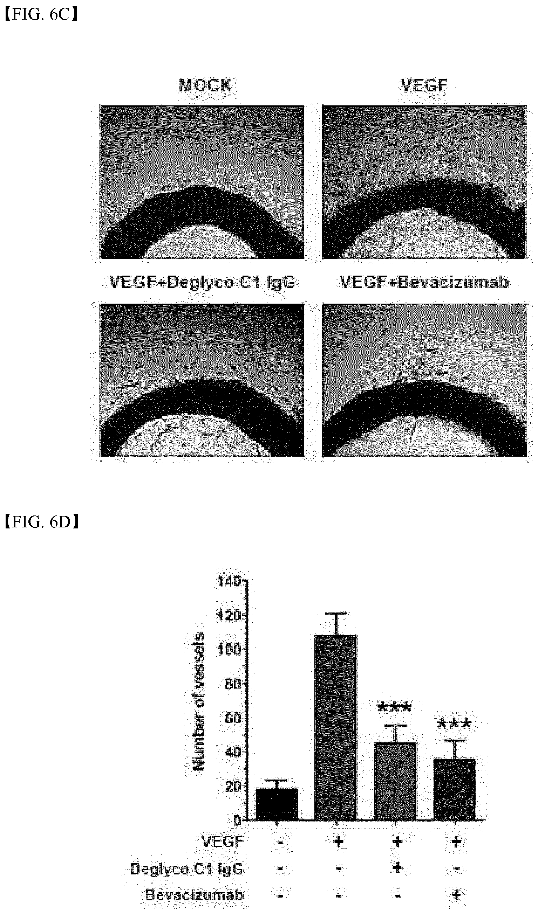

In order to further investigate effects of deglyco C1 IgG on VEGF-dependent angiogenesis, *ex vivo* rat aortic ring assay was conducted in the presence or absence of deglyco C1 IgG and bevacizumab. Blood vessels were almost not grown in the rat aorta in endothelial basal medium (EBM), whereas most blood vessels were grown in the rat aorta under VEGF expression conditions. Furthermore, it was observed that deglyco C1 IgG reduced the number of blood vessels grown in the rat aorta to a similar extent to bevacizumab, when cultured in combination with VEGF (FIGS. 6B and 6C).

In order to observe *in vivo* efficacies of deglyco C1 IgG on VEGF-dependent angiogenesis, a mouse Matrigel model was established, hemoglobin level was measured as a marker of microvessel formation, and inhibitory activities against microvessel formation of deglyco C1 IgG and bevacizumab were compared by a mouse Matrigel plug assay. Deglyco C1 IgG significantly reduced a hemoglobin content in a similar manner to bevacizumab (FIGS. 6D and 6E).

Overall, these results suggest that the produced antibodies have efficacy of *in vivo* inhibiting VEGF-dependent abnormal angiogenesis.

### Example 7: Effects of optimized candidate antibodies on tumor angiogenesis

In order to investigate correlations between tumor angiogenesis and clec14a, first, xenograft tumor models including SNU182 human liver cell or CFPAC-1 human pancreatic cancer cell carcinomas were established, and immunohistochemistry was conducted on commercially available antibodies of clec14a and CD31, which is a well-known endothelial marker protein. Immunohistochemistry was conducted as follows. HUVECs (5X10⁴) grown on a 0.1% (w/v) gelatin-coated glass cover slip were incubated in the presence or absence of deglyco C1 IgG for 24 hours at 37°C. The cells were immobilized with 4% (w/v) PFA, blocked with PBS supplemented with 5% (w/v) BSA and 0.1% (v/v) Triton X-100 at 37°C for one hour and then incubated in Rhodamine-Phalloidin (1 unit/well) and Hoechst 33258 stains for one hour.

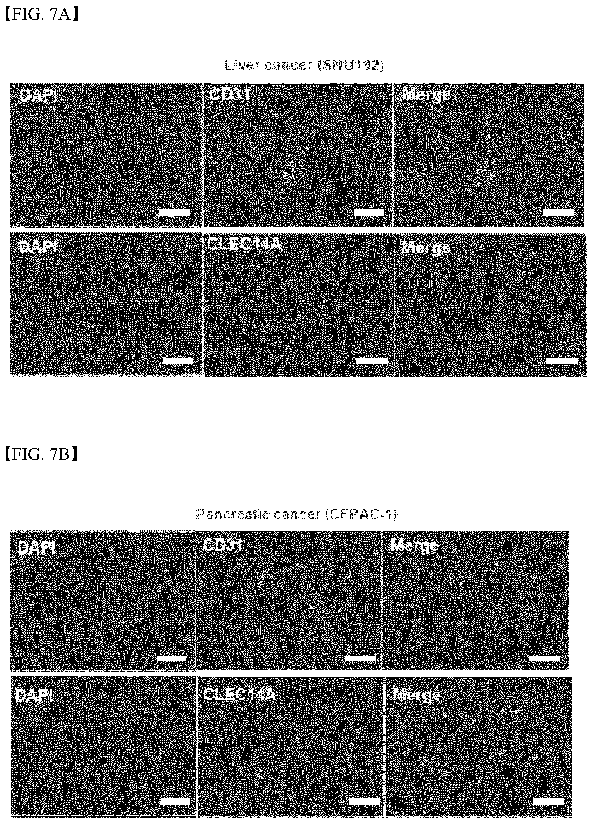

Clec14a was expressed in SNU182- and CFPAC-1 tumor-xenograft blood vessels, similar to CD31, which means that clec14a is expressed specifically in tumor blood vessels (FIGS. 7A and 7B).

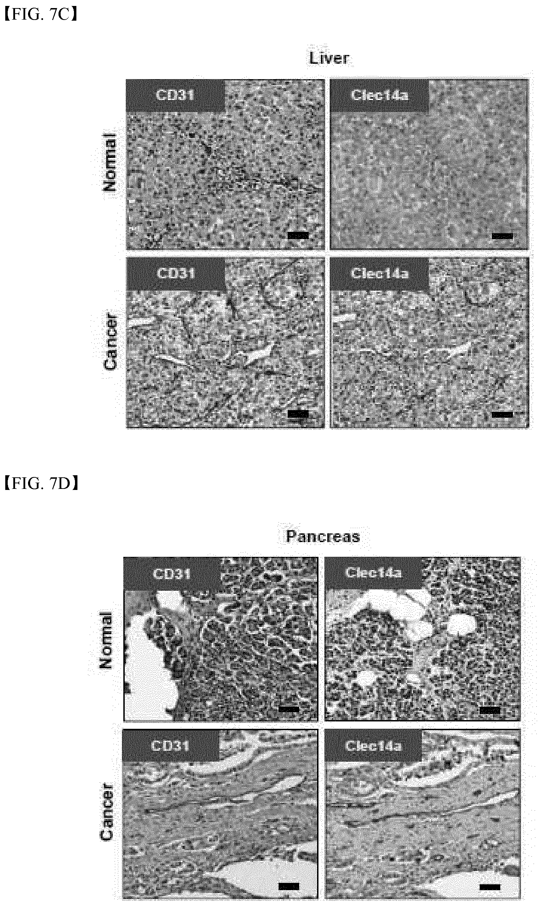

In order to evaluate specific expression of clec14a in tumor blood vessels among clinical samples using immunohistochemistry, expression of clec14a was compared between blood vessels of normal tissues and blood vessels of tissues derived from liver cancer and pancreatic cancer patients. As a result, blood vessels of cancer patient tissues showed a remarkable specific increase in clec14a, whereas normal blood vessels did not show the same (FIGS. 7C and 7D).

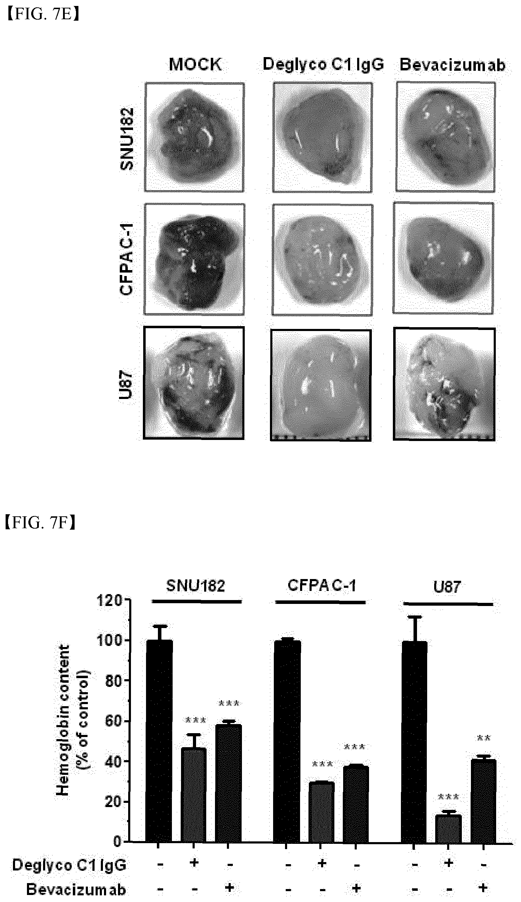

In order to investigate *in vivo* effects of deglyco C1 IgG on tumor angiogenesis, tumor cell-derived Matrigel plug angiogenesis assay was conducted on thymus-removed nude mice. SNU182- and CFPAC-1 cells and U87 human glioma cells containing Matrigel were transplanted into thymus-removed nude mice in the presence of deglyco C1 IgG and bevacizumab with a single dose of 10 mg/kg or in the absence thereof. 2 weeks later, the Matrigel plug was removed and the total hemoglobulin content of each group was measured with an ELISA reader. Deglyco C1 IgG significantly reduced hemoglobin derived from SNU182-, CFPAC-1 and U87 human glioma cells to a similar extent to bevacizumab (FIGS. 7E and 7F).

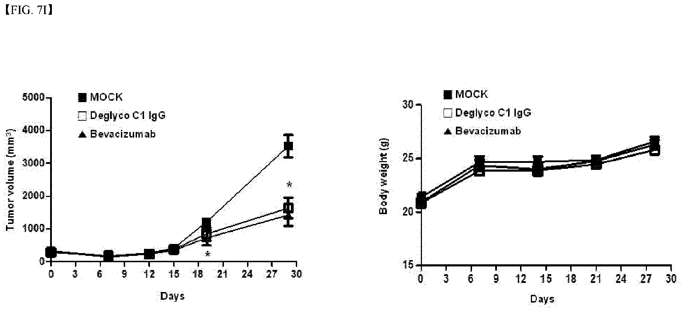

With respect to U87 human glioma, deglyco C1 IgG has no impact on body weight and significantly reduces the tumor sizes of U87 cells, like bevacizumab (FIG. 7I). It can be seen that deglyco C1 IgG has no significant impact on survival, shape or activation of HUVECs *in vitro.* This means that the optimized antibodies do not induce significant *in vivo* endothelial toxicity.

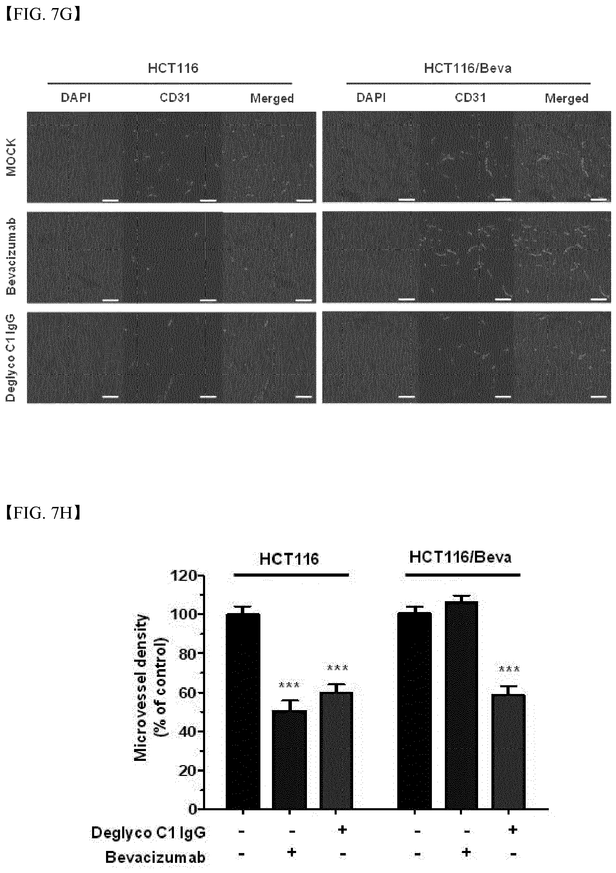

Matrigel plug angiogenesis assay was conducted in the presence of deglyco C1 IgG or bevacizumab with a single dose of 5 mg/kg or the absence thereof using HCT116 human colorectal cancer cells and bevacizumab-applied HCT116 human colorectal cancer cells (HCT116 and HCT116/Beva). 14 days later, the Matrigel plug was removed, and immunohistochemistry was conducted on CD31 and microvascular markers. A microvessel density was measured by quantifying CD31 positive per field in an image each obtained by a confocal microscope. It can be seen that deglyco C1 IgG and bevacizumab similarly and significantly reduced microvessel formation ability of HCT116 cells. Similarly, microvessel formation by HCT116/Beva cells was significantly suppressed by deglyco C1 IgG, but was not suppressed by bevacizumab (FIGS. 7G and 7H). This shows that deglyco C1 IgG is potently capable of suppressing angiogenesis of tumors in bevacizumab-tolerant colorectal cancer cells.

Overall, these results suggest that the produced antibody has efficacy of *in vivo* suppressing tumor angiogenesis.

### Example 8: Selection and efficacy analysis of optimized candidate antibodies

### Example 8-1. Selection of optimized candidate antibodies

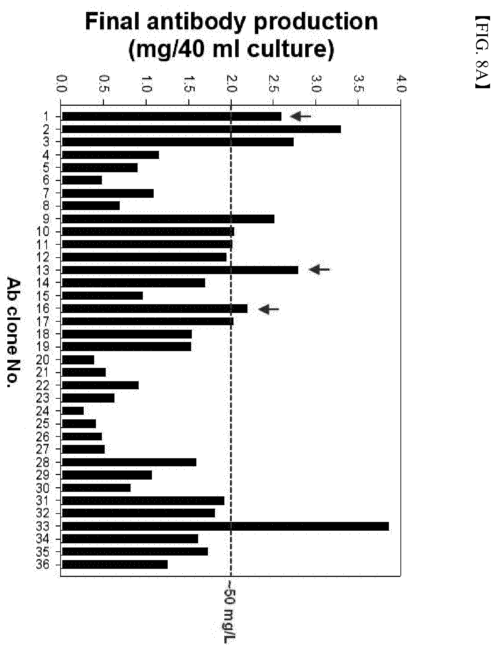

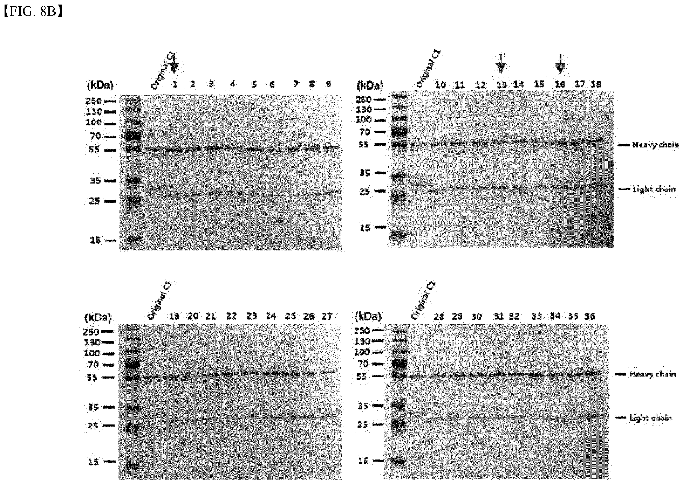

In order to select (screening) additional optimized antibodies, 36 types of antibodies were further selected and antibodies having improved affinity, as compared with deglycoC1, were selected. Mammal expression vectors including 36 types of antibodies (IgG antibodies) shown in Table 5 were transfected into HEK293 cells using polyethylenimine (PEI) and then each cultured in an amount of 40 ml for seven days. The culture solution was obtained by centrifugation and then purified by protein A affinity chromatography (affinity column chromatography with protein A sepharose column). A purification degree of 90% was identified by SDS-PAGE and molecular weights of light chains and heavy chains were simultaneously identified (FIG. 8A).

Opti 1, Opti 3 and Opti 16 (clones 1, 3 and 16) were primarily selected as clones having excellent production efficiency (about 50 mg/L or more) when calculated as liter-based culture.

**[Table 5]**

| VH | FR1 | CDR1 | FR2 | CDR2 | FR3 | CDR3 | | FR4 |
|---|---|---|---|---|---|---|---|---|
| Opti 1 | | GFTFS GYDMS | WIRQAP GKGLEW VA | GIYPDGG NTYYADS VKG | RFTISRDDSKN TFYLQMNSLRA EDTAVYYCAR | GAATK VLGKF DC | SEQ ID No: 25 | WGQGT LVTVS S |
| Opti 2 | | GFTFS GYDMS | WIRQAP GKGLEW VA | GIYPDGG NTYYADS VKG | RFTISRDDSKN TFYLQMNSLRA EDTAVYYCAR | GAASP VLGPF DE | SEQ ID No: 26 | WGQGT LVTVS S |
| Opti 3 | | GFTFS GYDMS | WIRQAP GKGLEW VA | GIYPDGG NTYYADS VKG | RFTISRDDSKN TFYLQMNSLRA EDTAVYYCAR | GATWW VLGAF DY | SEQ ID No: 27 | WGQGT LVTVS S |
| Opti 4 | | GFTFS GYDMS | WIRQAP GKGLEW VA | GIYPDGG NTYYADS VKG | RFTISRDDSKN TFYLQMNSLRA EDTAVYYCAR | GAEFV VLGSF DM | SEQ ID No: 28 | WGQGT LVTVS S |
| Opti 5 | | GFTFS GYDMS | WIRQAP GKGLEW VA | GIYPDGG NTYYADS VKG | RFTISRDDSKN TFYLQMNSLRA EDTAVYYCAR | GAARC VLGNF DH | SEQ ID No: 29 | WGQGT LVTVS S |
| Opti 6 | | GFTFS GYDMS | WIRQAP GKGLEW VA | GIYPDGG NTYYADS VKG | RFTISRDDSKN TFYLQMNSLRA EDTAVYYCAR | GASRT VLGNF DD | SEQ ID No: 30 | WGQGT LVTVS S |
| Opti 7 | | GFTFS GYDMS | WIRQAP GKGLEW VA | GIYPDGG NTYYADS VKG | RFTISRDDSKN TFYLQMNSLRA EDTAVYYCAR | GAHLT VLGIF DT | SEQ ID No: 31 | WGQGT LVTVS S |
| Opti 8 | | GFTFS GYDMS | WIRQAP GKGLEW VA | GIYPDGG NTYYADS VKG | RFTISRDDSKN TFYLQMNSLRA EDTAVYYCAR | GAHIY VLGQF DG | SEQ ID No: 32 | WGQGT LVTVS S |
| Opti 9 | | GFTFS GYDMS | WIRQAP GKGLEW VA | GIYPDGG NTYYADS VKG | RFTISRDDSKN TFYLQMNSLRA EDTAVYYCAR | GANLG VLGEF DK | SEQ ID No: 33 | WGQGT LVTVS S |
| Opti 10 | | GFTFS GYDMS | WIRQAP GKGLEW VA | GIYPDGG NTYYADS VKG | RFTISRDDSKN TFYLQMNSLRA EDTAVYYCAR | GAVPP VLGAF DT | SEQ ID No: 34 | WGQGT LVTVS S |
| Opti 11 | | GFTFS GYDMS | WIRQAP GKGLEW VA | GIYPDGG NTYYADS VKG | RFTISRDDSKN TFYLQMNSLRA EDTAVYYCAR | GARID VLGPF DA | SEQ ID No: 35 | WGQGT LVTVS S |
| Opti 12 | | GFTFS GYDMS | WIRQAP GKGLEW VA | GIYPDGG NTYYADS VKG | RFTISRDDSKN TFYLQMNSLRA EDTAVYYCAR | GAKPP VLGQF DR | SEQ ID No: 36 | WGQGT LVTVS S |
| Opti 13 | | GFTFS GYDMS | WIRQAP GKGLEW VA | GIYPDGG NTYYADS VKG | RFTISRDDSKN TFYLQMNSLRA EDTAVYYCAR | GAMEQ VLGPF DL | SEQ ID No: 37 | WGQGT LVTVS S |
| Opti 14 | | GFTFS GYDMS | WIRQAP GKGLEW VA | GIYPDGG NTYYADS VKG | RFTISRDDSKN TFYLQMNSLRA EDTAVYYCAR | GARSW VLGEF DK | SEQ ID No: 38 | WGQGT LVTVS S |
| Opti 15 | | GFTFS GYDMS | WIRQAP GKGLEW VA | GIYPDGG NTYYADS VKG | RFTISRDDSKN TFYLQMNSLRA EDTAVYYCAR | GAECA VLGNF DY | SEQ ID No: 39 | WGQGT LVTVS S |
| Opti 16 | | GFTFS GYDMS | WIRQAP GKGLEW VA | GIYPDGG NTYYADS VKG | RFTISRDDSKN TFYLQMNSLRA EDTAVYYCAR | GARRQ VLGCF DF | SEQ ID No: 40 | WGQGT LVTVS S |
| Opti 17 | | GFTFS GYDMS | WIRQAP GKGLEW VA | GIYPDGG NTYYADS VKG | RFTISRDDSKN TFYLQMNSLRA EDTAVYYCAR | GASAP VLGDF DY | SEQ ID No: 41 | WGQGT LVTVS S |
| Opti 18 | | GFTFS GYDMS | WIRQAP GKGLEW VA | GIYPDGG NTYYADS VKG | RFTISRDDSKN TFYLQMNSLRA EDTAVYYCAR | GASTP VLGSF DN | SEQ ID No: 42 | WGQGT LVTVS S |
| Opti 19 | | GFTFS GYDMS | WIRQAP GKGLEW VA | GIYPDGG NTYYADS VKG | RFTISRDDSKN TFYLQMNSLRA EDTAVYYCAR | GAHYN VLGPF DS | SEQ ID No: 43 | WGQGT LVTVS S |
| Opti 20 | | GFTFS GYDMS | WIRQAP GKGLEW VA | GIYPDGG NTYYADS VKG | RFTISRDDSKN TFYLQMNSLRA EDTAVYYCAR | GARTC VLGPF DH | SEQ ID No: 44 | WGQGT LVTVS S |
| Opti 21 | | GFTFS GYDMS | WIRQAP GKGLEW VA | GIYPDGG NTYYADS VKG | RFTISRDDSKN TFYLQMNSLRA EDTAVYYCAR | GAIAP VLGGF DR | SEQ ID No: 45 | WGQGT LVTVS S |
| Opti 22 | | GFTFS GYDMS | WIRQAP GKGLEW VA | GIYPDGG NTYYADS VKG | RFTISRDDSKN TFYLQMNSLRA EDTAVYYCAR | GARQS VLGPF DA | SEQ ID No: 46 | WGQGT LVTVS S |
| Opti 23 | | GFTFS GYDMS | WIRQAP GKGLEW VA | GIYPDGG NTYYADS VKG | RFTISRDDSKN TFYLQMNSLRA EDTAVYYCAR | GAHYN VLGPF DS | SEQ ID No: 47 | WGQGT LVTVS S |
| Opti 24 | | GFTFS GYDMS | WIRQAP GKGLEW VA | GIYPDGG NTYYADS VKG | RFTISRDDSKN TFYLQMNSLRA EDTAVYYCAR | GAKYY VLGHF DE | SEQ ID No: 48 | WGQGT LVTVS S |
| Opti 25 | | GFTFS GYDMS | WIRQAP GKGLEW VA | GIYPDGG NTYYADS VKG | RFTISRDDSKN TFYLQMNSLRA EDTAVYYCAR | GAALR VLGMF DL | SEQ ID No: 49 | WGQGT LVTVS S |
| Opti 26 | | GFTFS GYDMS | WIRQAP GKGLEW VA | GIYPDGG NTYYADS VKG | RFTISRDDSKN TFYLQMNSLRA EDTAVYYCAR | GAHRR VLGWF DD | SEQ ID No: 50 | WGQGT LVTVS S |
| Opti 27 | | GFTFS GYDMS | WIRQAP GKGLEW VA | GIYPDGG NTYYADS VKG | RFTISRDDSKN TFYLQMNSLRA EDTAVYYCAR | GAYHT VLGQF DQ | SEQ ID No: 51 | WGQGT LVTVS S |
| Opti 28 | | GFTFS GYDMS | WIRQAP GKGLEW VA | GIYPDGG NTYYADS VKG | RFTISRDDSKN TFYLQMNSLRA EDTAVYYCAR | GAQST VLGNF DT | SEQ ID No: 52 | WGQGT LVTVS S |
| Opti 29 | | GFTFS GYDMS | WIRQAP GKGLEW VA | GIYPDGG NTYYADS VKG | RFTISRDDSKN TFYLQMNSLRA EDTAVYYCAR | GAHMR VLGPF DG | SEQ ID No: 53 | WGQGT LVTVS S |
| Opti 30 | | GFTFS GYDMS | WIRQAP GKGLEW VA | GIYPDGG NTYYADS VKG | RFTISRDDSKN TFYLQMNSLRA EDTAVYYCAR | GAALH VLGPF DP | SEQ ID No: 54 | WGQGT LVTVS S |
| Opti 31 | | GFTFS GYDMS | WIRQAP GKGLEW VA | GIYPDGG NTYYADS VKG | RFTISRDDSKN TFYLQMNSLRA EDTAVYYCAR | GAHGQ VLGDF DQ | SEQ ID No: 55 | WGQGT LVTVS S |
| Opti 32 | | GFTFS GYDMS | WIRQAP GKGLEW VA | GIYPDGG NTYYADS VKG | RFTISRDDSKN TFYLQMNSLRA EDTAVYYCAR | GASQE VLGDF DV | SEQ ID No: 56 | WGQGT LVTVS S |
| Opti 33 | | GFTFS GYDMS | WIRQAP GKGLEW VA | GIYPDGG NTYYADS VKG | RFTISRDDSKN TFYLQMNSLRA EDTAVYYCAR | SATWW MSEPR SY | SEQ ID No: 57 | WGQGT LVTVS S |
| Opti 34 | | GFTFS GYDMS | WIRQAP GKGLEW VA | GIYPDGG NTYYADS VKG | RFTISRDDSKN TFYLQMNSLRA EDTAVYYCAR | GADNH VLGDF DV | SEQ ID No: 58 | WGQGT LVTVS S |
| Opti 35 | | GFTFS GYDMS | WIRQAP GKGLEW VA | GIYPDGG NTYYADS VKG | RFTISRDDSKN TFYLQMNSLRA EDTAVYYCAR | GAHKP VLGDF DA | SEQ ID No: 59 | WGQGT LVTVS S |
| Opti 36 | | GFTFS GYDMS | WIRQAP GKGLEW VA | GIYPDGG NTYYADS VKG | RFTISRDDSKN TFYLQMNSLRA EDTAVYYCAR | GAHHA VLGPF DR | SEQ ID No: 60 | WGQGT LVTVS S |
| VL | FR1 | CDR1 | FR2 | CDR2 | FR3 | CDR3 | | FR4 |
| Deg C1 | DIQLTQSPS SLSASVGDR VTITC | TGSSS NIGRC GVT | WYQQKP GKAPKL LIY | ADSHRPS | GVPSRFSGSGS GTDFTLTISSL QPEDFATYYC | GAWDDSLSGYV | | FGQGT KVEIK R |

### Example 8-2. Analysis of in vitro efficacy of selected antibodies

In order to identify inhibitory activity of optimized candidate antibodies against angiogenesis dependent upon VEGF, which is a key factor of angiogenesis, anti-angiogenesis activity was comparatively analyzed using an IncuCyte FLR live content imaging system (Essen Bioscience Inc.) providing real-time analysis of angiogenesis. First, GFP-transfected HUVECs were seeded on a 94-well plate, were treated with 36 types of antibodies at a concentration of 20 ug/ml and *in vitro* efficacy was then compared. At this time, optimized antibodies were comparatively analyzed in terms of efficacy with parental IgG (parent antibody: clone 1 of WO 2013/187556), deglyco C1 and bevacizumab (Avastin) as positive control groups.

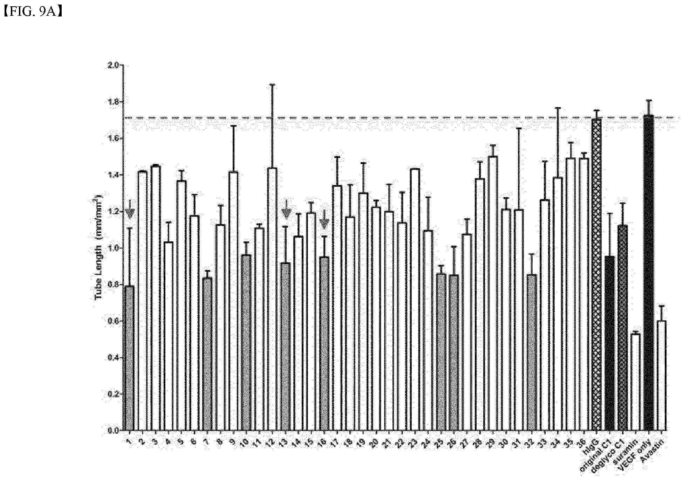

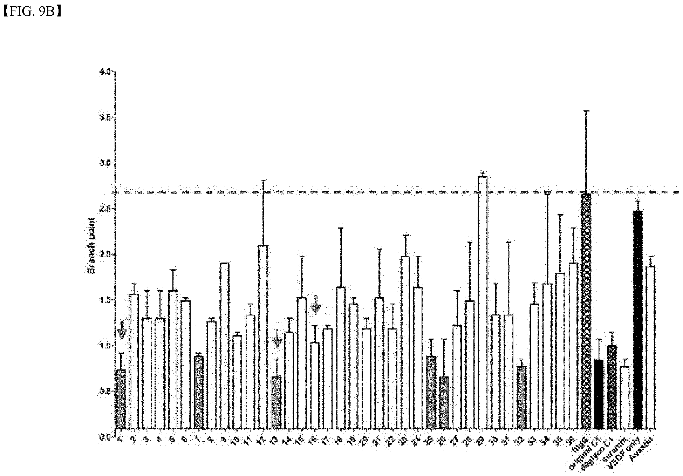

Results are shown in FIGS. 9A and 9B. The results showed that, in consideration of both tube length (FIG. 9A) and branch point (FIG. 9B), the treated antibodies have anti-angiogenesis activity and, in particular, clones 1, 3 and 16 have potent efficacy.

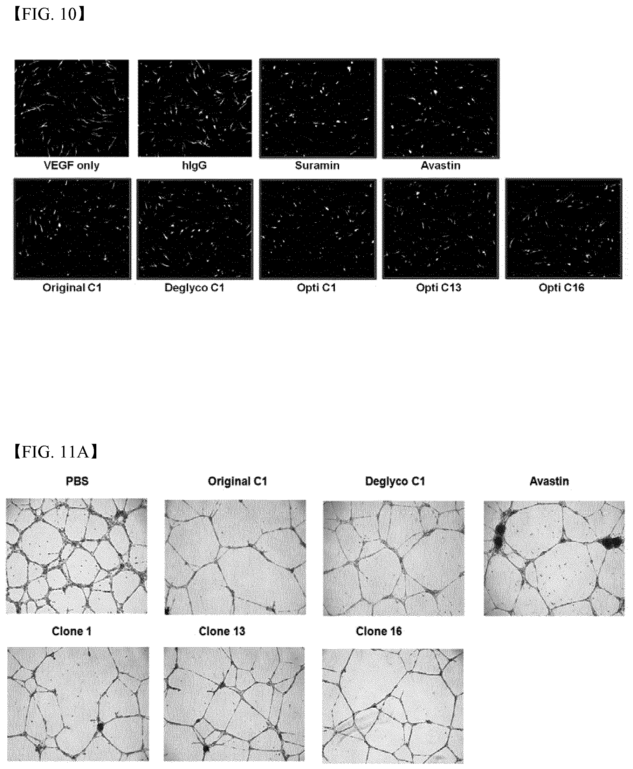

As can be seen from FIG. 10 showing representative results obtained by investigating changes in tube length (progression of angiogenesis) after treatment with VEGF and optimized antibodies, a single group (treated with only VEGF) showed an increase in tube length (progression of angiogenesis), and hIgG (human IgG, control group antibody), which is a negative control group of the administered antibody, had no response. In addition, Suramin (angiogenesis inhibitor, small chemical) and Avastin (IgG) were used as positive control groups. In particular, optimized antibody clones 1, 3 and 16 have the same efficacy as Suramin and Avastin as the positive control groups.

EGM is a type of complex including a variety of angiogenesis factors, which has been used to induce strong angiogenesis using vascular endothelial cells. Accordingly, in order to investigate whether or not anti-angiogenesis activity exists in the presence of various angiogenesis factors such as EGM as well as in the presence of VEGF-dependent angiogenesis of optimized antibodies, HUVEC cells were seeded on a 48-well microtiter plate, the parent antibody (original C1) and deglyco C1 were used as types of positive control groups, the cells were treated with 20 ug/ml of clones 1, 13 and 16 exhibiting excellent anti-angiogenesis activity, and tube formation degree was then comparatively analyzed.

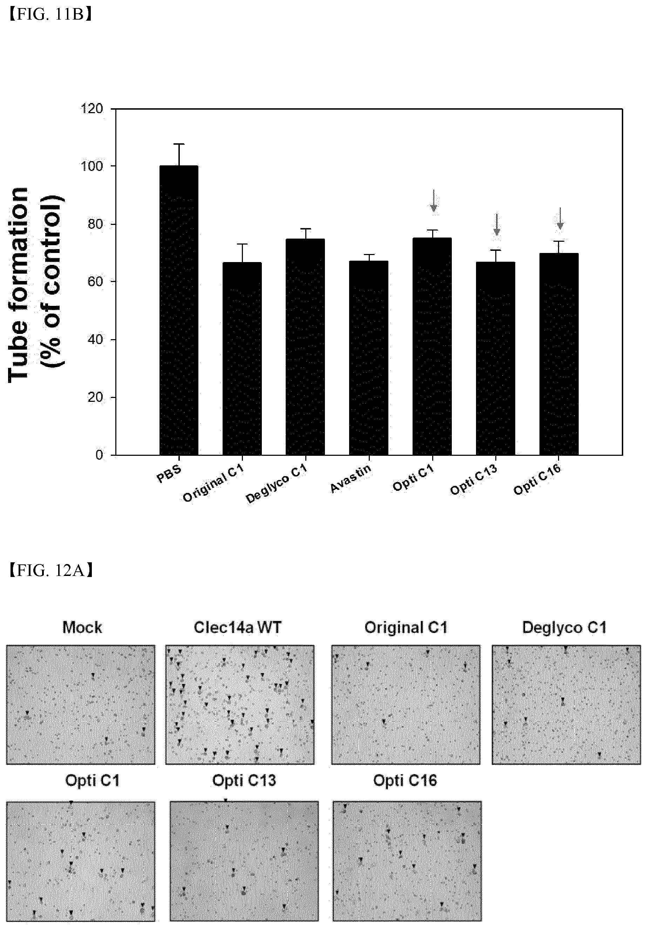

Results are shown in FIGS. 11A and 11B. The results showed that the selected antibodies significantly suppressed EGM-dependant angiogenesis. Accordingly, it can be seen that the optimized antibodies including clone 1, 13, 16 antibodies are capable of efficiently suppressing angiogenesis that can be caused by various angiogenesis factors.

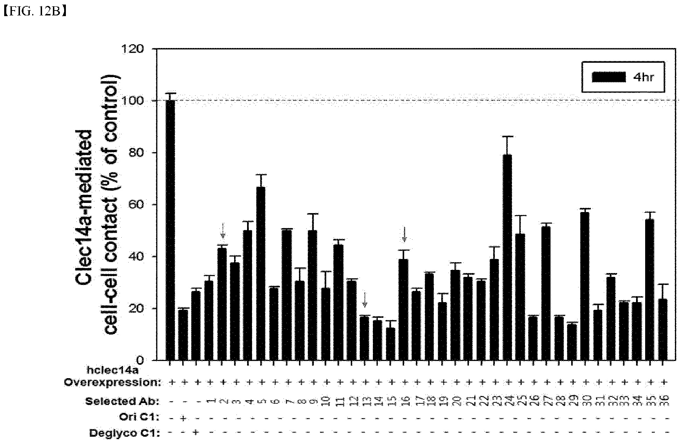

In addition to tube length, the numbers of branches and tube formation as described above, the major mechanisms of angiogenesis are considered to be endothelial cell-cell contacts. Accordingly, in order to conduct Clec14a-mediated cell-cell contacts, clec14a-containing mammal expression vectors were transfected into HEK293 cells. It could be seen that cell-cell contacts (aggregates, used as a marker of clec14a-mediated cell conjugation) of HEK293 cells were increased. Aggregates were directly counted with a manual counter under a microscope and efficacies of the treated antibodies were comparatively analyzed. In order to investigate anti-angiogenesis activity of the treated antibodies under this condition, 20 ug/ml of each antibody was treated, and parent antibody (original C1) and deglyco C1 were used as positive control groups. Results are shown in FIGS. 12A and 12B. The results showed that, in conclusion, all the antibodies potently inhibited Clec14a-mediated cell-cell contacts.

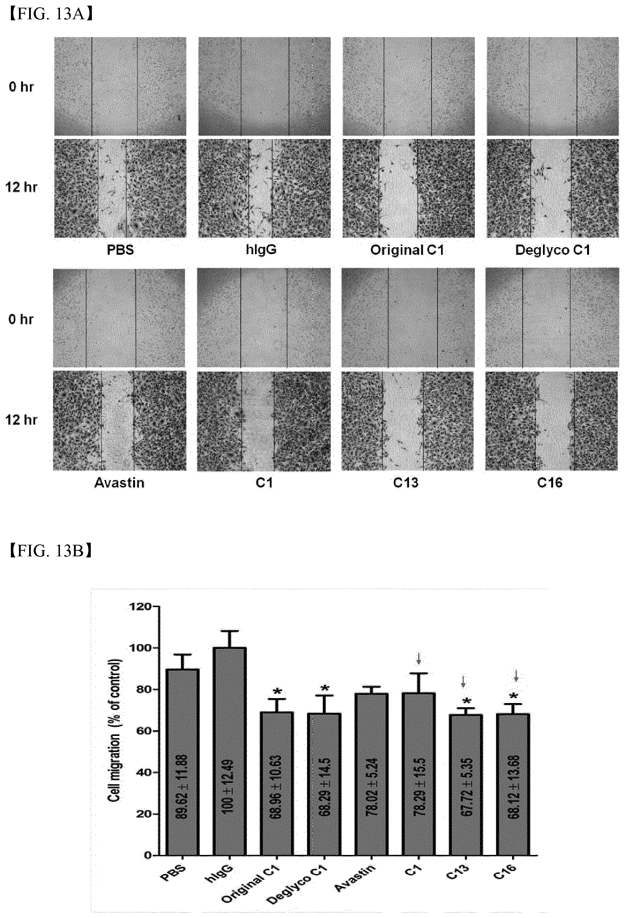

In order to analyze *in vitro* efficacies of the optimized candidate antibodies on endothelial migration, which is another function of angiogenesis, HUVEC migration assay was conducted using an IncuCyte FLR live content imaging system (Essen Bioscience Inc). At this time, regarding a wound, a uniform wound was created using a wound maker commercially available from Incucyte and was treated with 20 ug/ml of respective antibodies, and inhibitory activity against migration was comparatively evaluated. Results are shown in FIGS. 13A and 13B. In conclusion, the results showed tha the optimized antibodies including clones 1, 13 and 16 have the same endothelial migration inhibitory activity as parent antibody (original C1), deglyco C1 IgG, and Avastin.

### Example 8-3. Identification of antigen-binding sites of selected antibodies

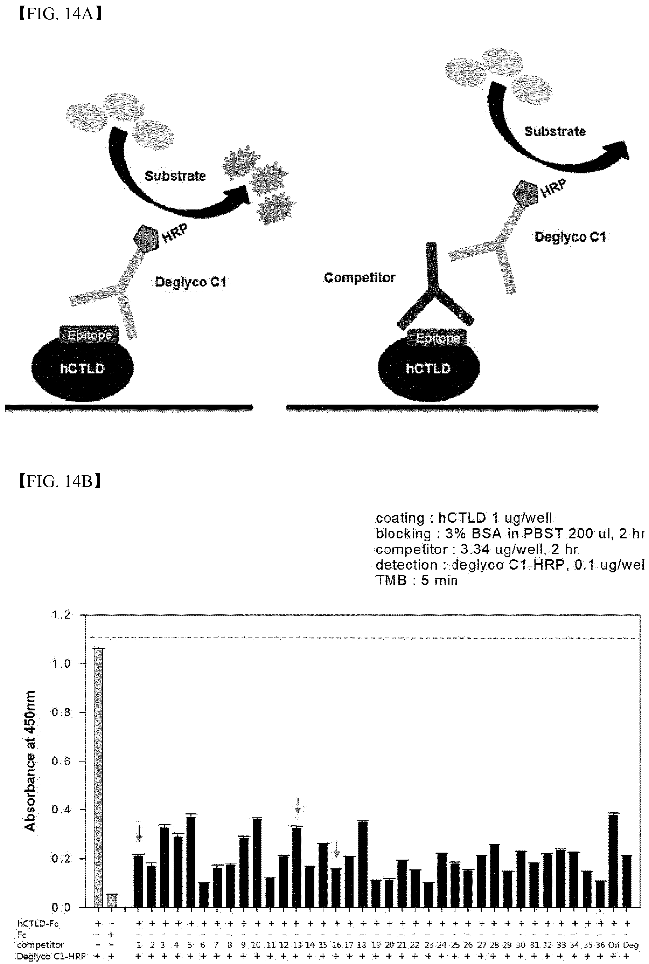

In order to identify antigen-binding sites of selected antibodies, competitive ELISA was conducted. Specifically, first, a CTLD antigen was coated on a 96-well microtiter plate and binding of the CTLD antigen to HRP-conjugated deglyco C1 IgG was induced (FIG. 14A). At the same time, the resulting structure was treated with 36 types of antibodies and whether or not they bound to the antigen comparatively with deglyco C1 was investigated to identify antigen binding sites of deglyco C1 IgG and 36 types of antibodies. Results are shown in FIG. 14B, which showed that all the 36 types of antibodies have similar antigen-binding sites to deglyco C1 IgG.

### Example 8-4. Identification of cross-species reactivity of optimized antibodies

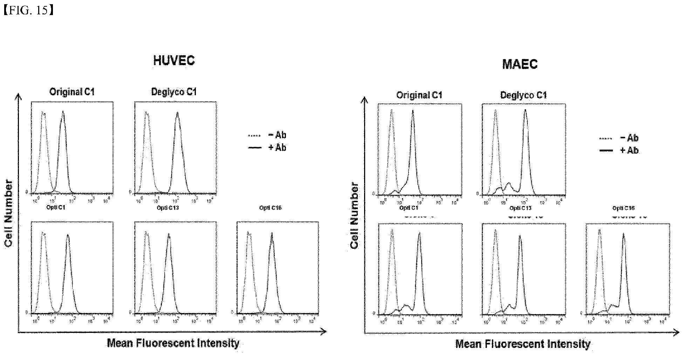

In order to identify cross-species reactivity of the optimized antibodies, HUVECs (human umbilical vein endothelial cells) and MAECs (mouse aortic endothelial cells) were each cultured, the two cell groups were treated with 20 ug/ml of the parent antibody (original C1), deglyco C1, and clones 1, 13 and 16, and whether or not binding ability exists on the surface of two types of cells was identified by flow cytometry. Results are shown in FIG. 15. In conclusion, three types of clones 1, 13 and 16 of the optimized antibodies had cross-species reactivity meaning the ability to bind to human and mouse CLEC14a.

### Example 8-5. Analysis of action mechanism of optimized antibodies

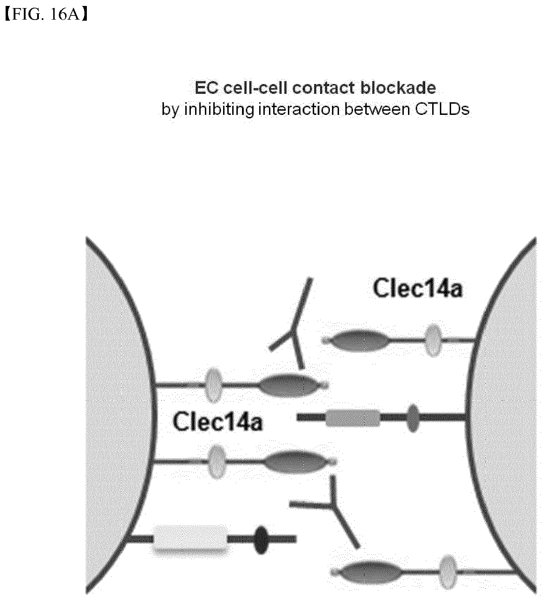

In order to analyze action mechanisms of optimized antibodies, first, whether or not the optimized antibodies have inhibitory activity against cell-cell contacts in vascular endothelial cells was investigated. The present inventors found that CLEC14a, in particular, the CTLD domain, plays a key role for cell-cell contacts in vascular endothelial cells and the CLEC14-CTLD-conjugated antibody (original C1) serves as an inhibitor of interaction therebetween (FIG. 16A).

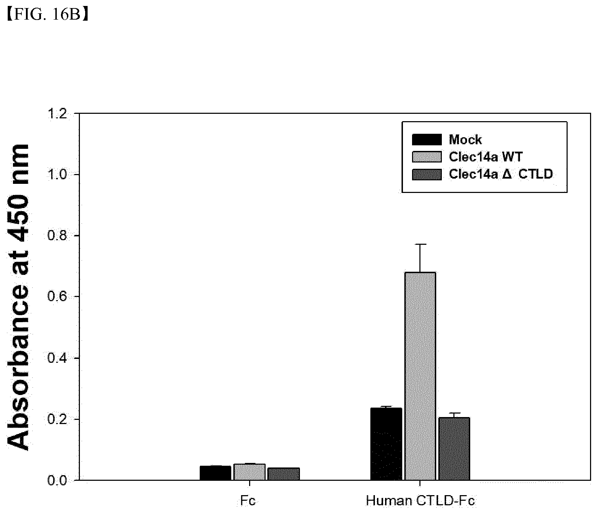

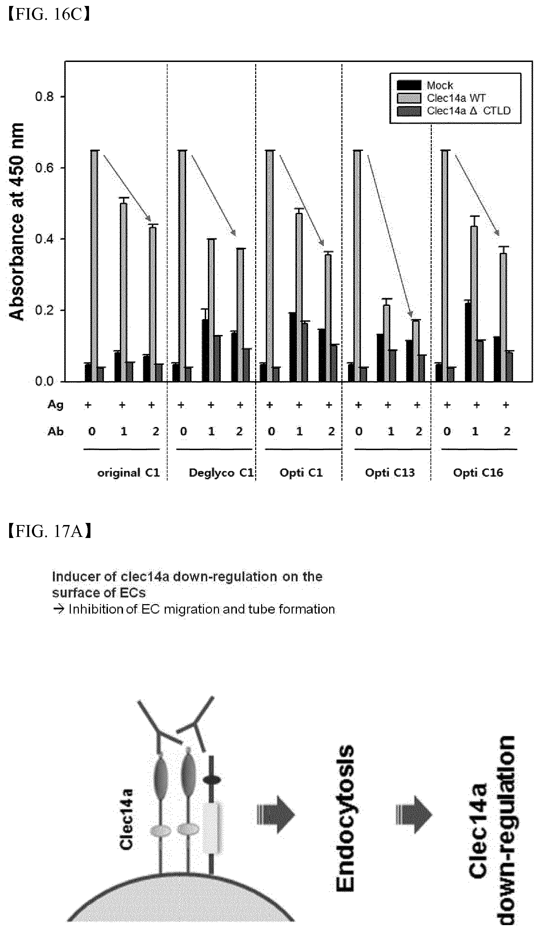

In order to demonstrate these features, wild-type CLEC14a and CTLD domain-deleted CLEC14a (DCTLD) were each transfected into COS-7 cells and crushed cells thereof were coated on a 96-well microtiter plate. In addition, purified HRP-conjugated CTLD-Fc was incubated and CTLD binding was observed. Results are shown in FIG. 16B. In conclusion, it can be seen that the CTLD domain, which is important for CLEC14a-CLEC14a binding. FIG. 16C shows results of comparative analysis using competitive ELISA regarding the binding ability of HRP-conjugated CTLD-Fc to CLEC14a, while increasing the molar ratio of antigen to antibody in an order of 0:1, 1:1 and 1:2. In this regard, for the antibodies, the parent antibody (original C1) and deglyco C1 were used as positive control groups, and three types of optimized antibody representative clones 1, 13 and 16 were treated as control groups. Results showed that all the treated antibodies concentration-dependently potently inhibited CTLD-mediated interaction between CLEC14a molecules.

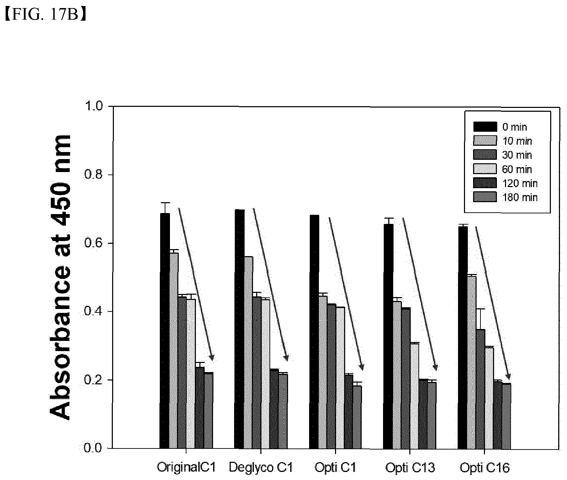

In addition, in order to analyze action mechanisms of the optimized antibodies, CLEC14a down-regulation on vascular endothelial cell surface was investigated. FIG. 17A illustrates CLEC14a down-regulation of the vascular endothelial cell surface by a conventional CLEC14a-CTLD-conjugated antibody (parent antibody: original C1). In order to identify whether or not the optimized antibodies have CLEC14a down-regulation, HUVECs were coated on a 96-well microtiter plate and treated with 20 ug/ml of the parent antibody (original C1), deglyco C1, and three types of clone 1, 13 and 16 optimized antibodies, and then quantity levels of CLEC14a present on HUVEC surfaces were finally measured over time by cell ELISA. The quantity levels of CLEC14a was measured by using a commercially available sheep anti-CLEC14a antibody (sheep anti-CLEC14a Ab), using an HRP-conjugated anti-sheep antibody as a secondary antibody, conducting staining using TMB and then measuring an absorbance at 450 nm with an ELISA reader. Results are shown in FIG. 17B, which indicated that the treated antibodies exhibit down-regulation of CLEC14a on vascular endothelial cell surfaces.

### [Application in Industrial Field]

The deglycosylated antibody specifically binding to clec14a according to the present disclosure has high production amounts, maintains cross-reactivity to humans and mice, exhibits desired antigen reactivity, and has excellent solubility and stability due to less or almost no aggregation, as well as improved inhibitory activity against tube formation, thereby providing an improved antibody with enhanced characteristics and efficacies.

Although the present disclosure has been described in detail with reference to specific configurations, those skilled in the art will appreciate that this description is provided as preferred embodiments for illustrative purposes and should not be construed as limiting the scope of the present disclosure. Therefore, the substantial scope of the present disclosure is defined by the accompanying claims filed and equivalents thereto.

## Claims

1. An antibody or antigen binding fragment thereof binding to clec14a, wherein the antibody comprises a light chain variable region comprising CDR1 of TGSSSNIGXXXVT (SEQ ID NO: 1), wherein each of amino acids X at positions 9, 10 and 11 in the SEQ ID NO: 1 is any one selected from the group consisting of R, C, G, A, T, W, S, N, and V.

2. The antibody or antigen binding fragment thereof according to claim 1, wherein X at positions 9, 10 and 11 in the SEQ ID NO: 1 is RCG, ATA, WSN or AVV.

3. The antibody or antigen binding fragment thereof according to claim 1, wherein the antibody is deglycosylated.

4. The antibody or antigen binding fragment thereof according to claim 1, wherein the antibody comprises a light chain variable region comprising framework selected from the group consisting of SEQ ID NOS: 21 to 24.

5. The antibody or antigen binding fragment thereof according to claim 1, wherein the antibody comprises a light chain variable region of selected from the group consisting of SEQ ID NOS: 7 to 10.

6. The antibody or antigen binding fragment thereof according to claim 1, wherein the antibody comprises a heavy chain variable region comprising CDR3 selected from the group consisting of SEQ ID NO: 13, and SEQ ID NOS: 25 to 60.

7. The antibody or antigen binding fragment thereof according to claim 1, wherein the antibody comprises a heavy chain variable region selected from the group consisting of SEQ ID NOS: 6, and 61 to 96.

8. The antibody or antigen binding fragment thereof according to claim 1, wherein the antibody comprises:
a light chain CDR1 of SEQ ID NO: 2, a light chain CDR2 of SEQ ID NO: 15 and a light chain CDR3 of SEQ ID NO: 16, a heavy chain CDR1 of SEQ ID NO: 11, a heavy chain CDR2 of SEQ ID NO: 12 and a heavy chain CDR3 of SEQ ID NO: 13;
a light chain CDR1 of SEQ ID NO: 3, a light chain CDR2 of SEQ ID NO: 15 and a light chain CDR3 of SEQ ID NO: 16, a heavy chain CDR1 of SEQ ID NO: 11, a heavy chain CDR2 of SEQ ID NO: 12 and a heavy chain CDR3 of SEQ ID NO: 13;
a light chain CDR1 of SEQ ID NO: 4, a light chain CDR2 of SEQ ID NO: 15 and a light chain CDR3 of SEQ ID NO: 16, a heavy chain CDR1 of SEQ ID NO: 11, a heavy chain CDR2 of SEQ ID NO: 12 and a heavy chain CDR3 of SEQ ID NO: 13;
a light chain CDR1 of SEQ ID NO: 5, a light chain CDR2 of SEQ ID NO: 15 and a light chain CDR3 of SEQ ID NO: 16, a heavy chain CDR1 of SEQ ID NO: 11, a heavy chain CDR2 of SEQ ID NO: 12 and a heavy chain CDR3 of SEQ ID NO: 13;
a light chain CDR1 of SEQ ID NO: 2, a light chain CDR2 of SEQ ID NO: 15 and a light chain CDR3 of SEQ ID NO: 16, a heavy chain CDR1 of SEQ ID NO: 11, a heavy chain CDR2 of SEQ ID NO: 12 and a heavy chain CDR3 of SEQ ID NO: 25;
a light chain CDR1 of SEQ ID NO: 2, a light chain CDR2 of SEQ ID NO: 15 and a light chain CDR3 of SEQ ID NO: 16, a heavy chain CDR1 of SEQ ID NO: 11, a heavy chain CDR2 of SEQ ID NO: 12 and a heavy chain CDR3 of SEQ ID NO: 37; or
a light chain CDR1 of SEQ ID NO: 2, a light chain CDR2 of SEQ ID NO: 15 and a light chain CDR3 of SEQ ID NO: 16, a heavy chain CDR1 of SEQ ID NO: 11, a heavy chain CDR2 of SEQ ID NO: 12 and a heavy chain CDR3 of SEQ ID NO: 40.

9. The antibody or antigen binding fragment thereof according to claim 1, wherein the antibody comprises: a light chain variable region of SEQ ID NO: 7 and a heavy chain variable region of SEQ ID NO: 6;
a light chain variable region of SEQ ID NO: 8 and a heavy chain variable region of SEQ ID NO: 6;
a light chain variable region of SEQ ID NO: 9 and a heavy chain variable region of SEQ ID NO: 6;
a light chain variable region of SEQ ID NO: 10 and a heavy chain variable region of SEQ ID NO: 6;
a light chain variable region of SEQ ID NO: 7 and a heavy chain variable region of SEQ ID NO: 61;
a light chain variable region of SEQ ID NO: 7 and a heavy chain variable region of SEQ ID NO: 73; or
a light chain variable region of SEQ ID NO: 7 and a heavy chain variable region of SEQ ID NO: 76.

10. A pharmaceutical composition for preventing or treating an angiogenesis-related disease comprising the antibody or antigen binding fragment thereof according to any one of claims 1 to 9.

11. The composition according to claim 10, wherein the angiogenesis-related disease is selected from the group consisting of cancer, metastasis, diabetic retinopathy, retinopathy of prematurity, corneal graft rejection, macular degeneration, neovascular glaucoma, erythrosis, proliferative retinopathy, psoriasis, hemophilic arthritis, microvessel formation of atherosclerotic plaques, keloid, wound granulation, vascular adhesion, rheumatoid arthritis, osteoarthritis, autoimmune diseases, Crohn's disease, restenosis, atherosclerosis, intestinal adhesions, cat scratch disease, ulcer, liver cirrhosis, nephritis, diabetic nephropathy, diabetes mellitus, inflammatory diseases and neurodegenerative diseases.

12. The composition according to claim 11, wherein the cancer is selected from the group consisting of esophageal cancer, stomach cancer, large intestine cancer, rectal cancer, oral cancer, pharynx cancer, larynx cancer, lung cancer, colon cancer, breast cancer, uterine cervical cancer, endometrial cancer, ovarian cancer, prostate cancer, testis cancer, bladder cancer, renal cancer, liver cancer, pancreatic cancer, bone cancer, connective tissue cancer, skin cancer, brain cancer, thyroid cancer, leukemia, Hodgkin's lymphoma, lymphoma and multiple myeloid blood cancer.


<110>    SCRIPPS KOREA ANTIBODY INSTITUTE

<120>    Deglycosyalted Antibody Binding Specifically to clec14a and Uses
         Thereof

<130>    PP-B1950

<150>    KR 10-2016-0115577
<151>    2016-09-08

<160>    96

<170>    KopatentIn 2.0

<210>    1
<211>    13
<212>    PRT
<213>    Artificial Sequence

<220>
<223>    L-CDR1


<400>    1
Thr Gly Ser Ser Ser Asn Ile Gly Xaa Xaa Xaa Val Thr
  1               5                   10


<210>    2
<211>    13
<212>    PRT
<213>    Artificial Sequence

<220>
<223>    L-CDR1


<400>    2
Thr Gly Ser Ser Ser Asn Ile Gly Arg Cys Gly Val Thr
  1               5                   10


<210>    3
<211>    13
<212>    PRT
<213>    Artificial Sequence

<220>
<223>    L-CDR1


<400>    3
Thr Gly Ser Ser Ser Asn Ile Gly Ala Thr Ala Val Thr
  1               5                   10


<210>    4
<211>    13
<212>    PRT
<213>    Artificial Sequence

<220>
<223>    L-CDR1

<400>    4
Thr Gly Ser Ser Ser Asn Ile Gly Trp Ser Asn Val Thr
1               5                   10

<210>    5
<211>    13
<212>    PRT
<213>    Artificial Sequence

<220>
<223>    L-CDR1

<400>    5
Thr Gly Ser Ser Ser Asn Ile Gly Ala Val Val Val Thr
1               5                   10

<210>    6
<211>    121
<212>    PRT
<213>    Artificial Sequence

<220>
<223>    Deg C1 VH

<400>    6
Glu Val Gln Leu Val Glu Ser Gly Gly Gly Leu Val Gln Pro Gly Gly
1               5                   10                  15

Ser Leu Arg Leu Ser Cys Ala Val Ser Gly Phe Thr Phe Ser Gly Tyr
            20                  25                  30

Asp Met Ser Trp Ile Arg Gln Ala Pro Gly Lys Gly Leu Glu Trp Val
        35                  40                  45

Ala Gly Ile Tyr Pro Asp Gly Gly Asn Thr Tyr Tyr Ala Asp Ser Val
    50                  55                  60

Lys Gly Arg Phe Thr Ile Ser Arg Asp Asp Ser Lys Asn Thr Phe Tyr
65                  70                  75                  80

Leu Gln Met Asn Ser Leu Arg Ala Glu Asp Thr Ala Val Tyr Tyr Cys
                85                  90                  95

Ala Arg Gly Ala Thr Trp Trp Val Leu Gly Pro Phe Asp Tyr Trp Gly
            100                 105                 110

Gln Gly Thr Leu Val Thr Val Ser Ser
        115                 120

<210>    7
<211>    112
<212>    PRT
<213>    Artificial Sequence

<220>
<223>    Deg C1 VL

<400>    7
Asp Ile Gln Leu Thr Gln Ser Pro Ser Ser Leu Ser Ala Ser Val Gly
1               5                   10                  15

Asp Arg Val Thr Ile Thr Cys Thr Gly Ser Ser Ser Asn Ile Gly Arg
            20                  25                  30

Cys Gly Val Thr Trp Tyr Gln Gln Lys Pro Gly Lys Ala Pro Lys Leu
        35                  40                  45

Leu Ile Tyr Ala Asp Ser His Arg Pro Ser Gly Val Pro Ser Arg Phe
    50                  55                  60

Ser Gly Ser Gly Ser Gly Thr Asp Phe Thr Leu Thr Ile Ser Ser Leu
65                  70                  75                  80

Gln Pro Glu Asp Phe Ala Thr Tyr Tyr Cys Gly Ala Trp Asp Asp Ser
                85                  90                  95

Leu Ser Gly Tyr Val Phe Gly Gln Gly Thr Lys Val Glu Ile Lys Arg
            100                 105                 110

<210>    8
<211>    112
<212>    PRT
<213>    Artificial Sequence

<220>
<223>    Deg C2 VL

<400>    8
Asp Ile Gln Leu Thr Gln Ser Pro Ser Ser Leu Ser Ala Ser Val Gly
1               5                   10                  15

Asp Arg Val Thr Ile Thr Cys Thr Gly Ser Ser Ser Asn Ile Gly Ala
            20                  25                  30

Thr Ala Val Thr Trp Tyr Gln Gln Lys Pro Gly Lys Ala Pro Lys Leu
        35                  40                  45

Leu Ile Tyr Ala Asp Ser His Arg Pro Ser Gly Val Pro Ser Arg Phe
    50                  55                  60

Ser Gly Ser Gly Ser Gly Thr Asp Phe Thr Leu Thr Ile Ser Ser Leu
65                  70                  75                  80

Gln Pro Glu Asp Phe Ala Thr Tyr Tyr Cys Gly Ala Trp Asp Asp Ser
                85                  90                  95

Leu Ser Gly Tyr Val Phe Gly Gln Gly Thr Lys Val Glu Ile Lys Arg
            100                 105                 110

<210>    9
<211>    112

<212>    PRT
<213>    Artificial Sequence

<220>
<223>    Deg C3 VL


<400>    9
Asp Ile Gln Leu Thr Gln Ser Pro Ser Ser Leu Ser Ala Ser Val Gly
1               5                   10                  15

Asp Arg Val Thr Ile Thr Cys Thr Gly Ser Ser Ser Asn Ile Gly Trp
            20                  25                  30

Ser Asn Val Thr Trp Tyr Gln Gln Lys Pro Gly Lys Ala Pro Lys Leu
        35                  40                  45

Leu Ile Tyr Ala Asp Ser His Arg Pro Ser Gly Val Pro Ser Arg Phe
    50                  55                  60

Ser Gly Ser Gly Ser Gly Thr Asp Phe Thr Leu Thr Ile Ser Ser Leu
65                  70                  75                  80

Gln Pro Glu Asp Phe Ala Thr Tyr Tyr Cys Gly Ala Trp Asp Asp Ser
                85                  90                  95

Leu Ser Gly Tyr Val Phe Gly Gln Gly Thr Lys Val Glu Ile Lys Arg
            100                 105                 110




<210>    10
<211>    112
<212>    PRT
<213>    Artificial Sequence

<220>
<223>    Deg C4 VL


<400>    10
Asp Ile Gln Leu Thr Gln Ser Pro Ser Ser Leu Ser Ala Ser Val Gly
1               5                   10                  15

Asp Arg Val Thr Ile Thr Cys Thr Gly Ser Ser Ser Asn Ile Gly Ala
            20                  25                  30

Val Val Val Thr Trp Tyr Gln Gln Lys Pro Gly Lys Ala Pro Lys Leu
        35                  40                  45

Leu Ile Tyr Ala Asp Ser His Arg Pro Ser Gly Val Pro Ser Arg Phe
    50                  55                  60

Ser Gly Ser Gly Ser Gly Thr Asp Phe Thr Leu Thr Ile Ser Ser Leu
65                  70                  75                  80

Gln Pro Glu Asp Phe Ala Thr Tyr Tyr Cys Gly Ala Trp Asp Asp Ser
                85                  90                  95

Leu Ser Gly Tyr Val Phe Gly Gln Gly Thr Lys Val Glu Ile Lys Arg
            100                 105                 110

<210>    11
<211>    10
<212>    PRT
<213>    Artificial Sequence

<220>
<223>    H-CDR1


<400>    11
Gly Phe Thr Phe Ser Gly Tyr Asp Met Ser
1               5                   10


<210>    12
<211>    17
<212>    PRT
<213>    Artificial Sequence

<220>
<223>    H-CDR2


<400>    12
Gly Ile Tyr Pro Asp Gly Gly Asn Thr Tyr Tyr Ala Asp Ser Val Lys
1               5                   10                  15

Gly



<210>    13
<211>    12
<212>    PRT
<213>    Artificial Sequence

<220>
<223>    H-CDR3


<400>    13
Gly Ala Thr Trp Trp Val Leu Gly Pro Phe Asp Tyr
1               5                   10


<210>    14
<211>    13
<212>    PRT
<213>    Artificial Sequence

<220>
<223>    L-CDR1


<400>    14
Thr Gly Ser Ser Ser Asn Ile Gly Asn Asn Ser Val Thr
1               5                   10


<210>    15

<211>    7
<212>    PRT
<213>    Artificial Sequence

<220>
<223>    L-CDR2


<400>    15
Ala Asp Ser His Arg Pro Ser
1               5


<210>    16
<211>    11
<212>    PRT
<213>    Artificial Sequence

<220>
<223>    L-CDR3


<400>    16
Gly Ala Trp Asp Asp Ser Leu Ser Gly Tyr Val
1               5                   10


<210>    17
<211>    25
<212>    PRT
<213>    Artificial Sequence

<220>
<223>    VH FR1


<400>    17


<210>    18
<211>    14
<212>    PRT
<213>    Artificial Sequence

<220>
<223>    VH FR2


<400>    18
Trp Ile Arg Gln Ala Pro Gly Lys Gly Leu Glu Trp Val Ala
1               5                   10


<210>    19
<211>    32
<212>    PRT
<213>    Artificial Sequence

<220>
<223>    VH FR3


<400>    19
Arg Phe Thr Ile Ser Arg Asp Asp Ser Lys Asn Thr Phe Tyr Leu Gln
1               5                   10                  15

Met Asn Ser Leu Arg Ala Glu Asp Thr Ala Val Tyr Tyr Cys Ala Arg
            20                  25                  30




<210>    20
<211>    11
<212>    PRT
<213>    Artificial Sequence

<220>
<223>    VH FR4


<400>    20
Trp Gly Gln Gly Thr Leu Val Thr Val Ser Ser
1               5                   10


<210>    21
<211>    23
<212>    PRT
<213>    Artificial Sequence

<220>
<223>    VL FR1


<400>    21
Asp Ile Gln Leu Thr Gln Ser Pro Ser Ser Leu Ser Ala Ser Val Gly
1               5                   10                  15

Asp Arg Val Thr Ile Thr Cys
            20


<210>    22
<211>    15
<212>    PRT
<213>    Artificial Sequence

<220>
<223>    VL FR2


<400>    22
Trp Tyr Gln Gln Lys Pro Gly Lys Ala Pro Lys Leu Leu Ile Tyr
1               5                   10                  15


<210>    23
<211>    32
<212>    PRT
<213>    Artificial Sequence

<220>
<223>    VL FR3

<400>    23
Gly Val Pro Ser Arg Phe Ser Gly Ser Gly Ser Gly Thr Asp Phe Thr
1               5                   10                  15

Leu Thr Ile Ser Ser Leu Gln Pro Glu Asp Phe Ala Thr Tyr Tyr Cys
            20                  25                  30

<210>    24
<211>    11
<212>    PRT
<213>    Artificial Sequence

<220>
<223>    VL FR4

<400>    24
Phe Gly Gln Gly Thr Lys Val Glu Ile Lys Arg
1               5                   10

<210>    25
<211>    12
<212>    PRT
<213>    Artificial Sequence

<220>
<223>    H-CDR3

<400>    25
Gly Ala Ala Thr Lys Val Leu Gly Lys Phe Asp Cys
1               5                   10

<210>    26
<211>    12
<212>    PRT
<213>    Artificial Sequence

<220>
<223>    H-CDR3

<400>    26
Gly Ala Ala Ser Pro Val Leu Gly Pro Phe Asp Glu
1               5                   10

<210>    27
<211>    12
<212>    PRT
<213>    Artificial Sequence

<220>

<223>    H-CDR3

<400>    27
Gly Ala Thr Trp Trp Val Leu Gly Ala Phe Asp Tyr
1               5                   10

<210>    28
<211>    12
<212>    PRT
<213>    Artificial Sequence

<220>
<223>    H-CDR3

<400>    28
Gly Ala Glu Phe Val Val Leu Gly Ser Phe Asp Met
1               5                   10

<210>    29
<211>    12
<212>    PRT
<213>    Artificial Sequence

<220>
<223>    H-CDR3

<400>    29
Gly Ala Ala Arg Cys Val Leu Gly Asn Phe Asp His
1               5                   10

<210>    30
<211>    12
<212>    PRT
<213>    Artificial Sequence

<220>
<223>    H-CDR3

<400>    30
Gly Ala Ser Arg Thr Val Leu Gly Asn Phe Asp Asp
1               5                   10

<210>    31
<211>    12
<212>    PRT
<213>    Artificial Sequence

<220>
<223>    H-CDR3

<400>    31
Gly Ala His Leu Thr Val Leu Gly Ile Phe Asp Thr
1               5                   10

<210>    32
<211>    12
<212>    PRT
<213>    Artificial Sequence

<220>
<223>    H-CDR3


<400>    32
Gly Ala His Ile Tyr Val Leu Gly Gln Phe Asp Gly
1               5                   10


<210>    33
<211>    12
<212>    PRT
<213>    Artificial Sequence

<220>
<223>    H-CDR3


<400>    33
Gly Ala Asn Leu Gly Val Leu Gly Glu Phe Asp Lys
1               5                   10


<210>    34
<211>    12
<212>    PRT
<213>    Artificial Sequence

<220>
<223>    H-CDR3


<400>    34
Gly Ala Val Pro Pro Val Leu Gly Ala Phe Asp Thr
1               5                   10


<210>    35
<211>    12
<212>    PRT
<213>    Artificial Sequence

<220>
<223>    H-CDR3


<400>    35
Gly Ala Arg Ile Asp Val Leu Gly Pro Phe Asp Ala
1               5                   10


<210>    36
<211>    12
<212>    PRT
<213>    Artificial Sequence

<220>

<223>    H-CDR3

<400>    36
Gly Ala Lys Pro Pro Val Leu Gly Gln Phe Asp Arg
1               5                   10

<210>    37
<211>    12
<212>    PRT
<213>    Artificial Sequence

<220>
<223>    H-CDR3

<400>    37
Gly Ala Met Glu Gln Val Leu Gly Pro Phe Asp Leu
1               5                   10

<210>    38
<211>    12
<212>    PRT
<213>    Artificial Sequence

<220>
<223>    H-CDR3

<400>    38
Gly Ala Arg Ser Trp Val Leu Gly Glu Phe Asp Lys
1               5                   10

<210>    39
<211>    12
<212>    PRT
<213>    Artificial Sequence

<220>
<223>    H-CDR3

<400>    39
Gly Ala Glu Cys Ala Val Leu Gly Asn Phe Asp Tyr
1               5                   10

<210>    40
<211>    12
<212>    PRT
<213>    Artificial Sequence

<220>
<223>    H-CDR3

<400>    40
Gly Ala Arg Arg Gln Val Leu Gly Cys Phe Asp Phe
1               5                   10

<210>    41
<211>    12
<212>    PRT
<213>    Artificial Sequence

<220>
<223>    H-CDR3


<400>    41
Gly Ala Ser Ala Pro Val Leu Gly Asp Phe Asp Tyr
1               5                   10


<210>    42
<211>    12
<212>    PRT
<213>    Artificial Sequence

<220>
<223>    H-CDR3


<400>    42
Gly Ala Ser Thr Pro Val Leu Gly Ser Phe Asp Asn
1               5                   10


<210>    43
<211>    12
<212>    PRT
<213>    Artificial Sequence

<220>
<223>    H-CDR3


<400>    43
Gly Ala His Tyr Asn Val Leu Gly Pro Phe Asp Ser
1               5                   10


<210>    44
<211>    12
<212>    PRT
<213>    Artificial Sequence

<220>
<223>    H-CDR3


<400>    44
Gly Ala Arg Thr Cys Val Leu Gly Pro Phe Asp His
1               5                   10


<210>    45
<211>    12
<212>    PRT
<213>    Artificial Sequence

<220>

<223>    H-CDR3

<400>    45
Gly Ala Ile Ala Pro Val Leu Gly Gly Phe Asp Arg
1               5                   10

<210>    46
<211>    12
<212>    PRT
<213>    Artificial Sequence

<220>
<223>    H-CDR3

<400>    46
Gly Ala Arg Gln Ser Val Leu Gly Pro Phe Asp Ala
1               5                   10

<210>    47
<211>    12
<212>    PRT
<213>    Artificial Sequence

<220>
<223>    H-CDR3

<400>    47
Gly Ala His Tyr Asn Val Leu Gly Pro Phe Asp Ser
1               5                   10

<210>    48
<211>    12
<212>    PRT
<213>    Artificial Sequence

<220>
<223>    H-CDR3

<400>    48
Gly Ala Lys Tyr Tyr Val Leu Gly His Phe Asp Glu
1               5                   10

<210>    49
<211>    12
<212>    PRT
<213>    Artificial Sequence

<220>
<223>    H-CDR3

<400>    49
Gly Ala Ala Leu Arg Val Leu Gly Met Phe Asp Leu
1               5                   10

<210>    50
<211>    12
<212>    PRT
<213>    Artificial Sequence

<220>
<223>    H-CDR3


<400>    50
Gly Ala His Arg Arg Val Leu Gly Trp Phe Asp Asp
1               5                   10


<210>    51
<211>    12
<212>    PRT
<213>    Artificial Sequence

<220>
<223>    H-CDR3


<400>    51
Gly Ala Tyr His Thr Val Leu Gly Gln Phe Asp Gln
1               5                   10


<210>    52
<211>    12
<212>    PRT
<213>    Artificial Sequence

<220>
<223>    H-CDR3


<400>    52
Gly Ala Gln Ser Thr Val Leu Gly Asn Phe Asp Thr
1               5                   10


<210>    53
<211>    12
<212>    PRT
<213>    Artificial Sequence

<220>
<223>    H-CDR3


<400>    53
Gly Ala His Met Arg Val Leu Gly Pro Phe Asp Gly
1               5                   10


<210>    54
<211>    12
<212>    PRT
<213>    Artificial Sequence

<220>

<223>    H-CDR3

<400>    54
Gly Ala Ala Leu His Val Leu Gly Pro Phe Asp Pro
1               5                   10

<210>    55
<211>    12
<212>    PRT
<213>    Artificial Sequence

<220>
<223>    H-CDR3

<400>    55
Gly Ala His Gly Gln Val Leu Gly Asp Phe Asp Gln
1               5                   10

<210>    56
<211>    12
<212>    PRT
<213>    Artificial Sequence

<220>
<223>    H-CDR3

<400>    56
Gly Ala Ser Gln Glu Val Leu Gly Asp Phe Asp Val
1               5                   10

<210>    57
<211>    12
<212>    PRT
<213>    Artificial Sequence

<220>
<223>    H-CDR3

<400>    57
Ser Ala Thr Trp Trp Met Ser Glu Pro Arg Ser Tyr
1               5                   10

<210>    58
<211>    12
<212>    PRT
<213>    Artificial Sequence

<220>
<223>    H-CDR3

<400>    58
Gly Ala Asp Asn His Val Leu Gly Asp Phe Asp Val
1               5                   10

<210>    59
<211>    12
<212>    PRT
<213>    Artificial Sequence

<220>
<223>    H-CDR3


<400>    59
Gly Ala His Lys Pro Val Leu Gly Asp Phe Asp Ala
1               5                   10


<210>    60
<211>    12
<212>    PRT
<213>    Artificial Sequence

<220>
<223>    H-CDR3


<400>    60
Gly Ala His His Ala Val Leu Gly Pro Phe Asp Arg
1               5                   10


<210>    61
<211>    121
<212>    PRT
<213>    Artificial Sequence

<220>
<223>    VH


<400>    61
Glu Val Gln Leu Val Glu Ser Gly Gly Gly Leu Val Gln Pro Gly Gly
1               5                   10                  15

Ser Leu Arg Leu Ser Cys Ala Val Ser Gly Phe Thr Phe Ser Gly Tyr
            20                  25                  30

Asp Met Ser Trp Ile Arg Gln Ala Pro Gly Lys Gly Leu Glu Trp Val
        35                  40                  45

Ala Gly Ile Tyr Pro Asp Gly Gly Asn Thr Tyr Tyr Ala Asp Ser Val
    50                  55                  60

Lys Gly Arg Phe Thr Ile Ser Arg Asp Asp Ser Lys Asn Thr Phe Tyr
65                  70                  75                  80

Leu Gln Met Asn Ser Leu Arg Ala Glu Asp Thr Ala Val Tyr Tyr Cys
                85                  90                  95

Ala Arg Gly Ala Ala Thr Lys Val Leu Gly Lys Phe Asp Cys Trp Gly
            100                 105                 110

Gln Gly Thr Leu Val Thr Val Ser Ser
        115                 120

<210>    62
<211>    121
<212>    PRT
<213>    Artificial Sequence

<220>
<223>    VH


<400>    62
Glu Val Gln Leu Val Glu Ser Gly Gly Gly Leu Val Gln Pro Gly Gly
1               5                   10                  15

Ser Leu Arg Leu Ser Cys Ala Val Ser Gly Phe Thr Phe Ser Gly Tyr
            20                  25                  30

Asp Met Ser Trp Ile Arg Gln Ala Pro Gly Lys Gly Leu Glu Trp Val
        35                  40                  45

Ala Gly Ile Tyr Pro Asp Gly Gly Asn Thr Tyr Tyr Ala Asp Ser Val
    50                  55                  60

Lys Gly Arg Phe Thr Ile Ser Arg Asp Asp Ser Lys Asn Thr Phe Tyr
65                  70                  75                  80

Leu Gln Met Asn Ser Leu Arg Ala Glu Asp Thr Ala Val Tyr Tyr Cys
                85                  90                  95

Ala Arg Gly Ala Ala Ser Pro Val Leu Gly Pro Phe Asp Glu Trp Gly
            100                 105                 110

Gln Gly Thr Leu Val Thr Val Ser Ser
        115                 120


<210>    63
<211>    121
<212>    PRT
<213>    Artificial Sequence

<220>
<223>    VH


<400>    63
Glu Val Gln Leu Val Glu Ser Gly Gly Gly Leu Val Gln Pro Gly Gly
1               5                   10                  15

Ser Leu Arg Leu Ser Cys Ala Val Ser Gly Phe Thr Phe Ser Gly Tyr
            20                  25                  30

Asp Met Ser Trp Ile Arg Gln Ala Pro Gly Lys Gly Leu Glu Trp Val
        35                  40                  45

Ala Gly Ile Tyr Pro Asp Gly Gly Asn Thr Tyr Tyr Ala Asp Ser Val
    50                  55                  60

Lys Gly Arg Phe Thr Ile Ser Arg Asp Asp Ser Lys Asn Thr Phe Tyr
65                  70                  75                  80

Leu Gln Met Asn Ser Leu Arg Ala Glu Asp Thr Ala Val Tyr Tyr Cys
                85                  90                  95

Ala Arg Gly Ala Thr Trp Trp Val Leu Gly Ala Phe Asp Tyr Trp Gly
            100                 105                 110

Gln Gly Thr Leu Val Thr Val Ser Ser
        115                 120

<210>  64
<211>  121
<212>  PRT
<213>  Artificial Sequence

<220>
<223>  VH

<400>  64
Glu Val Gln Leu Val Glu Ser Gly Gly Gly Leu Val Gln Pro Gly Gly
1               5                   10                  15

Ser Leu Arg Leu Ser Cys Ala Val Ser Gly Phe Thr Phe Ser Gly Tyr
            20                  25                  30

Asp Met Ser Trp Ile Arg Gln Ala Pro Gly Lys Gly Leu Glu Trp Val
        35                  40                  45

Ala Gly Ile Tyr Pro Asp Gly Gly Asn Thr Tyr Tyr Ala Asp Ser Val
    50                  55                  60

Lys Gly Arg Phe Thr Ile Ser Arg Asp Asp Ser Lys Asn Thr Phe Tyr
65                  70                  75                  80

Leu Gln Met Asn Ser Leu Arg Ala Glu Asp Thr Ala Val Tyr Tyr Cys
                85                  90                  95

Ala Arg Gly Ala Glu Phe Val Val Leu Gly Ser Phe Asp Met Trp Gly
            100                 105                 110

Gln Gly Thr Leu Val Thr Val Ser Ser
        115                 120

<210>  65
<211>  121
<212>  PRT
<213>  Artificial Sequence

<220>
<223>  VH

<400>  65
Glu Val Gln Leu Val Glu Ser Gly Gly Gly Leu Val Gln Pro Gly Gly
1               5                   10                  15

Ser Leu Arg Leu Ser Cys Ala Val Ser Gly Phe Thr Phe Ser Gly Tyr
            20                  25                  30

Asp Met Ser Trp Ile Arg Gln Ala Pro Gly Lys Gly Leu Glu Trp Val
        35                  40                  45

Ala Gly Ile Tyr Pro Asp Gly Gly Asn Thr Tyr Tyr Ala Asp Ser Val

50                  55                  60

Lys Gly Arg Phe Thr Ile Ser Arg Asp Asp Ser Lys Asn Thr Phe Tyr
65                  70                  75                  80

Leu Gln Met Asn Ser Leu Arg Ala Glu Asp Thr Ala Val Tyr Tyr Cys
                85                  90                  95

Ala Arg Gly Ala Ala Arg Cys Val Leu Gly Asn Phe Asp His Trp Gly
            100                 105                 110

Gln Gly Thr Leu Val Thr Val Ser Ser
        115                 120


<210>   66
<211>   121
<212>   PRT
<213>   Artificial Sequence

<220>
<223>   VH


<400>   66
Glu Val Gln Leu Val Glu Ser Gly Gly Gly Leu Val Gln Pro Gly Gly
1               5                   10                  15

Ser Leu Arg Leu Ser Cys Ala Val Ser Gly Phe Thr Phe Ser Gly Tyr
            20                  25                  30

Asp Met Ser Trp Ile Arg Gln Ala Pro Gly Lys Gly Leu Glu Trp Val
        35                  40                  45

Ala Gly Ile Tyr Pro Asp Gly Gly Asn Thr Tyr Tyr Ala Asp Ser Val
    50                  55                  60

Lys Gly Arg Phe Thr Ile Ser Arg Asp Asp Ser Lys Asn Thr Phe Tyr
65                  70                  75                  80

Leu Gln Met Asn Ser Leu Arg Ala Glu Asp Thr Ala Val Tyr Tyr Cys
                85                  90                  95

Ala Arg Gly Ala Ser Arg Thr Val Leu Gly Asn Phe Asp Asp Trp Gly
            100                 105                 110

Gln Gly Thr Leu Val Thr Val Ser Ser
        115                 120


<210>   67
<211>   121
<212>   PRT
<213>   Artificial Sequence

<220>
<223>   VH


<400>   67
Glu Val Gln Leu Val Glu Ser Gly Gly Gly Leu Val Gln Pro Gly Gly
1               5                   10                  15

Ser Leu Arg Leu Ser Cys Ala Val Ser Gly Phe Thr Phe Ser Gly Tyr
            20                  25                  30

Asp Met Ser Trp Ile Arg Gln Ala Pro Gly Lys Gly Leu Glu Trp Val
        35                  40                  45

Ala Gly Ile Tyr Pro Asp Gly Gly Asn Thr Tyr Tyr Ala Asp Ser Val
    50                  55                  60

Lys Gly Arg Phe Thr Ile Ser Arg Asp Asp Ser Lys Asn Thr Phe Tyr
65                  70                  75                  80

Leu Gln Met Asn Ser Leu Arg Ala Glu Asp Thr Ala Val Tyr Tyr Cys
                85                  90                  95

Ala Arg Gly Ala His Leu Thr Val Leu Gly Ile Phe Asp Thr Trp Gly
            100                 105                 110

Gln Gly Thr Leu Val Thr Val Ser Ser
        115                 120


<210>    68
<211>    121
<212>    PRT
<213>    Artificial Sequence

<220>
<223>    VH


<400>    68
Glu Val Gln Leu Val Glu Ser Gly Gly Gly Leu Val Gln Pro Gly Gly
1               5                   10                  15

Ser Leu Arg Leu Ser Cys Ala Val Ser Gly Phe Thr Phe Ser Gly Tyr
            20                  25                  30

Asp Met Ser Trp Ile Arg Gln Ala Pro Gly Lys Gly Leu Glu Trp Val
        35                  40                  45

Ala Gly Ile Tyr Pro Asp Gly Gly Asn Thr Tyr Tyr Ala Asp Ser Val
    50                  55                  60

Lys Gly Arg Phe Thr Ile Ser Arg Asp Asp Ser Lys Asn Thr Phe Tyr
65                  70                  75                  80

Leu Gln Met Asn Ser Leu Arg Ala Glu Asp Thr Ala Val Tyr Tyr Cys
                85                  90                  95

Ala Arg Gly Ala His Ile Tyr Val Leu Gly Gln Phe Asp Gly Trp Gly
            100                 105                 110

Gln Gly Thr Leu Val Thr Val Ser Ser
        115                 120


<210>    69
<211>    121
<212>    PRT
<213>    Artificial Sequence

<220>

<223>    VH


<400>    69
Glu Val Gln Leu Val Glu Ser Gly Gly Gly Leu Val Gln Pro Gly Gly
1               5                   10                  15

Ser Leu Arg Leu Ser Cys Ala Val Ser Gly Phe Thr Phe Ser Gly Tyr
            20                  25                  30

Asp Met Ser Trp Ile Arg Gln Ala Pro Gly Lys Gly Leu Glu Trp Val
        35                  40                  45

Ala Gly Ile Tyr Pro Asp Gly Gly Asn Thr Tyr Tyr Ala Asp Ser Val
    50                  55                  60

Lys Gly Arg Phe Thr Ile Ser Arg Asp Asp Ser Lys Asn Thr Phe Tyr
65                  70                  75                  80

Leu Gln Met Asn Ser Leu Arg Ala Glu Asp Thr Ala Val Tyr Tyr Cys
                85                  90                  95

Ala Arg Gly Ala Asn Leu Gly Val Leu Gly Glu Phe Asp Lys Trp Gly
            100                 105                 110

Gln Gly Thr Leu Val Thr Val Ser Ser
        115                 120


<210>    70
<211>    121
<212>    PRT
<213>    Artificial Sequence

<220>
<223>    VH


<400>    70
Glu Val Gln Leu Val Glu Ser Gly Gly Gly Leu Val Gln Pro Gly Gly
1               5                   10                  15

Ser Leu Arg Leu Ser Cys Ala Val Ser Gly Phe Thr Phe Ser Gly Tyr
            20                  25                  30

Asp Met Ser Trp Ile Arg Gln Ala Pro Gly Lys Gly Leu Glu Trp Val
        35                  40                  45

Ala Gly Ile Tyr Pro Asp Gly Gly Asn Thr Tyr Tyr Ala Asp Ser Val
    50                  55                  60

Lys Gly Arg Phe Thr Ile Ser Arg Asp Asp Ser Lys Asn Thr Phe Tyr
65                  70                  75                  80

Leu Gln Met Asn Ser Leu Arg Ala Glu Asp Thr Ala Val Tyr Tyr Cys
                85                  90                  95

Ala Arg Gly Ala Val Pro Pro Val Leu Gly Ala Phe Asp Thr Trp Gly
            100                 105                 110

Gln Gly Thr Leu Val Thr Val Ser Ser
        115                 120

<210>    71
<211>    121
<212>    PRT
<213>    Artificial Sequence

<220>
<223>    VH


<400>    71
Glu Val Gln Leu Val Glu Ser Gly Gly Gly Leu Val Gln Pro Gly Gly
1               5                   10                  15

Ser Leu Arg Leu Ser Cys Ala Val Ser Gly Phe Thr Phe Ser Gly Tyr
            20                  25                  30

Asp Met Ser Trp Ile Arg Gln Ala Pro Gly Lys Gly Leu Glu Trp Val
        35                  40                  45

Ala Gly Ile Tyr Pro Asp Gly Gly Asn Thr Tyr Tyr Ala Asp Ser Val
    50                  55                  60

Lys Gly Arg Phe Thr Ile Ser Arg Asp Asp Ser Lys Asn Thr Phe Tyr
65                  70                  75                  80

Leu Gln Met Asn Ser Leu Arg Ala Glu Asp Thr Ala Val Tyr Tyr Cys
                85                  90                  95

Ala Arg Gly Ala Arg Ile Asp Val Leu Gly Pro Phe Asp Ala Trp Gly
            100                 105                 110

Gln Gly Thr Leu Val Thr Val Ser Ser
        115                 120


<210>    72
<211>    121
<212>    PRT
<213>    Artificial Sequence

<220>
<223>    VH


<400>    72
Glu Val Gln Leu Val Glu Ser Gly Gly Gly Leu Val Gln Pro Gly Gly
1               5                   10                  15

Ser Leu Arg Leu Ser Cys Ala Val Ser Gly Phe Thr Phe Ser Gly Tyr
            20                  25                  30

Asp Met Ser Trp Ile Arg Gln Ala Pro Gly Lys Gly Leu Glu Trp Val
        35                  40                  45

Ala Gly Ile Tyr Pro Asp Gly Gly Asn Thr Tyr Tyr Ala Asp Ser Val
    50                  55                  60

Lys Gly Arg Phe Thr Ile Ser Arg Asp Asp Ser Lys Asn Thr Phe Tyr
65                  70                  75                  80

Leu Gln Met Asn Ser Leu Arg Ala Glu Asp Thr Ala Val Tyr Tyr Cys
                85                  90                  95

Ala Arg Gly Ala Lys Pro Pro Val Leu Gly Gln Phe Asp Arg Trp Gly
            100                 105                 110

Gln Gly Thr Leu Val Thr Val Ser Ser
        115                 120

<210>  73
<211>  121
<212>  PRT
<213>  Artificial Sequence

<220>
<223>  VH

<400>  73
Glu Val Gln Leu Val Glu Ser Gly Gly Gly Leu Val Gln Pro Gly Gly
1               5                   10                  15

Ser Leu Arg Leu Ser Cys Ala Val Ser Gly Phe Thr Phe Ser Gly Tyr
            20                  25                  30

Asp Met Ser Trp Ile Arg Gln Ala Pro Gly Lys Gly Leu Glu Trp Val
        35                  40                  45

Ala Gly Ile Tyr Pro Asp Gly Gly Asn Thr Tyr Tyr Ala Asp Ser Val
    50                  55                  60

Lys Gly Arg Phe Thr Ile Ser Arg Asp Asp Ser Lys Asn Thr Phe Tyr
65                  70                  75                  80

Leu Gln Met Asn Ser Leu Arg Ala Glu Asp Thr Ala Val Tyr Tyr Cys
                85                  90                  95

Ala Arg Gly Ala Met Glu Gln Val Leu Gly Pro Phe Asp Leu Trp Gly
            100                 105                 110

Gln Gly Thr Leu Val Thr Val Ser Ser
        115                 120

<210>  74
<211>  121
<212>  PRT
<213>  Artificial Sequence

<220>
<223>  VH

<400>  74
Glu Val Gln Leu Val Glu Ser Gly Gly Gly Leu Val Gln Pro Gly Gly
1               5                   10                  15

Ser Leu Arg Leu Ser Cys Ala Val Ser Gly Phe Thr Phe Ser Gly Tyr
            20                  25                  30

Asp Met Ser Trp Ile Arg Gln Ala Pro Gly Lys Gly Leu Glu Trp Val
        35                  40                  45

Ala Gly Ile Tyr Pro Asp Gly Gly Asn Thr Tyr Tyr Ala Asp Ser Val

50                  55                  60

Lys Gly Arg Phe Thr Ile Ser Arg Asp Asp Ser Lys Asn Thr Phe Tyr
65                  70                  75                  80

Leu Gln Met Asn Ser Leu Arg Ala Glu Asp Thr Ala Val Tyr Tyr Cys
                85                  90                  95

Ala Arg Gly Ala Arg Ser Trp Val Leu Gly Glu Phe Asp Lys Trp Gly
            100                 105                 110

Gln Gly Thr Leu Val Thr Val Ser Ser
        115                 120


<210>   75
<211>   121
<212>   PRT
<213>   Artificial Sequence

<220>
<223>   VH


<400>   75
Glu Val Gln Leu Val Glu Ser Gly Gly Gly Leu Val Gln Pro Gly Gly
1               5                   10                  15

Ser Leu Arg Leu Ser Cys Ala Val Ser Gly Phe Thr Phe Ser Gly Tyr
            20                  25                  30

Asp Met Ser Trp Ile Arg Gln Ala Pro Gly Lys Gly Leu Glu Trp Val
        35                  40                  45

Ala Gly Ile Tyr Pro Asp Gly Gly Asn Thr Tyr Tyr Ala Asp Ser Val
    50                  55                  60

Lys Gly Arg Phe Thr Ile Ser Arg Asp Asp Ser Lys Asn Thr Phe Tyr
65                  70                  75                  80

Leu Gln Met Asn Ser Leu Arg Ala Glu Asp Thr Ala Val Tyr Tyr Cys
                85                  90                  95

Ala Arg Gly Ala Glu Cys Ala Val Leu Gly Asn Phe Asp Tyr Trp Gly
            100                 105                 110

Gln Gly Thr Leu Val Thr Val Ser Ser
        115                 120


<210>   76
<211>   121
<212>   PRT
<213>   Artificial Sequence

<220>
<223>   VH


<400>   76
Glu Val Gln Leu Val Glu Ser Gly Gly Gly Leu Val Gln Pro Gly Gly
1               5                   10                  15

Ser Leu Arg Leu Ser Cys Ala Val Ser Gly Phe Thr Phe Ser Gly Tyr
            20                  25                  30

Asp Met Ser Trp Ile Arg Gln Ala Pro Gly Lys Gly Leu Glu Trp Val
        35                  40                  45

Ala Gly Ile Tyr Pro Asp Gly Gly Asn Thr Tyr Tyr Ala Asp Ser Val
    50                  55                  60

Lys Gly Arg Phe Thr Ile Ser Arg Asp Asp Ser Lys Asn Thr Phe Tyr
65                  70                  75                  80

Leu Gln Met Asn Ser Leu Arg Ala Glu Asp Thr Ala Val Tyr Tyr Cys
                85                  90                  95

Ala Arg Gly Ala Arg Arg Gln Val Leu Gly Cys Phe Asp Phe Trp Gly
            100                 105                 110

Gln Gly Thr Leu Val Thr Val Ser Ser
        115                 120


<210> 77
<211> 121
<212> PRT
<213> Artificial Sequence

<220>
<223> VH


<400> 77
Glu Val Gln Leu Val Glu Ser Gly Gly Gly Leu Val Gln Pro Gly Gly
1               5                   10                  15

Ser Leu Arg Leu Ser Cys Ala Val Ser Gly Phe Thr Phe Ser Gly Tyr
            20                  25                  30

Asp Met Ser Trp Ile Arg Gln Ala Pro Gly Lys Gly Leu Glu Trp Val
        35                  40                  45

Ala Gly Ile Tyr Pro Asp Gly Gly Asn Thr Tyr Tyr Ala Asp Ser Val
    50                  55                  60

Lys Gly Arg Phe Thr Ile Ser Arg Asp Asp Ser Lys Asn Thr Phe Tyr
65                  70                  75                  80

Leu Gln Met Asn Ser Leu Arg Ala Glu Asp Thr Ala Val Tyr Tyr Cys
                85                  90                  95

Ala Arg Gly Ala Ser Ala Pro Val Leu Gly Asp Phe Asp Tyr Trp Gly
            100                 105                 110

Gln Gly Thr Leu Val Thr Val Ser Ser
        115                 120


<210> 78
<211> 121
<212> PRT
<213> Artificial Sequence

<220>

<223>    VH


<400>    78
Glu Val Gln Leu Val Glu Ser Gly Gly Gly Leu Val Gln Pro Gly Gly
1               5                   10                  15

Ser Leu Arg Leu Ser Cys Ala Val Ser Gly Phe Thr Phe Ser Gly Tyr
            20                  25                  30

Asp Met Ser Trp Ile Arg Gln Ala Pro Gly Lys Gly Leu Glu Trp Val
        35                  40                  45

Ala Gly Ile Tyr Pro Asp Gly Gly Asn Thr Tyr Tyr Ala Asp Ser Val
    50                  55                  60

Lys Gly Arg Phe Thr Ile Ser Arg Asp Asp Ser Lys Asn Thr Phe Tyr
65                  70                  75                  80

Leu Gln Met Asn Ser Leu Arg Ala Glu Asp Thr Ala Val Phe Phe Cys
                85                  90                  95

Ala Arg Gly Ala Ser Thr Pro Val Leu Gly Ser Phe Asp Asn Trp Gly
            100                 105                 110

Gln Gly Thr Leu Val Thr Val Ser Ser
        115                 120


<210>    79
<211>    121
<212>    PRT
<213>    Artificial Sequence

<220>
<223>    VH


<400>    79
Glu Val Gln Leu Val Glu Ser Gly Gly Gly Leu Val Gln Pro Gly Gly
1               5                   10                  15

Ser Leu Arg Leu Ser Cys Ala Val Ser Gly Phe Thr Phe Ser Gly Tyr
            20                  25                  30

Asp Met Ser Trp Ile Arg Gln Ala Pro Gly Lys Gly Leu Glu Trp Val
        35                  40                  45

Ala Gly Ile Tyr Pro Asp Gly Gly Asn Thr Tyr Tyr Ala Asp Ser Val
    50                  55                  60

Lys Gly Arg Phe Thr Ile Ser Arg Asp Asp Ser Lys Asn Thr Phe Tyr
65                  70                  75                  80

Leu Gln Met Asn Ser Leu Arg Ala Glu Asp Thr Ala Val Phe Tyr Cys
                85                  90                  95

Ala Arg Gly Ala His Tyr Asn Val Leu Gly Pro Phe Asp Ser Trp Gly
            100                 105                 110

Gln Gly Thr Leu Val Thr Val Ser Ser
        115                 120

<210>    80
<211>    121
<212>    PRT
<213>    Artificial Sequence

<220>
<223>    VH


<400>    80
Glu Val Gln Leu Val Glu Ser Gly Gly Gly Leu Val Gln Pro Gly Gly
1               5                   10                  15

Ser Leu Arg Leu Ser Cys Ala Val Ser Gly Phe Thr Phe Ser Gly Tyr
            20                  25                  30

Asp Met Ser Trp Ile Arg Gln Ala Pro Gly Lys Gly Leu Glu Trp Val
        35                  40                  45

Ala Gly Ile Tyr Pro Asp Gly Gly Asn Thr Tyr Tyr Ala Asp Ser Val
    50                  55                  60

Lys Gly Arg Phe Thr Ile Ser Arg Asp Asp Ser Lys Asn Thr Phe Tyr
65                  70                  75                  80

Leu Gln Met Asn Ser Leu Arg Ala Glu Asp Thr Ala Val Tyr Tyr Cys
                85                  90                  95

Ala Arg Gly Ala Arg Thr Cys Val Leu Gly Pro Phe Asp His Trp Gly
            100                 105                 110

Gln Gly Thr Leu Val Thr Val Ser Ser
        115                 120


<210>    81
<211>    121
<212>    PRT
<213>    Artificial Sequence

<220>
<223>    VH


<400>    81
Glu Val Gln Leu Val Glu Ser Gly Gly Gly Leu Val Gln Pro Gly Gly
1               5                   10                  15

Ser Leu Arg Leu Ser Cys Ala Val Ser Gly Phe Thr Phe Ser Gly Tyr
            20                  25                  30

Asp Met Ser Trp Ile Arg Gln Ala Pro Gly Lys Gly Leu Glu Trp Val
        35                  40                  45

Ala Gly Ile Tyr Pro Asp Gly Gly Asn Thr Tyr Tyr Ala Asp Ser Val
    50                  55                  60

Lys Gly Arg Phe Thr Ile Ser Arg Asp Asp Ser Lys Asn Thr Phe Tyr
65                  70                  75                  80

Leu Gln Met Asn Ser Leu Arg Ala Glu Asp Thr Ala Val Tyr Tyr Cys
                85                  90                  95

Ala Arg Gly Ala Ile Ala Pro Val Leu Gly Gly Phe Asp Arg Trp Gly
            100                 105                 110

Gln Gly Thr Leu Val Thr Val Ser Ser
        115                 120

<210>  82
<211>  121
<212>  PRT
<213>  Artificial Sequence

<220>
<223>  VH

<400>  82
Glu Val Gln Leu Val Glu Ser Gly Gly Gly Leu Val Gln Pro Gly Gly
1               5                   10                  15

Ser Leu Arg Leu Ser Cys Ala Val Ser Gly Phe Thr Phe Ser Gly Tyr
            20                  25                  30

Asp Met Ser Trp Ile Arg Gln Ala Pro Gly Lys Gly Leu Glu Trp Val
        35                  40                  45

Ala Gly Ile Tyr Pro Asp Gly Gly Asn Thr Tyr Tyr Ala Asp Ser Val
    50                  55                  60

Lys Gly Arg Phe Thr Ile Ser Arg Asp Asp Ser Lys Asn Thr Phe Tyr
65                  70                  75                  80

Leu Gln Met Asn Ser Leu Arg Ala Glu Asp Thr Ala Val Tyr Tyr Cys
                85                  90                  95

Ala Arg Gly Ala Arg Gln Ser Val Leu Gly Pro Phe Asp Ala Trp Gly
            100                 105                 110

Gln Gly Thr Leu Val Thr Val Ser Ser
        115                 120

<210>  83
<211>  121
<212>  PRT
<213>  Artificial Sequence

<220>
<223>  VH

<400>  83
Glu Val Gln Leu Val Glu Ser Gly Gly Gly Leu Val Gln Pro Gly Gly
1               5                   10                  15

Ser Leu Arg Leu Ser Cys Ala Val Ser Gly Phe Thr Phe Ser Gly Tyr
            20                  25                  30

Asp Met Ser Trp Ile Arg Gln Ala Pro Gly Lys Gly Leu Glu Trp Val
        35                  40                  45

Ala Gly Ile Tyr Pro Asp Gly Gly Asn Thr Tyr Tyr Ala Asp Ser Val

50                  55                  60

Lys Gly Arg Phe Thr Ile Ser Arg Asp Asp Ser Lys Asn Thr Phe Tyr
65                  70                  75                  80

Leu Gln Met Asn Ser Leu Arg Ala Glu Asp Thr Ala Val Tyr Tyr Cys
                85                  90                  95

Ala Arg Gly Ala His Tyr Asn Val Leu Gly Pro Phe Asp Ser Trp Gly
            100                 105                 110

Gln Gly Thr Leu Val Thr Val Ser Ser
        115                 120


<210>   84
<211>   121
<212>   PRT
<213>   Artificial Sequence

<220>
<223>   VH


<400>   84
Glu Val Gln Leu Val Glu Ser Gly Gly Gly Leu Val Gln Pro Gly Gly
1               5                   10                  15

Ser Leu Arg Leu Ser Cys Ala Val Ser Gly Phe Thr Phe Ser Gly Tyr
            20                  25                  30

Asp Met Ser Trp Ile Arg Gln Ala Pro Gly Lys Gly Leu Glu Trp Val
        35                  40                  45

Ala Gly Ile Tyr Pro Asp Gly Gly Asn Thr Tyr Tyr Ala Asp Ser Val
    50                  55                  60

Lys Gly Arg Phe Thr Ile Ser Arg Asp Asp Ser Lys Asn Thr Phe Tyr
65                  70                  75                  80

Leu Gln Met Asn Ser Leu Arg Ala Glu Asp Thr Ala Val Tyr Tyr Cys
                85                  90                  95

Ala Arg Gly Ala Lys Tyr Tyr Val Leu Gly His Phe Asp Glu Trp Gly
            100                 105                 110

Gln Gly Thr Leu Val Thr Val Ser Ser
        115                 120


<210>   85
<211>   121
<212>   PRT
<213>   Artificial Sequence

<220>
<223>   VH


<400>   85
Glu Val Gln Leu Val Glu Ser Gly Gly Gly Leu Val Gln Pro Gly Gly
1               5                   10                  15

Ser Leu Arg Leu Ser Cys Ala Val Ser Gly Phe Thr Phe Ser Gly Tyr
            20                  25                  30

Asp Met Ser Trp Ile Arg Gln Ala Pro Gly Lys Gly Leu Glu Trp Val
        35                  40                  45

Ala Gly Ile Tyr Pro Asp Gly Gly Asn Thr Tyr Tyr Ala Asp Ser Val
    50                  55                  60

Lys Gly Arg Phe Thr Ile Ser Arg Asp Asp Ser Lys Asn Thr Phe Tyr
65                  70                  75                  80

Leu Gln Met Asn Ser Leu Arg Ala Glu Asp Thr Ala Val Tyr Tyr Cys
                85                  90                  95

Ala Arg Gly Ala Ala Leu Arg Val Leu Gly Met Phe Asp Leu Trp Gly
            100                 105                 110

Gln Gly Thr Leu Val Thr Val Ser Ser
        115                 120


<210>   86
<211>   121
<212>   PRT
<213>   Artificial Sequence

<220>
<223>   VH


<400>   86
Glu Val Gln Leu Val Glu Ser Gly Gly Gly Leu Val Gln Pro Gly Gly
1               5                   10                  15

Ser Leu Arg Leu Ser Cys Ala Val Ser Gly Phe Thr Phe Ser Gly Tyr
            20                  25                  30

Asp Met Ser Trp Ile Arg Gln Ala Pro Gly Lys Gly Leu Glu Trp Val
        35                  40                  45

Ala Gly Ile Tyr Pro Asp Gly Gly Asn Thr Tyr Tyr Ala Asp Ser Val
    50                  55                  60

Lys Gly Arg Phe Thr Ile Ser Arg Asp Asp Ser Lys Asn Thr Phe Tyr
65                  70                  75                  80

Leu Gln Met Asn Ser Leu Arg Ala Glu Asp Thr Ala Val Tyr Tyr Cys
                85                  90                  95

Ala Arg Gly Ala His Arg Arg Val Leu Gly Trp Phe Asp Asp Trp Gly
            100                 105                 110

Gln Gly Thr Leu Val Thr Val Ser Ser
        115                 120


<210>   87
<211>   121
<212>   PRT
<213>   Artificial Sequence

<220>

<223>    VH


<400>    87
Glu Val Gln Leu Val Glu Ser Gly Gly Gly Leu Val Gln Pro Gly Gly
1               5                   10                  15

Ser Leu Arg Leu Ser Cys Ala Val Ser Gly Phe Thr Phe Ser Gly Tyr
            20                  25                  30

Asp Met Ser Trp Ile Arg Gln Ala Pro Gly Lys Gly Leu Glu Trp Val
        35                  40                  45

Ala Gly Ile Tyr Pro Asp Gly Gly Asn Thr Tyr Tyr Ala Asp Ser Val
    50                  55                  60

Lys Gly Arg Phe Thr Ile Ser Arg Asp Asp Ser Lys Asn Thr Phe Tyr
65                  70                  75                  80

Leu Gln Met Asn Ser Leu Arg Ala Glu Asp Thr Ala Val Tyr Tyr Cys
                85                  90                  95

Ala Arg Gly Ala Tyr His Thr Val Leu Gly Gln Phe Asp Gln Trp Gly
            100                 105                 110

Gln Gly Thr Leu Val Thr Val Ser Ser
        115                 120


<210>    88
<211>    121
<212>    PRT
<213>    Artificial Sequence

<220>
<223>    VH


<400>    88
Glu Val Gln Leu Val Glu Ser Gly Gly Gly Leu Val Gln Pro Gly Gly
1               5                   10                  15

Ser Leu Arg Leu Ser Cys Ala Val Ser Gly Phe Thr Phe Ser Gly Tyr
            20                  25                  30

Asp Met Ser Trp Ile Arg Gln Ala Pro Gly Lys Gly Leu Glu Trp Val
        35                  40                  45

Ala Gly Ile Tyr Pro Asp Gly Gly Asn Thr Tyr Tyr Ala Asp Ser Val
    50                  55                  60

Lys Gly Arg Phe Thr Ile Ser Arg Asp Asp Ser Lys Asn Thr Phe Tyr
65                  70                  75                  80

Leu Gln Met Asn Ser Leu Arg Ala Glu Asp Thr Ala Val Tyr Tyr Cys
                85                  90                  95

Ala Arg Gly Ala Gln Ser Thr Val Leu Gly Asn Phe Asp Thr Trp Gly
            100                 105                 110

Gln Gly Thr Leu Val Thr Val Ser Ser
        115                 120

<210>    89
<211>    121
<212>    PRT
<213>    Artificial Sequence

<220>
<223>    VH


<400>    89
Glu Val Gln Leu Val Glu Ser Gly Gly Gly Leu Val Gln Pro Gly Gly
1               5                   10                  15

Ser Leu Arg Leu Ser Cys Ala Val Ser Gly Phe Thr Phe Ser Gly Tyr
            20                  25                  30

Asp Met Ser Trp Ile Arg Gln Ala Pro Gly Lys Gly Leu Glu Trp Val
        35                  40                  45

Ala Gly Ile Tyr Pro Asp Gly Gly Asn Thr Tyr Tyr Ala Asp Ser Val
    50                  55                  60

Lys Gly Arg Phe Thr Ile Ser Arg Asp Asp Ser Lys Asn Thr Phe Tyr
65                  70                  75                  80

Leu Gln Met Asn Ser Leu Arg Ala Glu Asp Thr Ala Val Tyr Tyr Cys
                85                  90                  95

Ala Arg Gly Ala His Met Arg Val Leu Gly Pro Phe Asp Gly Trp Gly
            100                 105                 110

Gln Gly Thr Leu Val Thr Val Ser Ser
        115                 120


<210>    90
<211>    121
<212>    PRT
<213>    Artificial Sequence

<220>
<223>    VH


<400>    90
Glu Val Gln Leu Val Glu Ser Gly Gly Gly Leu Val Gln Pro Gly Gly
1               5                   10                  15

Ser Leu Arg Leu Ser Cys Ala Val Ser Gly Phe Thr Phe Ser Gly Tyr
            20                  25                  30

Asp Met Ser Trp Ile Arg Gln Ala Pro Gly Lys Gly Leu Glu Trp Val
        35                  40                  45

Ala Gly Ile Tyr Pro Asp Gly Gly Asn Thr Tyr Tyr Ala Asp Ser Val
    50                  55                  60

Lys Gly Arg Phe Thr Ile Ser Arg Asp Asp Ser Lys Asn Thr Phe Tyr
65                  70                  75                  80

Leu Gln Met Asn Ser Leu Arg Ala Glu Asp Thr Ala Val Tyr Tyr Cys
                85                  90                  95

Ala Arg Gly Ala Ala Leu His Val Leu Gly Pro Phe Asp Pro Trp Gly
            100                 105                 110

Gln Gly Thr Leu Val Thr Val Ser Ser
        115                 120

<210>  91
<211>  121
<212>  PRT
<213>  Artificial Sequence

<220>
<223>  VH

<400>  91
Glu Val Gln Leu Val Glu Ser Gly Gly Gly Leu Val Gln Pro Gly Gly
1               5                   10                  15

Ser Leu Arg Leu Ser Cys Ala Val Ser Gly Phe Thr Phe Ser Gly Tyr
            20                  25                  30

Asp Met Ser Trp Ile Arg Gln Ala Pro Gly Lys Gly Leu Glu Trp Val
        35                  40                  45

Ala Gly Ile Tyr Pro Asp Gly Gly Asn Thr Tyr Tyr Ala Asp Ser Val
    50                  55                  60

Lys Gly Arg Phe Thr Ile Ser Arg Asp Asp Ser Lys Asn Thr Phe Tyr
65                  70                  75                  80

Leu Gln Met Asn Ser Leu Arg Ala Glu Asp Thr Ala Val Tyr Tyr Cys
                85                  90                  95

Ala Arg Gly Ala His Gly Gln Val Leu Gly Asp Phe Asp Gln Trp Gly
            100                 105                 110

Gln Gly Thr Leu Val Thr Val Ser Ser
        115                 120

<210>  92
<211>  121
<212>  PRT
<213>  Artificial Sequence

<220>
<223>  VH

<400>  92
Glu Val Gln Leu Val Glu Ser Gly Gly Gly Leu Val Gln Pro Gly Gly
1               5                   10                  15

Ser Leu Arg Leu Ser Cys Ala Val Ser Gly Phe Thr Phe Ser Gly Tyr
            20                  25                  30

Asp Met Ser Trp Ile Arg Gln Ala Pro Gly Lys Gly Leu Glu Trp Val
        35                  40                  45

Ala Gly Ile Tyr Pro Asp Gly Gly Asn Thr Tyr Tyr Ala Asp Ser Val

50                  55                  60

Lys Gly Arg Phe Thr Ile Ser Arg Asp Asp Ser Lys Asn Thr Phe Tyr
65                  70                  75                  80

Leu Gln Met Asn Ser Leu Arg Ala Glu Asp Thr Ala Val Tyr Tyr Cys
                85                  90                  95

Ala Arg Gly Ala Ser Gln Glu Val Leu Gly Asp Phe Asp Val Trp Gly
            100                 105                 110

Gln Gly Thr Leu Val Thr Val Ser Ser
        115                 120


<210>   93
<211>   121
<212>   PRT
<213>   Artificial Sequence

<220>
<223>   VH


<400>   93
Glu Val Gln Leu Val Glu Ser Gly Gly Gly Leu Val Gln Pro Gly Gly
1               5                   10                  15

Ser Leu Arg Leu Ser Cys Ala Val Ser Gly Phe Thr Phe Ser Gly Tyr
            20                  25                  30

Asp Met Ser Trp Ile Arg Gln Ala Pro Gly Lys Gly Leu Glu Trp Val
        35                  40                  45

Ala Gly Ile Tyr Pro Asp Gly Gly Asn Thr Tyr Tyr Ala Asp Ser Val
    50                  55                  60

Lys Gly Arg Phe Thr Ile Ser Arg Asp Asp Ser Lys Asn Thr Phe Tyr
65                  70                  75                  80

Leu Gln Met Asn Ser Leu Arg Ala Glu Asp Thr Ala Val Tyr Tyr Cys
                85                  90                  95

Ala Arg Ser Ala Thr Trp Trp Met Ser Glu Pro Arg Ser Tyr Trp Gly
            100                 105                 110

Gln Gly Thr Leu Val Thr Val Ser Ser
        115                 120


<210>   94
<211>   121
<212>   PRT
<213>   Artificial Sequence

<220>
<223>   VH


<400>   94
Glu Val Gln Leu Val Glu Ser Gly Gly Gly Leu Val Gln Pro Gly Gly
1               5                   10                  15

Ser Leu Arg Leu Ser Cys Ala Val Ser Gly Phe Thr Phe Ser Gly Tyr
            20                  25                  30

Asp Met Ser Trp Ile Arg Gln Ala Pro Gly Lys Gly Leu Glu Trp Val
        35                  40                  45

Ala Gly Ile Tyr Pro Asp Gly Gly Asn Thr Tyr Tyr Ala Asp Ser Val
    50                  55                  60

Lys Gly Arg Phe Thr Ile Ser Arg Asp Asp Ser Lys Asn Thr Phe Tyr
65                  70                  75                  80

Leu Gln Met Asn Ser Leu Arg Ala Glu Asp Thr Ala Val Tyr Tyr Cys
                85                  90                  95

Ala Arg Gly Ala Asp Asn His Val Leu Gly Asp Phe Asp Val Trp Gly
            100                 105                 110

Gln Gly Thr Leu Val Thr Val Ser Ser
        115                 120


<210>   95
<211>   121
<212>   PRT
<213>   Artificial Sequence

<220>
<223>   VH


<400>   95
Glu Val Gln Leu Val Glu Ser Gly Gly Gly Leu Val Gln Pro Gly Gly
1               5                   10                  15

Ser Leu Arg Leu Ser Cys Ala Val Ser Gly Phe Thr Phe Ser Gly Tyr
            20                  25                  30

Asp Met Ser Trp Ile Arg Gln Ala Pro Gly Lys Gly Leu Glu Trp Val
        35                  40                  45

Ala Gly Ile Tyr Pro Asp Gly Gly Asn Thr Tyr Tyr Ala Asp Ser Val
    50                  55                  60

Lys Gly Arg Phe Thr Ile Ser Arg Asp Asp Ser Lys Asn Thr Phe Tyr
65                  70                  75                  80

Leu Gln Met Asn Ser Leu Arg Ala Glu Asp Thr Ala Val Tyr Tyr Cys
                85                  90                  95

Ala Arg Gly Ala His Lys Pro Val Leu Gly Asp Phe Asp Ala Trp Gly
            100                 105                 110

Gln Gly Thr Leu Val Thr Val Ser Ser
        115                 120


<210>   96
<211>   121
<212>   PRT
<213>   Artificial Sequence

<220>

<223>    VH


<400>    96
Glu Val Gln Leu Val Glu Ser Gly Gly Gly Leu Val Gln Pro Gly Gly
1               5                   10                  15

Ser Leu Arg Leu Ser Cys Ala Val Ser Gly Phe Thr Phe Ser Gly Tyr
            20                  25                  30

Asp Met Ser Trp Ile Arg Gln Ala Pro Gly Lys Gly Leu Glu Trp Val
        35                  40                  45

Ala Gly Ile Tyr Pro Asp Gly Gly Asn Thr Tyr Tyr Ala Asp Ser Val
    50                  55                  60

Lys Gly Arg Phe Thr Ile Ser Arg Asp Asp Ser Lys Asn Thr Phe Tyr
65                  70                  75                  80

Leu Gln Met Asn Ser Leu Arg Ala Glu Asp Thr Ala Val Tyr Tyr Cys
                85                  90                  95

Ala Arg Gly Ala His His Ala Val Leu Gly Pro Phe Asp Arg Trp Gly
            100                 105                 110

Gln Gly Thr Leu Val Thr Val Ser Ser
        115                 120